# EUROPÄISCHE PATENTSCHRIFT

(11) **EP 3 107 977 B1**
(45) Veröffentlichungstag und Bekanntmachung des Hinweises auf die Patenterteilung: **24.01.2018**
(21) Anmeldenummer: 15702630.3
(22) Anmeldetag: 27.01.2015
(51) Int. Cl.: C09K 11/06, H01L 51/00, H01L 31/00, H01L 33/00, H05B 33/20, C07D 209/00, C07D 401/04, C07D 405/04, C07D 405/10, C07D 409/04, C07D 413/04, C07D 413/10, C07D 209/94, C07D 209/96, C09B 11/00, C09B 57/00, H01L 51/50, C09B 21/00

(54) **MATERIALIEN FÜR ORGANISCHE ELEKTROLUMINESZENZVORRICHTUNGEN**
MATERIALS FOR ORGANIC ELECTROLUMINESCENT DEVICES
MATIÈRES POUR DISPOSITIFS ÉLECTROLUMINESCENTS ORGANIQUES

(30) Priorität: 21.02.2014 EP 14000624
(43) Veröffentlichungstag der Anmeldung: 28.12.2016
(73) Patentinhaber: Merck Patent GmbH, 64293 Darmstadt (DE)
(72) Erfinder: JATSCH, Anja, 60489 Frankfurt am Main (DE); GROSSMANN, Tobias, 64297 Darmstadt (DE); PARHAM, Amir Hossain, 60486 Frankfurt am Main (DE); EBERLE, Thomas, 76829 Landau (DE); PFLUMM, Christof, 64291 Darmstadt (DE); KROEBER, Jonas Valentin, 60311 Frankfurt am Main (DE); STOESSEL, Philipp, 60389 Frankfurt am Main (DE); MONTENEGRO, Elvira, 69469 Weinheim (DE); LINGE, Rouven, 64291 Darmstadt (DE)
(86) Internationale Anmeldenummer: PCT/EP2015/000146
(87) Internationale Veröffentlichungsnummer: WO 2015/124255

(56) Entgegenhaltungen:
- WO-A1-2012/069121
- WO-A1-2013/041176
- WO-A1-2013/056776
- WO-A1-2014/010910
- DE-A1-102009 031 021
- DE-A1-102009 053 645
- US-A1- 2013 256 645

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.
In organischen Elektrolumineszenzvorrichtungen (OLEDs) werden als emittierende Materialien häufig metallorganische Komplexe eingesetzt, die Phosphoreszenz statt Fluoreszenz zeigen, da hiermit eine wesentlich höhere Energie- und Leistungseffizienz möglich ist als mit fluoreszierenden Emittern. Die Eigenschaften phosphoreszierender OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Gemäß dem Stand der Technik werden unter anderem Indenocarbazolderivate, z. B. gemäß WO 2010/136109, WO 2011/000455 oder KR 20120081539, oder Fluoren- bzw. Spirobifluorenderivate, z. B. gemäß WO 2012/074210, oder WO2014010910 A1 als Matrixmaterialien für phosphoreszierende Emitter in organischen Elektrolumineszenzvorrichtungen eingesetzt. Hier sind weitere Verbesserungen wünschenswert, insbesondere in Bezug auf die Effizienz, die Lebensdauer und die thermische Stabilität der Materialien. Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Lochtransport- und/oder Elektronenblockiermaterial oder gegebenenfalls als Lochblockiermaterial und/oder Elektronentransportmaterial. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß Formel (1), Formel (2) oder Formel (3), 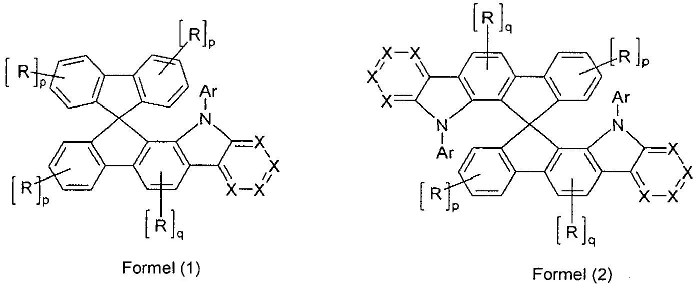 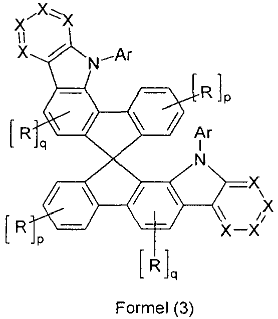 wobei für die verwendeten Symbole und Indizes gilt:
- X: ist bei jedem Auftreten gleich oder verschieden CR¹ oder N; oder zwei benachbarte X stehen für eine Gruppe der folgenden Formel (4), (5) oder (6), 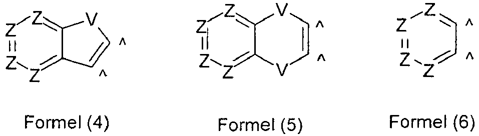 wobei ^ die entsprechenden benachbarten Gruppen X in der Formel (1) bzw. (2) bzw. (3) andeutet; d.h. an diesen Positionen ist die Gruppe der Formel (4), (5) bzw. (6) an die Verbindung der Formel (1), (2) bzw. (3) ankondensiert;
- V: ist bei jedem Auftreten gleich oder verschieden C(R¹)₂, NR¹, O, S, BR¹, Si(R¹)₂ oder C=O;
- Z: ist bei jedem Auftreten gleich oder verschieden CR¹ oder N;
- Ar: ist bei jedem Auftreten gleich oder verschieden ein aromatisches Ringsystem mit 6 bis 40 aromatischen Ringatomen, das mit einem oder mehreren Resten R² substituiert sein kann, oder ein hetero-aromatisches Ringsystem mit 5 bis 40 aromatischen Ringatomen, das keine elektronenarmen Heteroarylgruppen enthält und das mit einem oder mehreren Resten R² substituiert sein kann;
- R, R¹: ist bei jedem Auftreten gleich oder verschieden ausgewählt aus der Gruppe bestehend aus H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³, Si(R³)₂, C=O, C=S, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R³ substituiert sein kann, einer Aryloxy- oder Heteroaryloxygruppe mit 5 bis 40 aromatischen Ringatomen, die mit einem oder mehreren Resten R³ substituiert sein kann, oder einer Aralkyl-oder Heteroaralkylgruppe mit 5 bis 40 aromatischen Ringatomen, die mit einem oder mehreren Resten R³ substituiert sein kann; dabei können optional zwei Substituenten R¹, die an dasselbe Kohlenstoffatom gebunden sind, ein monocyclisches oder polycyclisches, aliphatisches, aromatisches oder heteroaromatisches Ringsystem bilden, das mit einem oder mehreren Resten R³ substituiert sein kann;
- R²: ist gleich oder verschieden bei jedem Auftreten ist bei jedem Auftreten gleich oder verschieden ausgewählt aus der Gruppe bestehend aus H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, einer geradkettigen Alkyl-, Alkoxy- oder Thioalkylgruppe mit 1 bis 20 C-Atomen oder einer verzweigten oder cyclischen Alkyl-, Alkoxy-oder Thioalkylgruppe mit 3 bis 20 C-Atomen oder einer Alkenyl-gruppe mit 2 bis 20 C-Atomen, die jeweils mit einem oder mehreren Resten R³ substituiert sein kann, wobei eine oder mehrere nicht-benachbarte CH₂-Gruppen durch R³C=CR³, Si(R³)₂, C=O, C=S, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das keine elektronenarmen Heteroarylgruppen enthält und das jeweils mit einem oder mehreren Resten R³ substituiert sein kann, einer Aryloxy- oder Heteroaryloxygruppe mit 5 bis 40 aromatischen Ringatomen, die keine elektronenarmen Heteroarylgruppen enthält und die mit einem oder mehreren Resten R³ substituiert sein kann, oder einer Aralkyl- oder Heteroaralkylgruppe mit 5 bis 40 aromatischen Ringatomen, die keine elektronenarmen Heteroarylgruppen enthält und die mit einem oder mehreren Resten R³ substituiert sein kann; dabei können zwei oder mehr benachbarte Substituenten R³ miteinander ein mono- oder polycyclisches, aliphatisches Ringsystem bilden;
- Ar¹: ist bei jedem Auftreten gleich oder verschieden ein aromatisches oder heteroaromatisches Ringsystem mit 5-30 aromatischen Ring-atomen, das mit einem oder mehreren nicht-aromatischen Resten R³ substituiert sein kann; dabei können zwei Reste Ar¹, welche an dasselbe N-Atom, P-Atom oder B-Atom binden, auch durch eine Einfachbindung oder eine Brücke, ausgewählt aus N(R³), C(R³)₂, O oder S, miteinander verbrückt sein;
- R³: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches Ringsystem bilden können;
- p: ist bei jedem Auftreten gleich oder verschieden 0, 1, 2, 3 oder 4;
- q: ist 0, 1 oder 2;
dabei sind die beiden folgenden Verbindungen von der Erfindung ausgenommen:

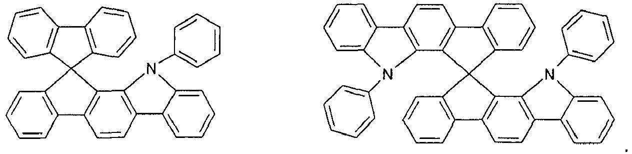

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe mit 5 aromatischen Ringatomen, welche mindestens zwei Heteroatome aufweist, oder eine Heteroarylgruppe mit 6 aromatischen Ringatomen, welche mindestens ein Heteroatom aufweist, sowie kondensierte Heteroarylgruppen enthaltend einen Fünfring mit mindestens zwei Heteroatomen und/oder einen Sechsring mit mindestens einem Heteroatom. Dabei ist eine kondensierte Heteroarylgruppe eine Heteroarylgruppe, in der mindestens eine Heteroarylgruppe und mindestens eine weitere Aryl- oder Heteroarylgruppe über eine gemeinsame Kante direkt aneinander ankondensiert sind. So sind beispielsweise Imidazol, Oxazol, Benzimidazol, Pyridin, Pyrimidin, Triazin, Chinolin, Chinoxalin, Phenanthrolin, etc. als elektronenarme Heteroarylgruppen anzusehen, während beispielsweise Pyrrol, Furan, Thiophen, Indol, Benzofuran, Benzothiophen, Carbazol, Dibenzofuran oder Dibenzothiophen nicht als elektronenarme Heteroarylgruppen im Sinne der vorliegenden Erfindung anzusehen sind, da diese weder heteroaromatische Fünfringe mit zwei oder mehr Heteroatomen noch heteroaromatische Sechsringe mit mindestens einem Heteroatom enthalten.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind.

In einer bevorzugten Ausführungsform der Erfindung steht X gleich oder verschieden bei jedem Auftreten für CR¹ oder N, wobei maximal eine Gruppe X pro Cyclus für N steht; oder zwei benachbarte Gruppen X stehen für eine Gruppe der Formel (4), wobei Z gleich oder verschieden bei jedem Auftreten für CR¹ steht und V gleich oder verschieden bei jedem Auftreten für NR¹, C(R¹)₂, O oder S steht. Besonders bevorzugt steht X gleich oder verschieden bei jedem Auftreten für CR¹. Wenn benachbarte Gruppen X für eine Gruppe der Formel (4) stehen, ist es bevorzugt, wenn die restlichen Gruppen X in diesem Cyclus für CR¹ stehen.

Bevorzugte Ausführungsformen der Verbindungen gemäß Formel (1) sind die Verbindungen der folgenden Formeln (7) bis (13), bevorzugte Ausführungsformen der Verbindungen gemäß Formel (2) sind die Verbindungen der folgenden Formel (14) und bevorzugte Ausführungsformen der Verbindungen gemäß Formel (3) sind die Verbindungen der folgenden Formel (15), 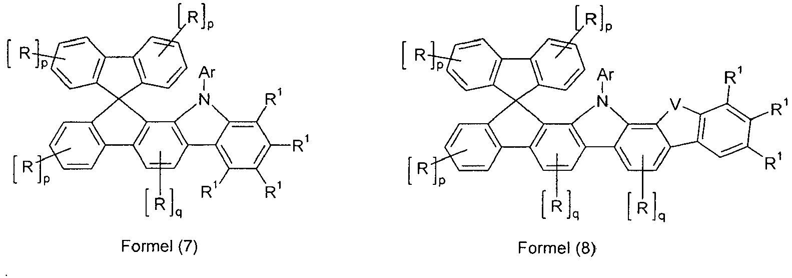 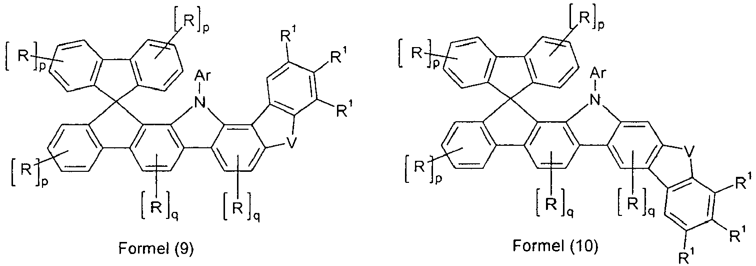 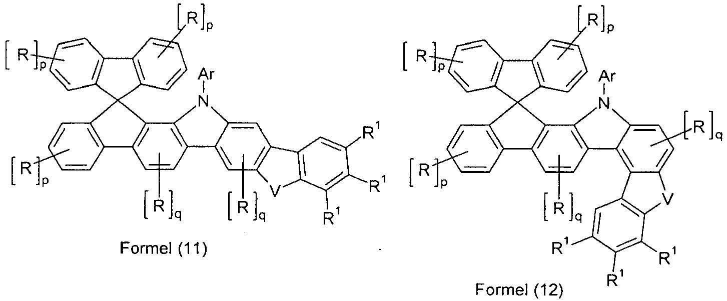 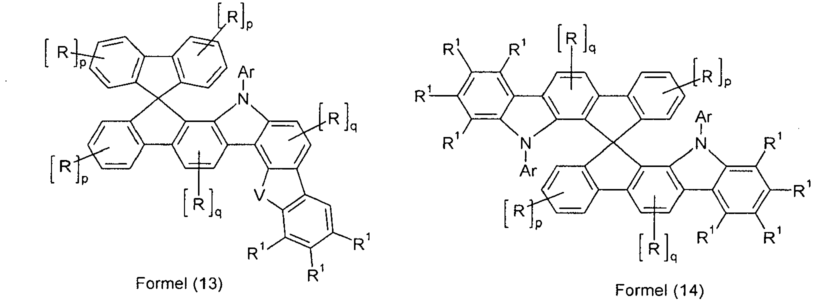 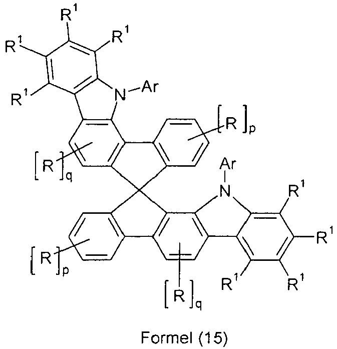 wobei die verwendeten Symbole und Indizes die oben genannten Bedeutungen aufweisen. Dabei steht V bevorzugt für NR¹, C(R¹)₂, O oder S. Es kann bevorzugt sein, wenn für V = C(R¹)₂ die beiden Reste R¹ miteinander einen Ring bilden und so ein Spirosystem aufspannen.

In einer bevorzugten Ausführungsform der Erfindung ist p bei jedem Auftreten gleich oder verschieden 0, 1 oder 2, besonders bevorzugt 0 oder 1 und ganz besonders bevorzugt gleich 0.

Weiterhin bevorzugt ist q bei jedem Auftreten gleich oder verschieden 0 oder 1, besonders bevorzugt gleich 0.

Besonders bevorzugte Ausführungsformen der Strukturen gemäß Formel (7) bis (15) sind die Strukturen der Formeln (7a) bis (15a), 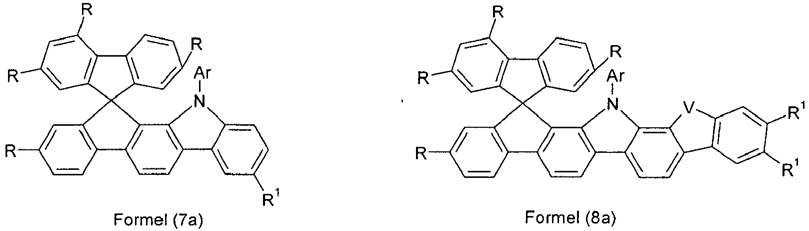 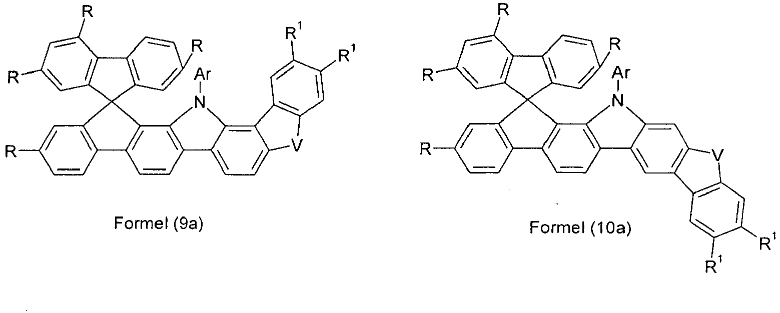 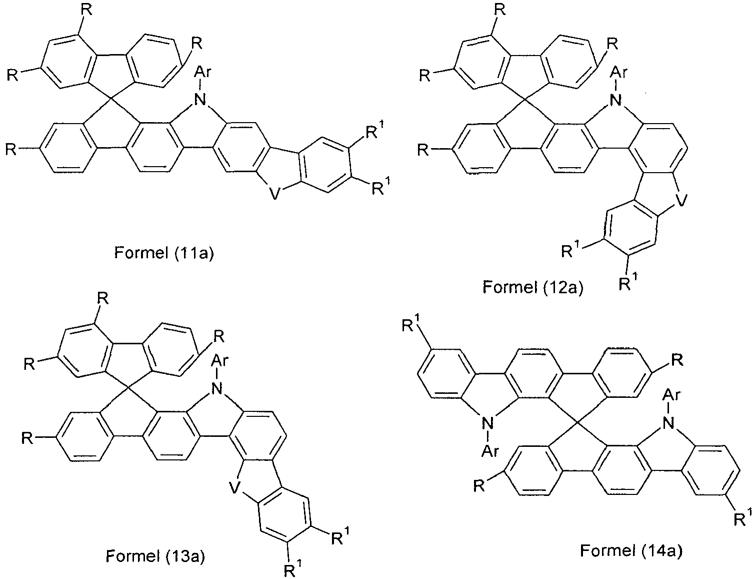 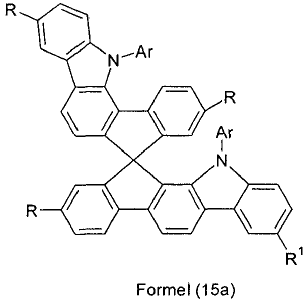 wobei die verwendeten Symbole und Indizes die oben genannten Bedeutungen aufweisen.

In einer bevorzugten Ausführungsform der Erfindung ist R gleich oder verschieden bei jedem Auftreten ausgewählt aus der Gruppe bestehend aus H, F, CN, N(Ar¹)₂, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 aromatischen Ringatomen, das mit einem oder mehreren nicht-aromatischen Resten R³ substituiert sein kann. In einer besonders bevorzugten Ausführungsform der Erfindung ist R gleich oder verschieden bei jedem Auftreten ausgewählt aus der Gruppe bestehend aus H, einer geradkettigen Alkylgruppe mit 1 bis 4 C-Atomen oder einer verzweigten oder cyclischen Alkylgruppe mit 3 bis 8 C-Atomen, ganz besonders bevorzugt gleich H.

Ganz besonders bevorzugt sind somit die Verbindungen der folgenden Formeln (7b) bis (15b), 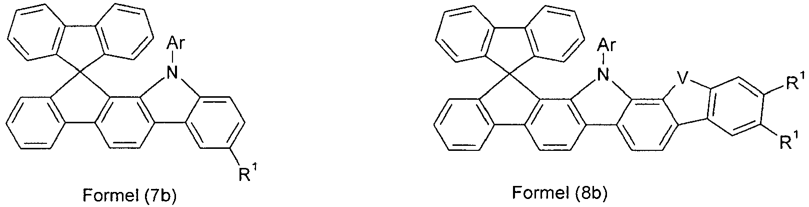 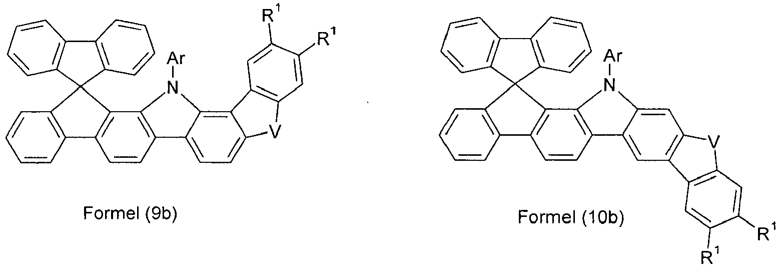 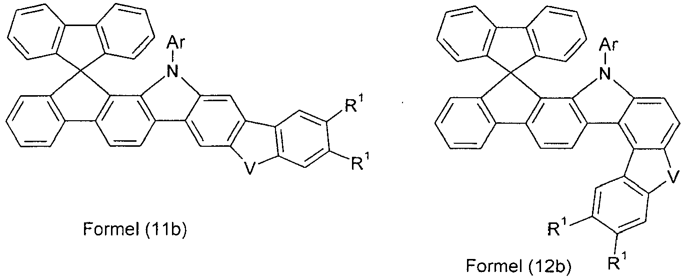 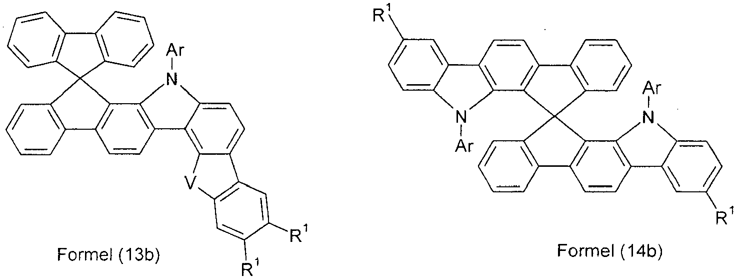 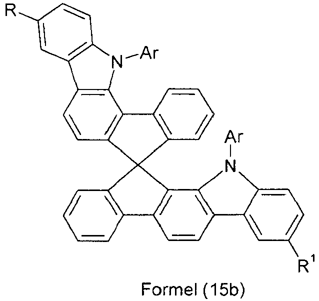 wobei die verwendeten Symbole die oben genannten Bedeutungen aufweisen.

Im Folgenden werden die bevorzugten Substituenten Ar und R¹ an den Verbindungen der Formel (1), (2) und (3) sowie den oben aufgeführten bevorzugten Ausführungsformen beschrieben.

Es ist bevorzugt, wenn die Verbindungen der Formel (1) und (3) bzw. die bevorzugten Ausführungsformen in den Substituenten Ar, R¹ und R² insgesamt mindestens 12 aromatische Ringatomen enthalten und die Verbindungen der Formel (2) bzw. die bevorzugten Ausführungsformen in den Substituenten Ar, R¹ und R² insgesamt mindestens 24 aromatische Ringatomen enthalten. Dies kann auf unterschiedliche Weise erfolgen. So ist es einerseits möglich, dass Ar oder Ar zusammen mit dem Substituenten R², welcher an Ar gebunden ist, ein aromatisches oder heteroaromatisches Ringsystem mit mindestens 12 aromatischen Ringatomen darstellt. In diesem Fall können auch alle Gruppen R¹ gleich H bzw. ungleich einem aromatischen oder heteroaromatischen Ringsystem sein. Weiterhin ist es möglich, dass mindestens ein Rest R¹ ein aromatisches oder heteroaromatisches Ringsystem mit mindestens 6 aromatischen Ringatomen oder eine Gruppe N(Ar¹)₂ darstellt. In diesem Fall kann die Gruppe Ar beispielsweise auch bevorzugt für eine Phenylgruppe stehen, da dann dennoch die Substituenten Ar, R¹ und R² in der erfindungsgemäßen Verbindung insgesamt 12 oder mehr aromatische Ringatome enthalten.

In einer bevorzugten Ausführungsform der Erfindung steht Ar gleich oder verschieden bei jedem Auftreten für ein aromatisches oder heteroaromatisches Ringsystem mit 12 bis 30 aromatischen Ringatomen, das jeweils mit einem oder mehreren Resten R² substituiert sein kann. Dabei enthält, wie oben definiert, das heteroaromatische Ringsystem keine elektronenarmen Heteroarylgruppen. Besonders bevorzugte aromatische oder heteroaromatische Ringsysteme enthalten 12 bis 24 aromatische Ringatome und können durch einen oder mehrere Reste R² substituiert sein. Dabei enthalten die aromatischen bzw. heteroaromatischen Ringsysteme bevorzugt keine kondensierten Aryl- bzw. Heteroarylgruppen, in denen mehr als zwei aromatische Sechsringe direkt aneinander ankondensiert sind, außer gegebenenfalls Triphenylengruppen. Besonders bevorzugt enthalten sie überhaupt keine Aryl- bzw. Heteroarylgruppen, in denen aromatische Sechsringe direkt aneinander ankondensiert sind. Diese Bevorzugung gilt vor allem dann, wenn die erfindungsgemäße Verbindung als Matrixmaterial für einen phosphoreszierenden Emitter oder in einer Schicht, die direkt an eine phosphoreszierende emittierende Schicht angrenzt, eingesetzt wird und ist mit der höheren Triplettenergie nicht-kondensierter Substituenten zu begründen. So ist es besonders bevorzugt, wenn Ar beispielsweise keine Naphthylgruppen oder höhere kondensierte Arylgruppen aufweist und ebenso keine Chinolingruppen, Acridingruppen, etc.. Dagegen ist es möglich, dass Ar beispielsweise Carbazolgruppen, Dibenzofurangruppen, Fluorengruppen, etc. aufweist, weil in diesen Strukturen keine 6-Ring-Aromaten bzw. -Heteroaromaten direkt aneinander ankondensiert sind, also keine gemeinsame Kante aufweisen.

Bevorzugte Gruppen Ar sind gleich oder verschieden bei jedem Auftreten ausgewählt aus der Gruppe bestehend aus Phenyl, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol oder Kombinationen aus zwei oder drei dieser Gruppen, welche jeweils mit einem oder mehreren Resten R² substituiert sein können. Weitere geeignete Gruppen Ar sind Anthracen, Phenanthren, Triphenylen, Pyren oder Benzanthracen, die jeweils mit einem oder mehreren Resten R² substituiert sein können, oder Kombinationen aus zwei oder drei dieser Gruppen miteinander und/oder mit den oben genannten Gruppen. Diese Gruppen eignen sich insbesondere dann, wenn die Triplettenergie der erfindungsgemäßen Verbindung nicht von Bedeutung ist.

Bevorzugte aromatische und heteroaromatische Ringsysteme Ar sind die Gruppen der folgenden Formeln Ar-1 bis Ar-57, 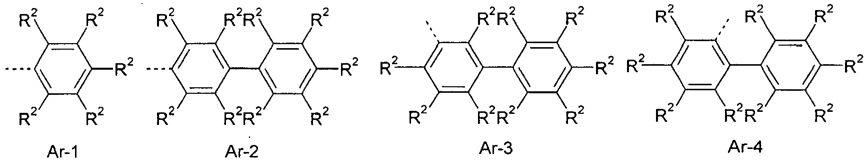 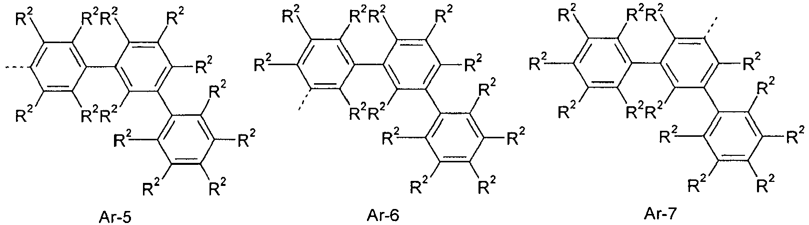 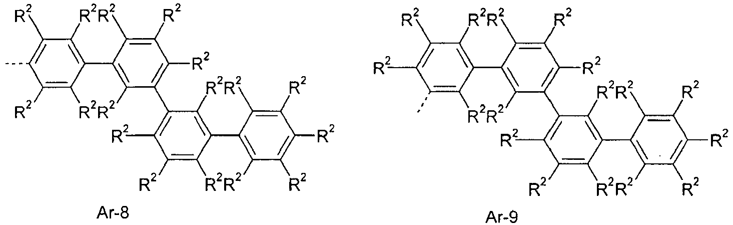 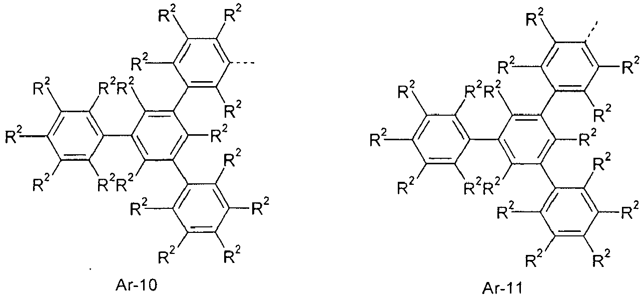 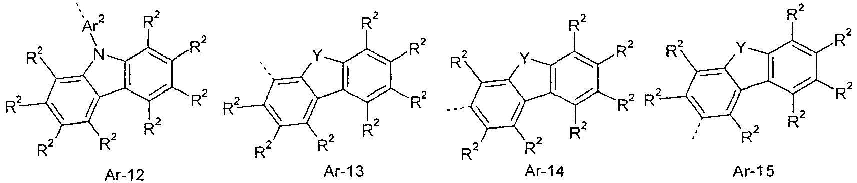 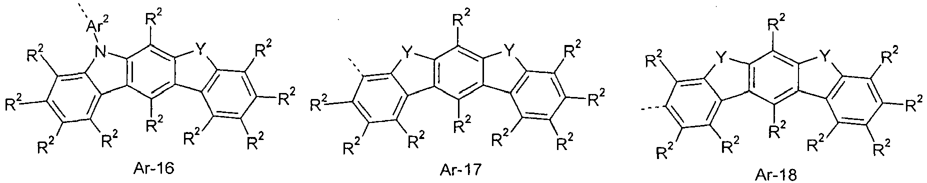 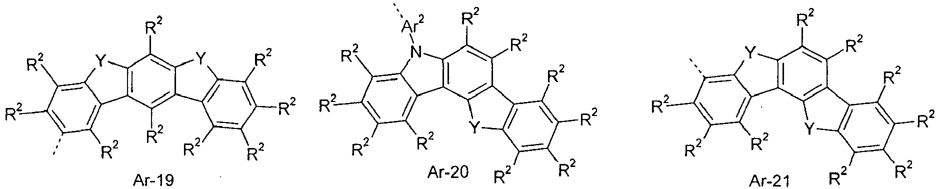 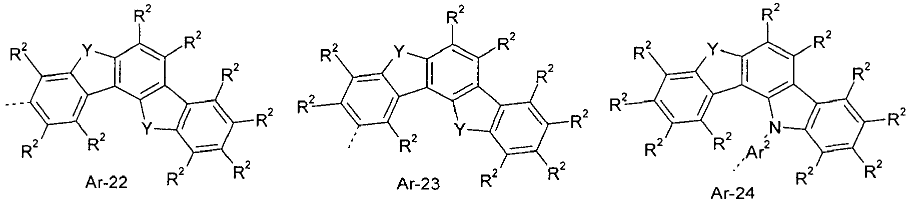 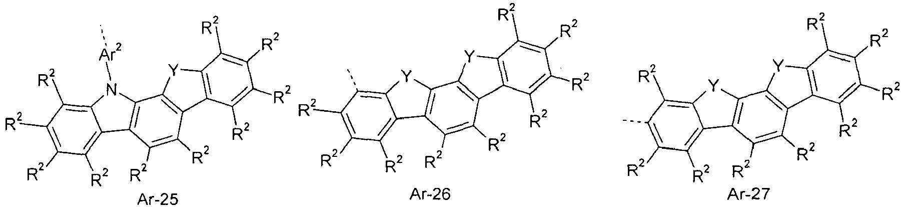 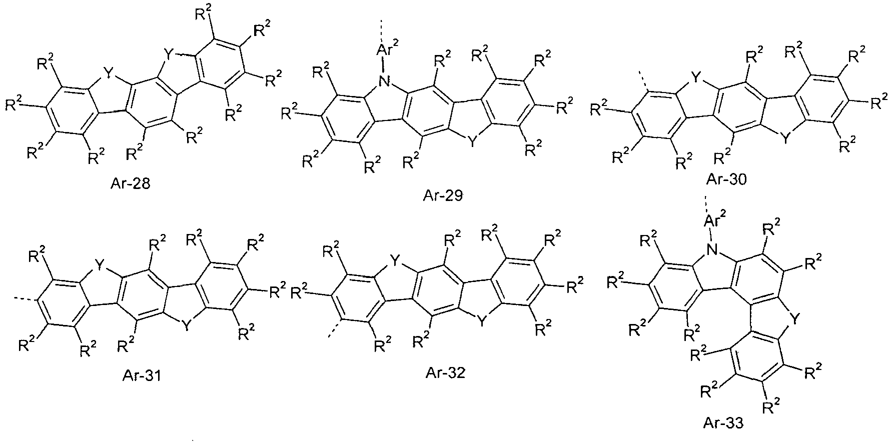 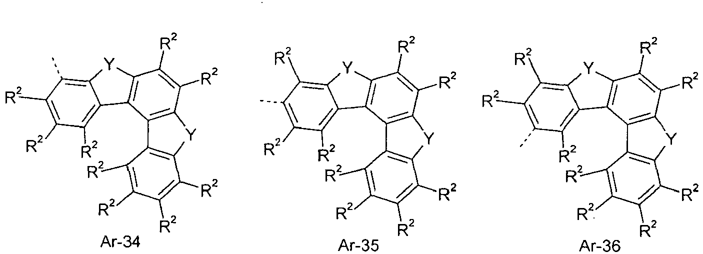 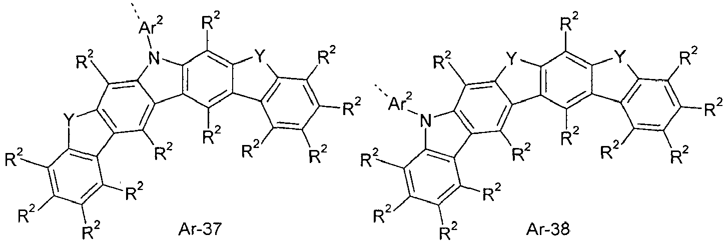 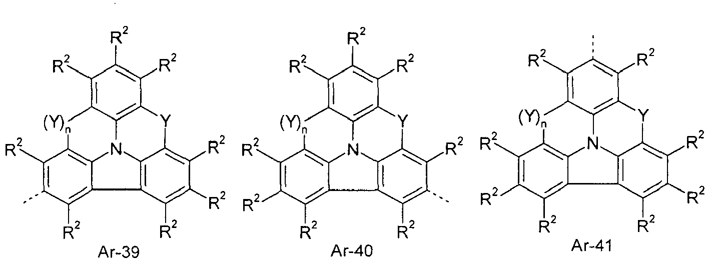 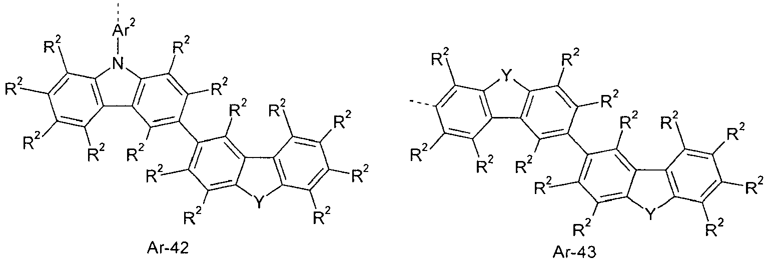 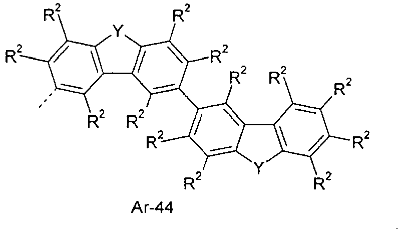 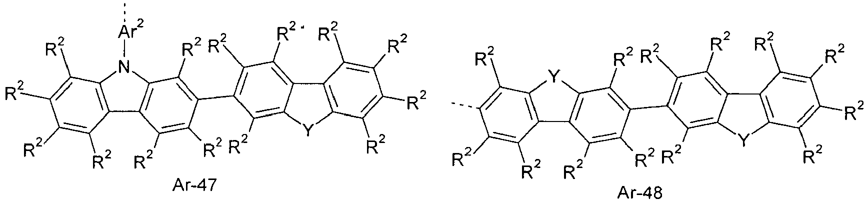 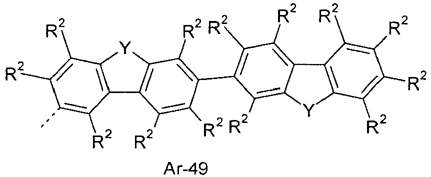 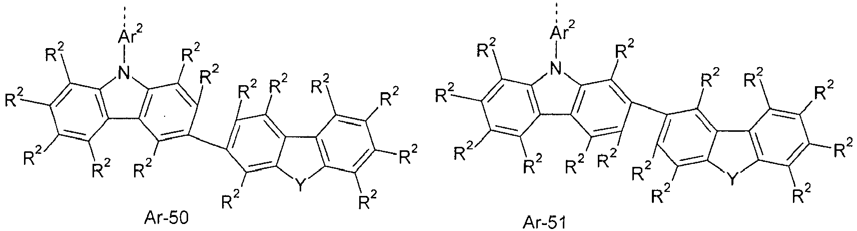 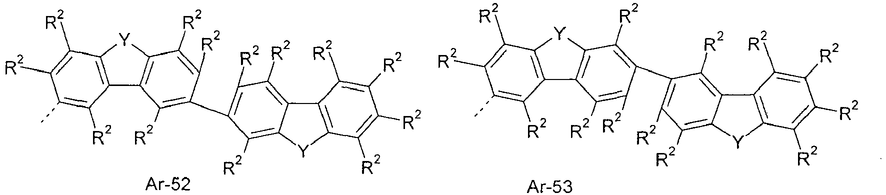 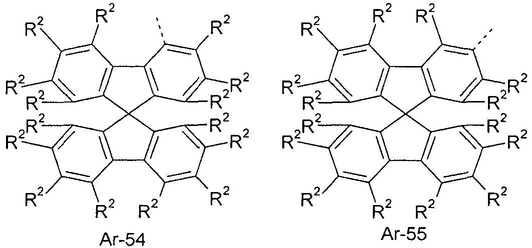 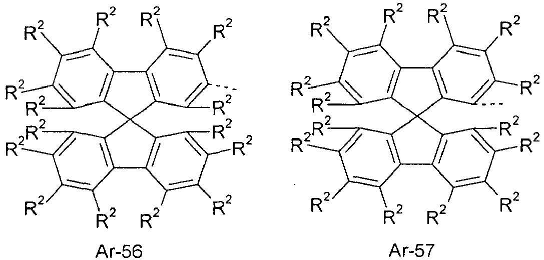 wobei R² die oben genannten Bedeutungen aufweist, die gestrichelte Bindung die Bindung an die Gruppe der Formel (1) bzw. (2) bzw. (3) darstellt und weiterhin gilt:
- Ar²: ist bei jedem Auftreten gleich oder verschieden ein bivalentes aromatisches oder heteroaromatisches Ringsystem mit 6 bis 18 aromatischen Ringatomen, welches keine elektronenarmen Heteroarylgruppen enthält und welches jeweils mit einem oder mehreren Resten R² substituiert sein kann;
- Y: ist bei jedem Auftreten gleich oder verschieden C(R²)₂, NR², O oder S;
- n: ist 0 oder 1, wobei n gleich 0 bedeutet, dass an dieser Position keine Gruppe Y gebunden ist und an den entsprechenden Kohlenstoffatomen statt dessen Reste R² gebunden sind.

Wenn die oben genannten Gruppen für Ar mehrere Gruppen Y aufweisen, so kommen hierfür alle Kombinationen aus der Definition von Y in Frage. Bevorzugt sind Gruppen, in denen eine Gruppe Y für NR² und die andere Gruppe Y für C(R²)₂ steht oder in denen beide Gruppen Y für NR² stehen oder in denen beide Gruppen Y für O stehen.

In einer weiteren bevorzugten Ausführungsform der Erfindung steht mindestens eine Gruppe Y für C(R²)₂ oder für NR².

Wenn Y für NR² steht, steht der Substituent R², der an das Stickstoffatom gebunden ist, bevorzugt für ein aromatisches oder heteroaromatisches Ringsystem mit 5 bis 24 aromatischen Ringatomen, welches auch durch einen oder mehrere Reste R³ substituiert sein kann. In einer besonders bevorzugten Ausführungsform steht dieser Substituent R² gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in welchen zwei oder mehr aromatische bzw. heteroaromatische 6-Ring-Gruppen direkt aneinander ankondensiert sind, aufweist und welches jeweils auch durch einen oder mehrere Reste R³ substituiert sein kann. Besonders bevorzugt sind Phenyl, Biphenyl, Terphenyl und Quaterphenyl mit Verknüpfungsmustern, wie vorne für Ar-1 bis Ar-11 aufgeführt, wobei diese Strukturen bevorzugt unsubstituiert sind.

Wenn Y für C(R²)₂ steht, steht R² bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R³ substituiert sein kann. Ganz besonders bevorzugt steht R² für eine Methylgruppe oder für eine Phenylgruppe. Dabei können die Reste R² auch miteinander ein Ringsystem bilden, was zu einem Spirosystem führt.

In einer bevorzugten Ausführungsform der Erfindung steht Ar² für ortho-, meta- oder para-Phenylen oder eine Biphenylgruppe, die jeweils durch einen oder mehrere Reste R² substituiert sein können, bevorzugt aber unsubstituiert sind.

Weiterhin kann es bevorzugt sein, wenn die Gruppen Ar-1 bis Ar-57 nicht direkt an das Stickstoffatom in Formel (1) bzw. Formel (2) bzw. Formel (3) binden, sondern über eine verbrückende Gruppe. Bevorzugte verbrückende Gruppen sind ortho-, meta- oder para-Phenylen oder eine Biphenylgruppe, die jeweils durch einen oder mehrere Reste R² substituiert sein können, bevorzugt aber unsubstituiert sind.

In einer bevorzugten Ausführungsform der Erfindung ist R² ausgewählt aus der Gruppe bestehend aus H, D oder einer Alkylgruppe mit 1 bis 10 C-Atomen, bevorzugt 1 bis 4 C-Atomen. Besonders bevorzugt ist R² gleich H.

In einer weiteren bevorzugten Ausführungsform der Erfindung steht Ar für eine Triarylamingruppe, welche durch einen oder mehrere Reste R¹ substituiert sein kann. Diese ist bevorzugt ausgewählt aus den Strukturen der folgenden Formel Ar-58, 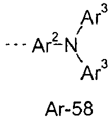 wobei die gestrichelte Bindung die Verknüpfung mit dem Stickstoffatom andeutet, Ar² die oben genannte Bedeutung aufweist und Ar³ gleich oder verschieden bei jedem Auftreten für ein aromatisches oder heteroaromatisches Ringsystem, bevorzugt mit 6 bis 18 aromatischen Ringatomen, steht, welches keine elektronenarmen Heteroarylgruppen enthält und welches jeweils mit einem oder mehreren Resten R² substituiert sein kann.

In einer bevorzugten Ausführungsform der Erfindung ist R¹ gleich oder verschieden bei jedem Auftreten ausgewählt aus der Gruppe bestehend aus H, D, N(Ar¹)₂, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R³ substituiert sein kann. Besonders bevorzugt ist R¹ gleich oder verschieden bei jedem Auftreten ausgewählt aus der Gruppe bestehend aus H, N(Ar¹)₂, einer geradkettigen Alkylgruppe mit 1 bis 4 C-Atomen oder einer verzweigten oder cyclischen Alkylgruppe mit 3 oder 4 C-Atomen, die jeweils mit einem oder mehreren Resten R³ substituiert sein kann, oder einem aromatischen oder heteroaromatischen Ringsystem mit 5 bis 18 aromatischen Ringatomen, das jeweils mit einem oder mehreren Resten R³ substituiert sein kann.

Dabei ist es bevorzugt, wenn die Gruppen Ar und R² zusammen weniger als 12 aromatische Ringatome aufweisen, wenn mindestens eine Gruppe R¹, bevorzugt genau eine Gruppe R¹ für eine Gruppe N(Ar¹)₂ oder für ein aromatisches oder heteroaromatisches Ringsystem stehen.

Dabei ist in Verbindungen der Formel (1) bzw. den bevorzugten Ausführungsformen (7) bis (13), (7a) bis (13a) und (7b) bis (13b) jeweils bevorzugt maximal eine der Gruppen R¹ ungleich H und die anderen Gruppen R¹ sind gleich H. Weiterhin ist in Verbindungen der Formel (2) und (3) bzw. den bevorzugten Ausführungsformen (14), (15), (14a), (14b), (15a) und (15b) jeweils bevorzugt maximal eine der Gruppen R¹, die an denselben Cyclus gebunden sind, ungleich H und die anderen Gruppen R¹, die an denselben Cyclus gebunden sind, sind gleich H. Es kann auch bevorzugt sein, wenn alle Gruppen R¹ gleich H sind.

In einer bevorzugten Ausführungsform der Formel (1) bzw. den bevorzugten Ausführungsformen steht genau einer der Substituenten R¹ für eine Gruppe N(Ar¹)₂ oder für ein aromatisches oder heteroaromatisches Ringsystem mit 5 bis 24 aromatischen Ringatomen, das jeweils mit einem oder mehreren Resten R² substituiert sein kann, und die anderen Substituenten R¹ stehen für H. In einer bevorzugten Ausführungsform der Formel (2) und (3) bzw. den bevorzugten Ausführungsformen steht genau einer der Substituenten R¹, die an denselben Cyclus gebunden sind, für eine Gruppe N(Ar¹)₂ oder für ein aromatisches oder heteroaromatisches Ringsystem mit 5 bis 24 aromatischen Ringatomen, das jeweils mit einem oder mehreren Resten R² substituiert sein kann, und die anderen Substituenten R¹ stehen für H.

Wenn alle Reste R¹ für H oder D oder Alkyl stehen, ist es bevorzugt, wenn die Gruppe Ar für ein aromatisches oder heteroaromatisches Ringsystem mit mindestens 12 aromatischen Ringatomen steht.

Wenn R¹ für ein aromatisches oder heteroaromatisches Ringsystem steht, dann sind die Gruppen R¹ bevorzugt ausgewählt aus den Gruppen R¹-1 bis R¹-42, 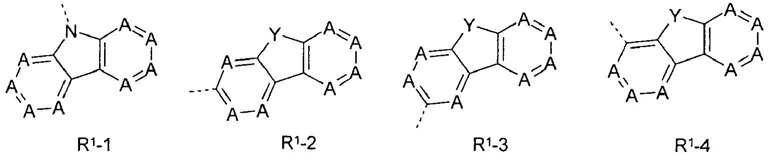 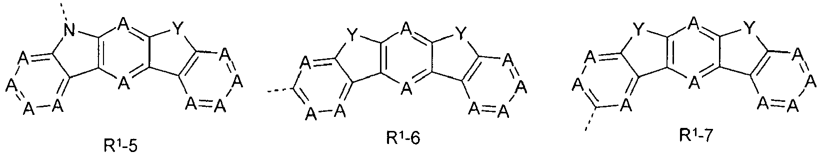 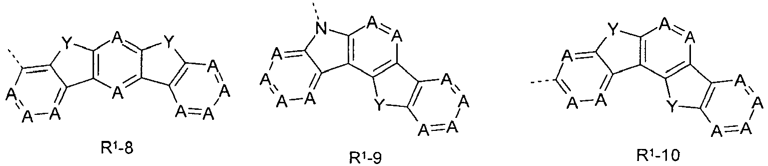 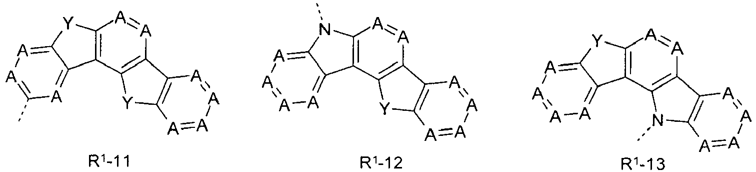 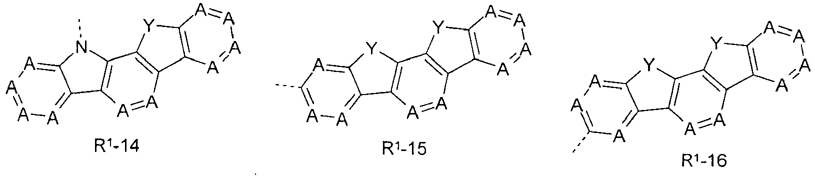 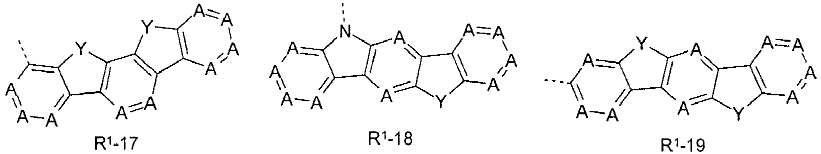 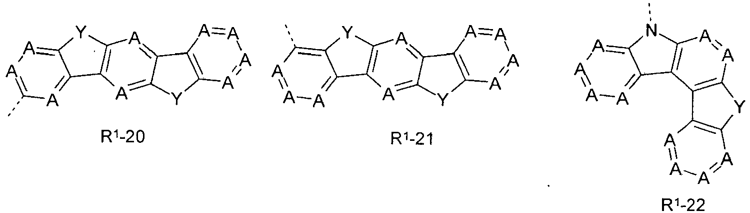 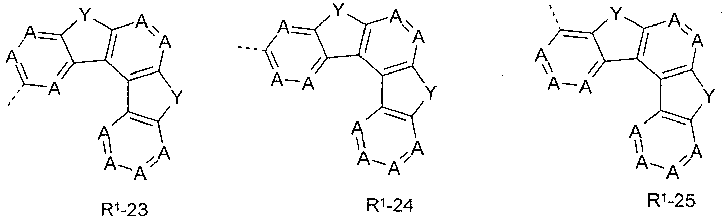 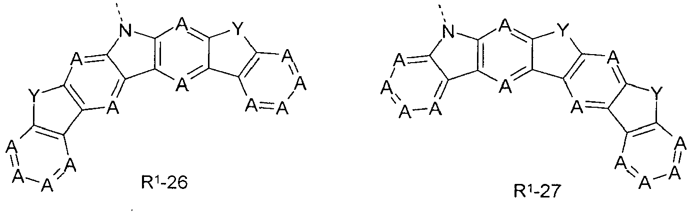 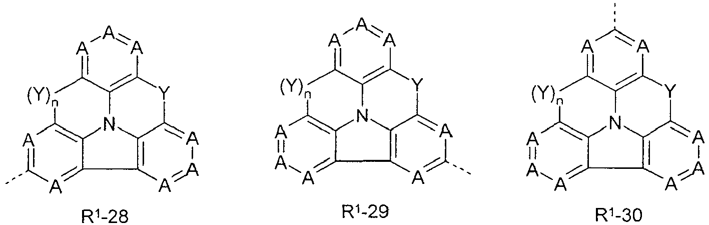 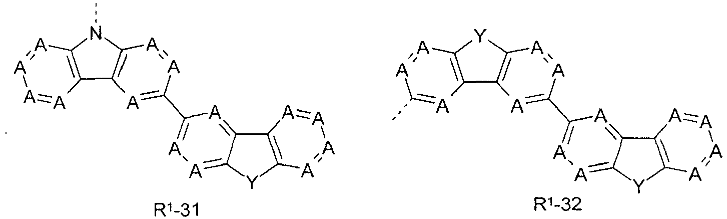 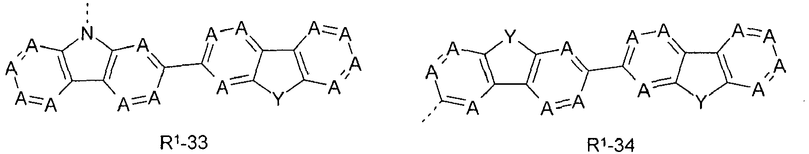 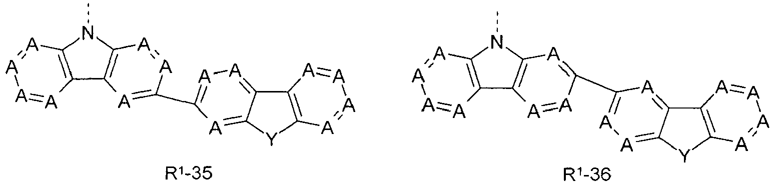 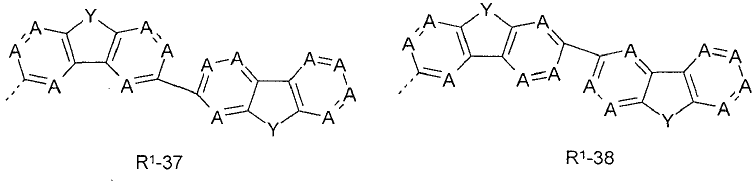 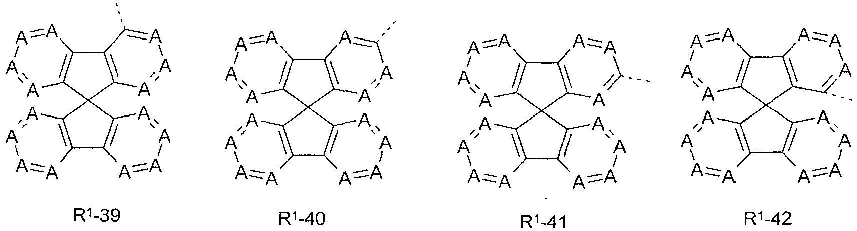 wobei R³ die oben genannten Bedeutungen aufweist, die gestrichelte Bindung die Bindung an die Gruppe der Formel (1) bzw. (2) bzw. (3) darstellt und weiterhin gilt:
- A: ist bei jedem Auftreten gleich oder verschieden CR³ oder N, wobei maximal 3 Symbole X pro Cyclus für N stehen;
- Y: ist bei jedem Auftreten gleich oder verschieden C(R³)₂, NR³, O oder S;
- n: ist 0 oder 1, wobei n gleich 0 bedeutet, dass an dieser Position keine Gruppe Y gebunden ist und an den entsprechenden Kohlenstoffatomen statt dessen Reste R³ gebunden sind.

Der oben genannte und auch im Folgenden verwendete Begriff "pro Cyclus" bezieht sich im Sinne der vorliegenden Anmeldung auf jeden einzelnen in der Verbindung enthaltenen Ring, also auf jeden einzelnen 5-bzw. 6-Ring.

In bevorzugten Gruppen der oben genannten Formeln R¹-1 bis R¹-42 steht maximal ein Symbol A pro Cyclus für N. Besonders bevorzugt steht das Symbol A gleich oder verschieden bei jedem Auftreten für CR³, insbesondere für CH.

Wenn die Gruppen der Formeln R¹-1 bis R¹-42 mehrere Gruppen Y aufweisen, so kommen hierfür alle Kombinationen aus der Definition von Y in Frage. Bevorzugt sind Gruppen, in denen eine Gruppe Y für NR³ und die andere Gruppe Y für C(R³)₂ steht oder in denen beide Gruppen Y für NR³ stehen oder in denen beide Gruppen Y für O stehen.

Wenn Y in den Formeln R¹-1 bis R¹-42 für NR³ steht, steht der Substituent R³, der an das Stickstoffatom gebunden ist, bevorzugt für ein aromatisches oder heteroaromatisches Ringsystem mit 5 bis 24 aromatischen Ringatomen. In einer besonders bevorzugten Ausführungsform steht dieser Substituent R³ gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in welchen zwei oder mehr aromatische bzw. heteroaromatische 6-Ring-Gruppen direkt aneinander ankondensiert sind, aufweist. Besonders bevorzugt sind Phenyl, Biphenyl, Terphenyl und Quaterphenyl mit Verknüpfungsmustern, wie vorne für Ar-1 bis Ar-11 aufgeführt, wobei diese Strukturen bevorzugt unsubstituiert sind.

Wenn Y in den Formeln R¹-1 bis R¹-42 für C(R³)₂ steht, steht R³ bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen. Ganz besonders bevorzugt steht R³ für eine Methylgruppe oder für eine Phenylgruppe. Dabei können die Reste R³ auch miteinander ein Ringsystem bilden, was zu einem Spirosystem führt.

Weiterhin kann es bevorzugt sein, wenn die Gruppen R¹-1 bis R¹-42 nicht direkt an das Stickstoffatom in Formel (1) bzw. Formel (2) bzw. Formel (3) binden, sondern über eine verbrückende Gruppe. Bevorzugte verbrückende Gruppen sind ortho-, meta- oder para-Phenylen oder eine Biphenylgruppe, die jeweils durch einen oder mehrere Reste R³ substituiert sein können, bevorzugt aber unsubstituiert sind.

In einer weiteren bevorzugten Ausführungsform der Erfindung steht R¹ für eine Triarylamingruppe, welche durch einen oder mehrere Reste R¹ substituiert sein kann. Diese ist bevorzugt ausgewählt aus den Strukturen der folgenden Formel R¹-43, 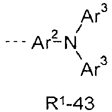 wobei die gestrichelte Bindung die Verknüpfung mit dem Kohlenstoffatom des Grundgerüsts andeutet, und Ar² und Ar³ für ein aromatisches oder heteroaromatisches Ringsystem mit 5 bis 14 aromatischen Ringatomen steht, welches jeweils mit einem oder mehreren Resten R³ substituiert sein kann.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Wenn die Verbindungen der Formel (1) bzw. Formel (2) bzw. Formel (3) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, wenn die Reste R, R¹, R² und Ar keine kondensierte Aryl- bzw. Heteroarylgruppe enthalten, in der zwei oder mehr Sechsringe direkt aneinander ankondensiert sind.

Beispiele für geeignete Verbindungen gemäß den oben aufgeführten Ausführungsformen sind die in der folgenden Tabelle aufgeführten Verbindungen.

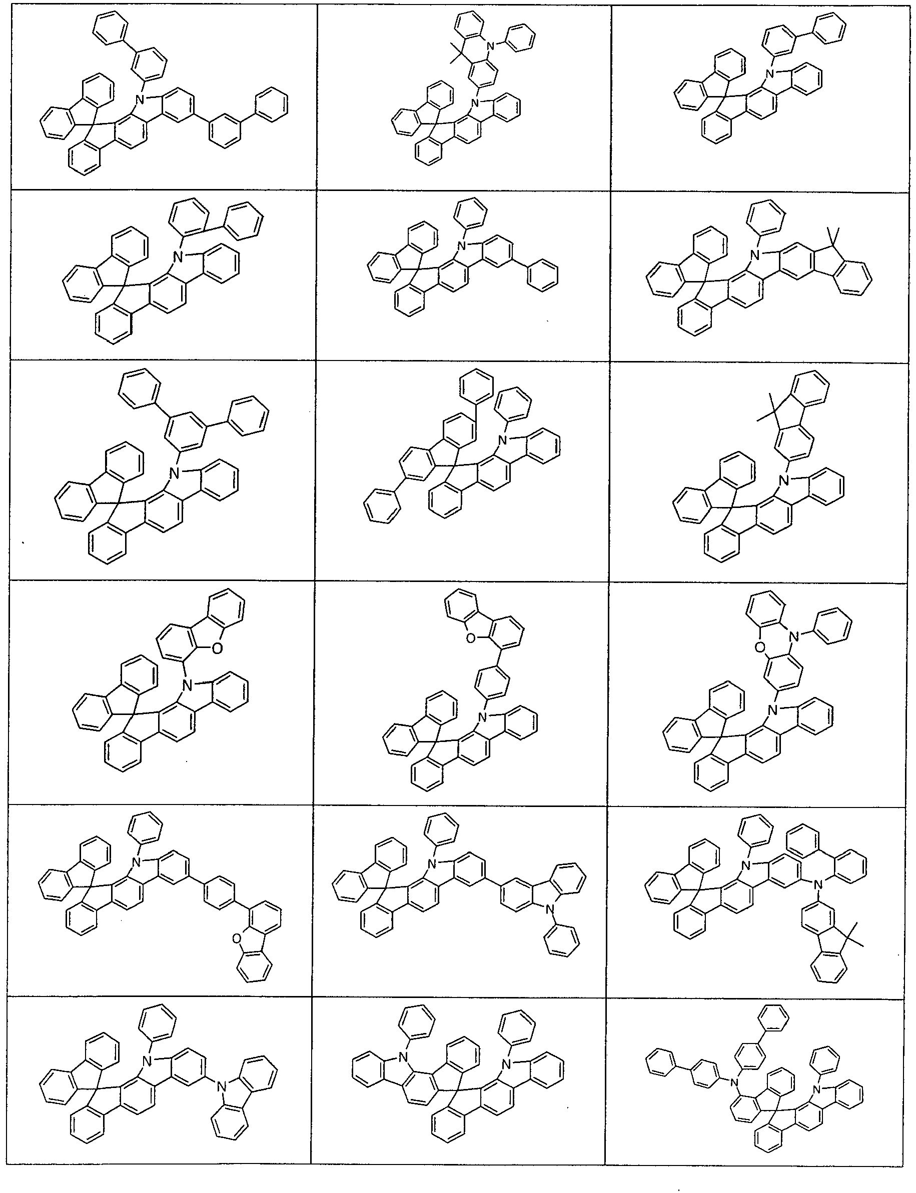

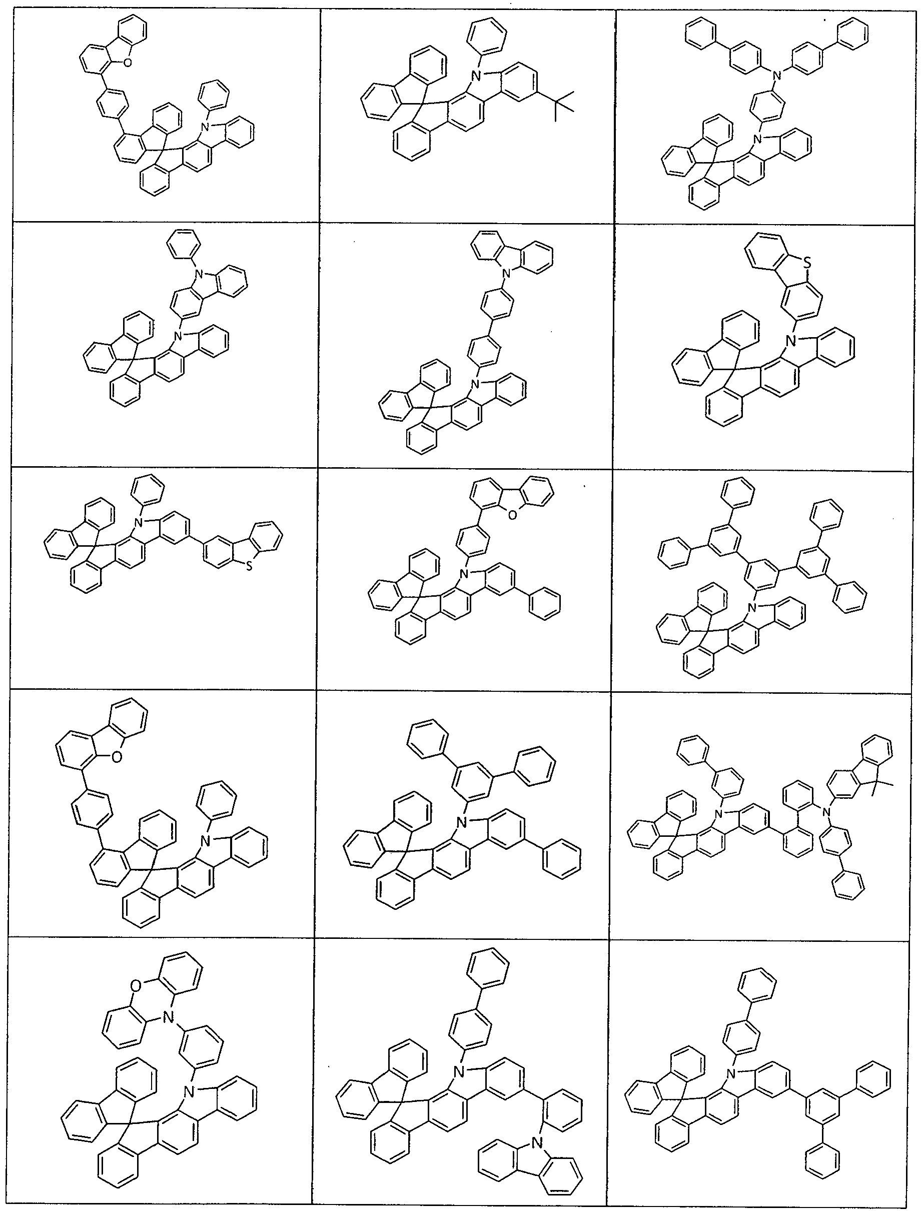

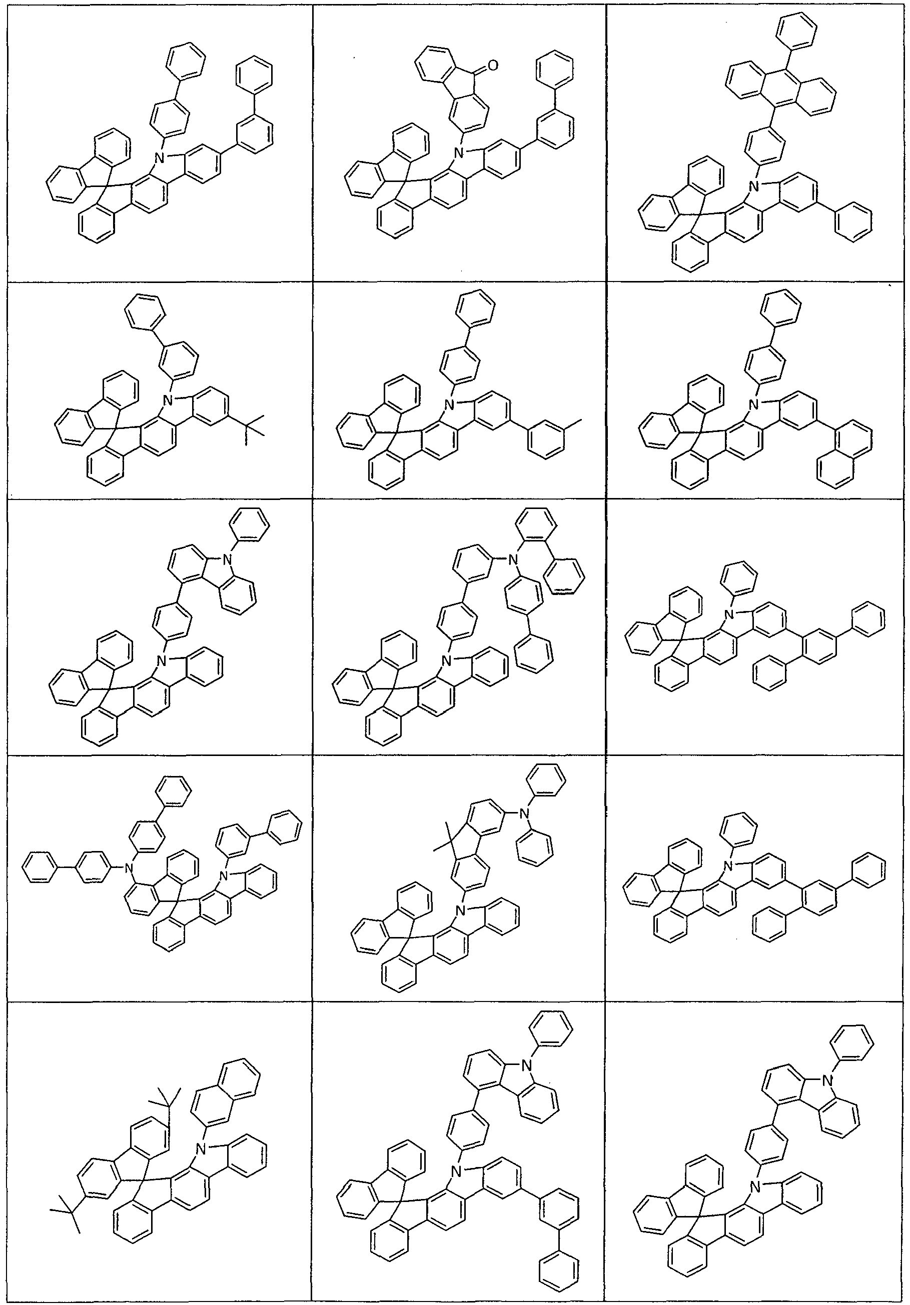

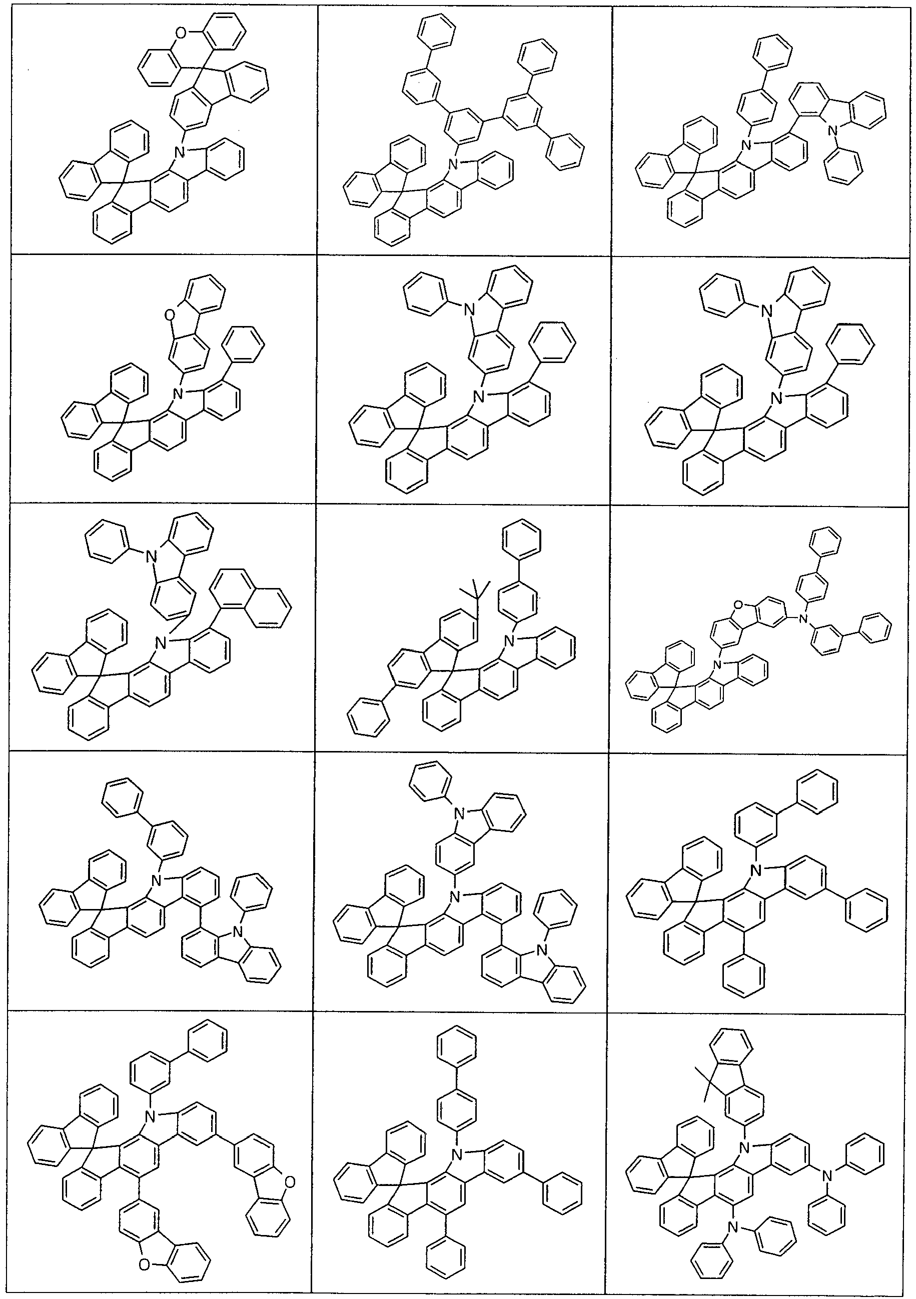

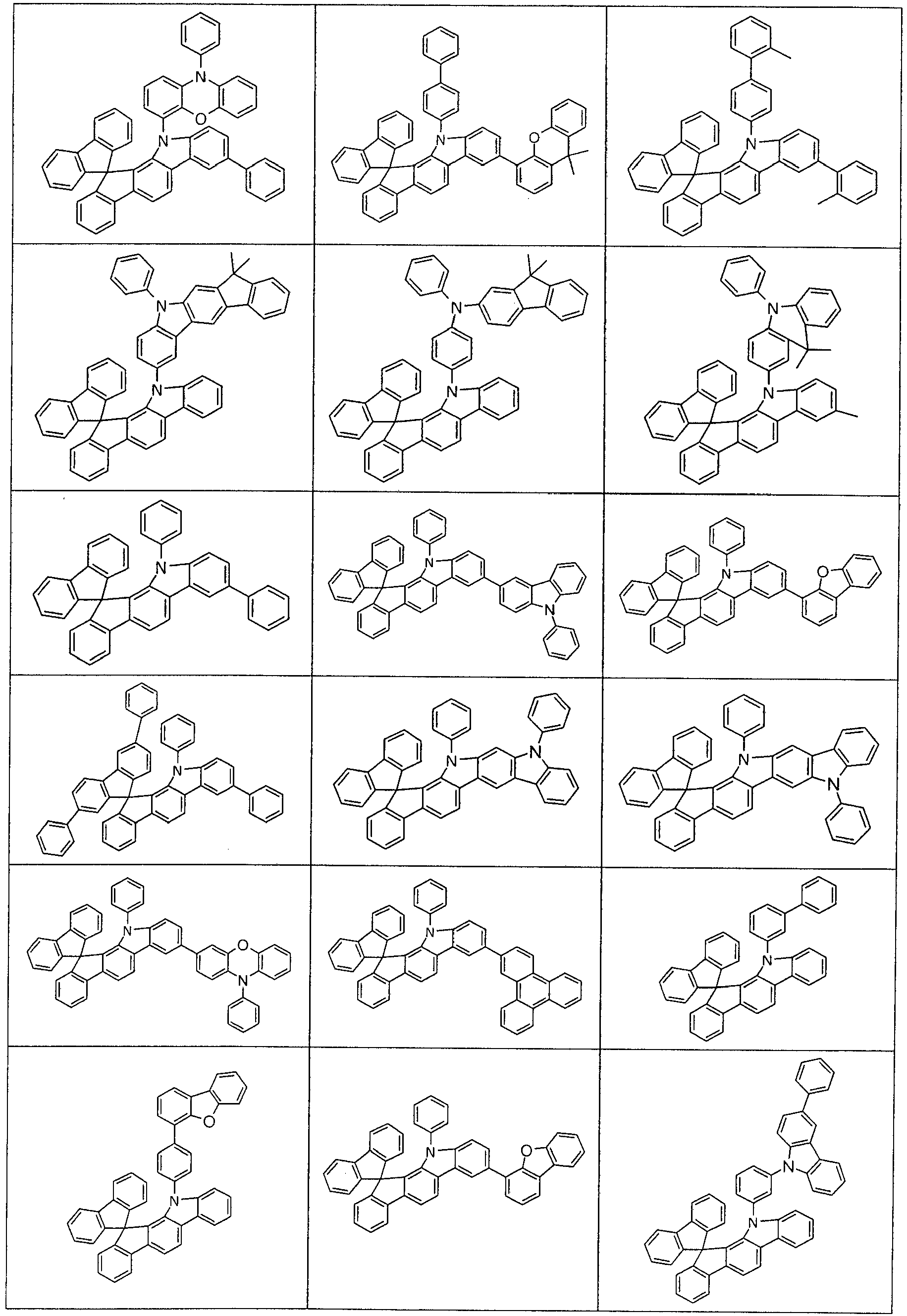

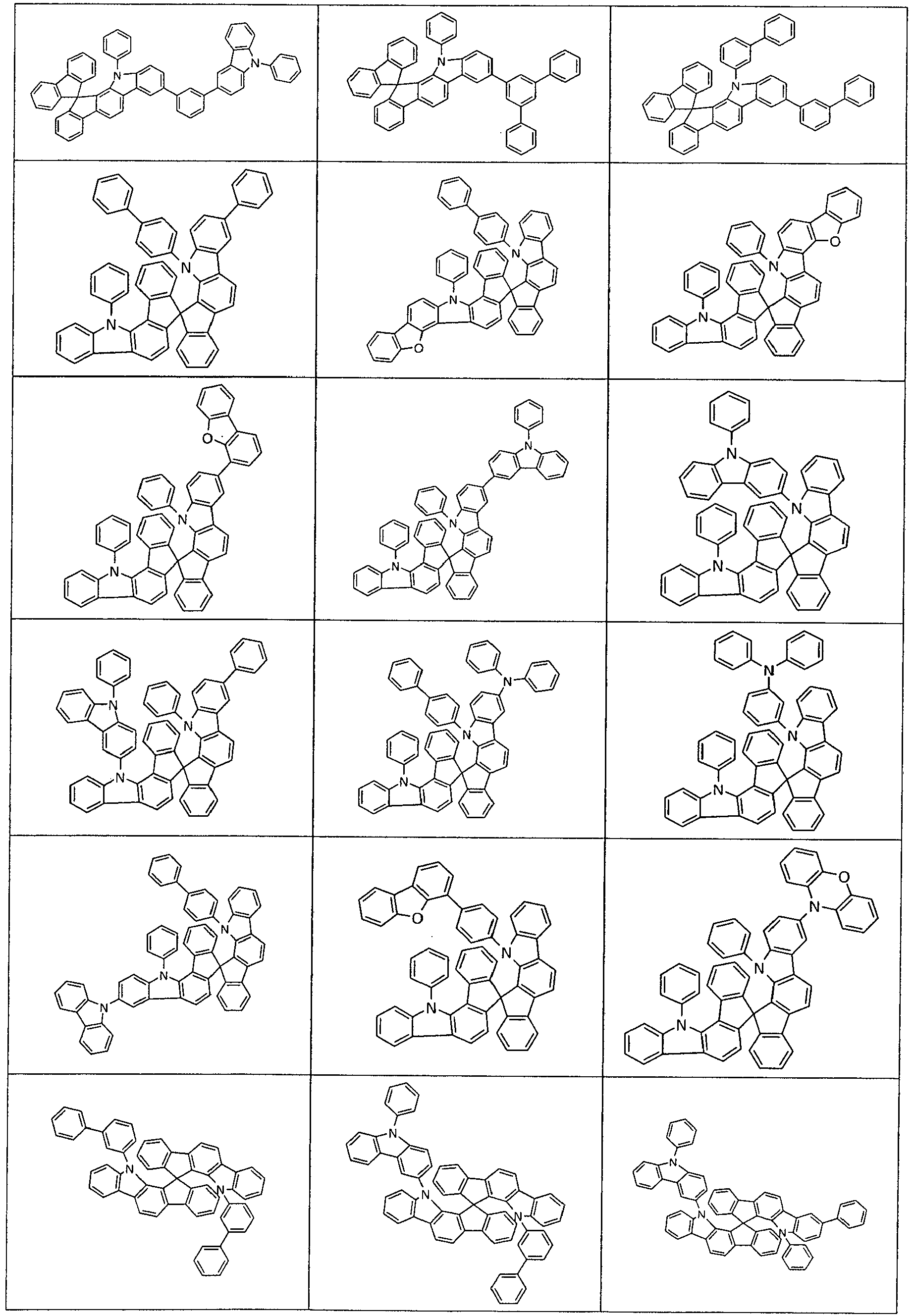

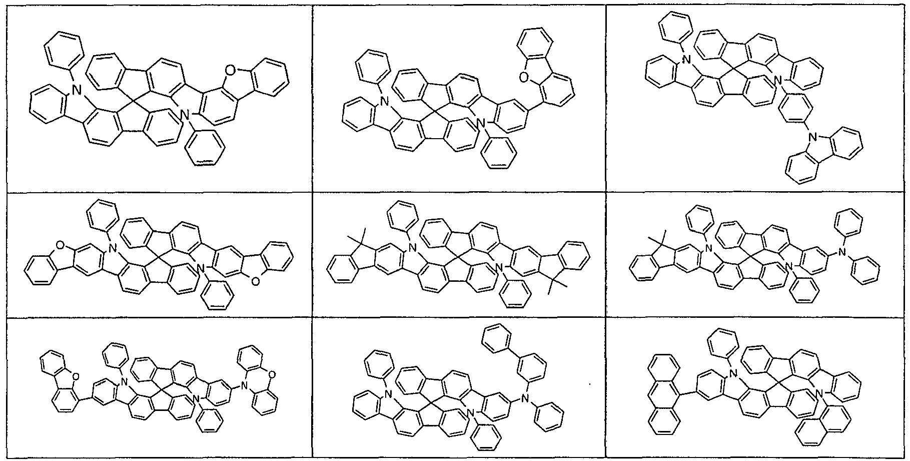

Die erfindungsgemäßen Verbindungen der Formel (1) können nach dem in Schema 1 skizzierten Weg dargestellt werden.

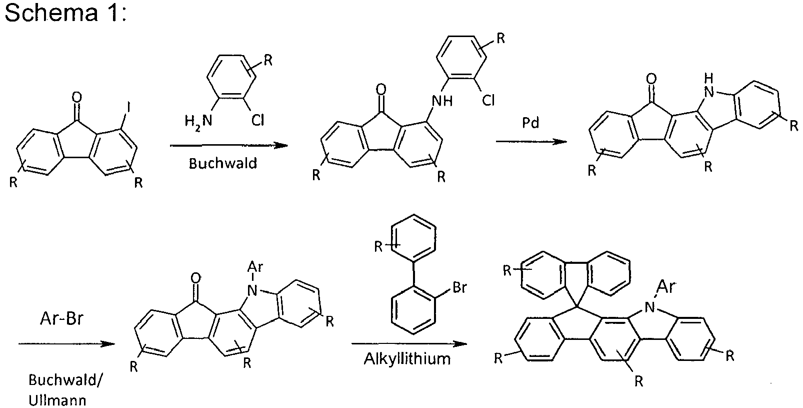

Dabei kann die Synthese ausgehend vom einem gegebenenfalls substituierten 1-Halogenfluorenon durchgeführt werden, wobei das Halogen bevorzugt Br oder I ist. Dieses wird mit einem gegebenenfalls substituierten ortho-Halogenaminobenzol in einer C-N-Kupplungsreaktion, beispielsweise unter Pd- oder Cu-Katalyse, umgesetzt, wobei das Halogen bevorzugt Cl, Br oder I ist. Ganz analog kann beispielsweise ein Fluoren-, Dibenzofuran-, Dibenzothiophen oder Naphthalinderivat eingesetzt werden, wodurch man zu Verbindungen gelangt, welche Gruppen der Formel (4) bzw. (5) enthalten. Der Ringschluss zum entsprechenden Carbazolderivat erfolgt durch eine intramolekulare Pd-katalysierte Kupplungsreaktion. Dieses Carbazolderivat kann durch eine Buchwald- oder Ullmann-Kupplungsreaktion am Stickstoff durch die Gruppe Ar substituiert werden. Im letzten Schritt erfolgt dann die Ringschlussreaktion zum Spirobifluorenderivat durch Umsetzung mit einem lithiierten Biphenylderivat, gefolgt von saurer Cyclisierung.

Die Synthese von Verbindungen der Formel (3) ist in Schema 2 dargestellt und kann ausgehend von dem Indolofluorenon erfolgen, das gemäß Schema 1 synthetisiert werden kann. Dabei wird zur Bildung der Spiroverbindung ein 2,2'-Dibrombiphenylderivat eingesetzt, so dass die erhaltene Spiroverbindung in 4'-Position ein Bromatom aufweist. Ausgehend hiervon kann analog zu Schema 1 die ankondensierte Indologruppe synthetisiert werden.

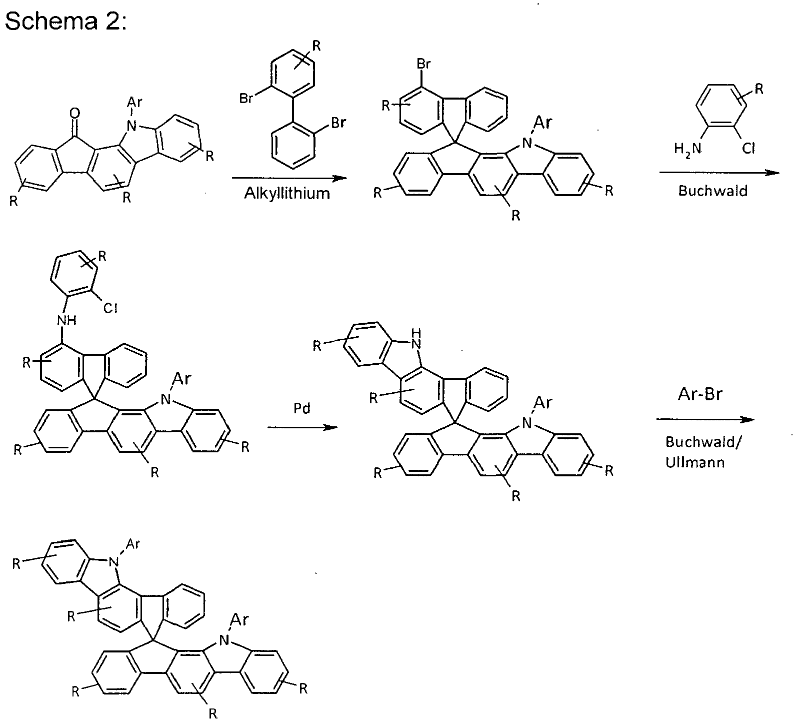

Die Synthese von Verbindungen der Formel (2) kann analog erfolgen.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen, Styrolen, Acrylaten oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei an einer oder mehreren Positionen statt Substituenten eine oder mehrere Bindungen der erfindungsgemäßen Verbindung zum Polymer, Oligomer oder Dendrimer vorhanden sind. Je nach Verknüpfung der erfindungsgemäßen Verbindung bildet diese eine Seitenkette des Oligomers oder Polymers oder ist in der Hauptkette verknüpft oder bildet den Kern eines Dendrimers. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. (2) bzw. (3) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt TriplettEmitter enthalten. Gerade die Kombination von den erfindungsgemäßen Oligomere, Polymeren oder Dendrimeren mit Triplett-Emittern führt zu besonders guten Ergebnissen.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-lsopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. Formel (2) bzw. Formel (3) bzw. die oben ausgeführten bevorzugten Ausführungsformen als Matrixmaterial für phosphoreszierende oder fluoreszierende Emitter, insbesondere für phosphoreszierende Emitter, und/oder in einer elektronenblockierenden bzw. exzitonenblockierenden Schicht und/oder in einer Lochtransportschicht und/oder in einer Lochblockierschicht und/oder in einer Lochblockier- oder Elektronentransportschicht, je nach genauer Substitution.

In einer bevorzugten Ausführungsform der Erfindung wird die erfindungsgemäße Verbindung als Matrixmaterial für eine phosphoreszierende Verbindung in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/157339, WO 2012/007086, WO 2012/163471, WO 2013/000531 und WO 2013/020631, WO 2014/008982 und WO 2014/023377 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Die erfindungsgemäßen Verbindungen sind insbesondere auch geeignet als Matrixmaterialien für phosphoreszierende Emitter in organischen Elektrolumineszenzvorrichtungen, wie sie z. B. in US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren Ausführungsform der Erfindung wird die erfindungsgemäße Verbindung in einer Lochtransportschicht bzw. in einer Elektronenblockierschicht bzw. Exzitonenblockierschicht eingesetzt.

In nochmals einer weiteren bevorzugten Ausführungsform der Erfindung wird die erfindungsgemäße Verbindung als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die erfindungsgemäße Verbindung in einer Lochblockierschicht eingesetzt. Unter einer Lochblockierschicht wird eine Schicht verstanden, die auf Kathodenseite direkt an eine emittierende Schicht angrenzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. Formel (2) bzw. Formel (3) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für fluoreszierende oder phosphoreszierende Emitter, führen zu langen Lebensdauern. Dies gilt insbesondere, wenn die Verbindungen als Matrixmaterial für einen phosphoreszierenden Emitter eingesetzt werden.
2. Die erfindungsgemäßen Verbindungen führen zu sehr niedrigen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### Synthesebeispiele

### Beispiet 1a: 1-(2-Chlorphenylamin)-fluoren-9-on

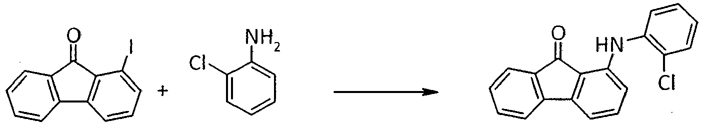

In einem 1-L Vierhalskolben werden 52 g (166 mmol) 1-lodfluoren-9-on (CAS 52086-21-2), 19.0 mL (171 mmol) 2-Chloranilin (CAS 95-51-2), 59.8 g (432 mmol) Kaliumcarbonat, 3.85 g (6.6 mmol) XantPhos und 746 mg (3.3 mmol) Palladiumdiacetat in 390 mL Toluol gelöst und 13 h bis zum vollständigen Umsatz unter Rückfluss erhitzt. Nach Abkühlen auf Raumtemperatur wird die organische Phase mit 200 mL Toluol erweitert und mit 500 mL Wasser hydrolysiert. Die organische Phase wird einmal mit 300 mL Wasser und zweimal mit je 200 mL 3M HCl-Lösung gewaschen. Die organische Phase wird über Al₂O₃ filtriert. Nach dem Entfernen der Lösungsmittel unter vermindertem Druck wird das Produkt als orangefarbener Feststoff erhalten. Die Ausbeute beträgt 48.0 g (157 mmol, entspr. 95 %)

Analog können folgende Verbindungen hergestellt werden:

| | Edukt 1 | Edukt 2 | Produkt | Ausbeute |
|---|---|---|---|---|
| 1b | 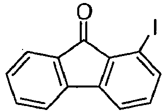 | 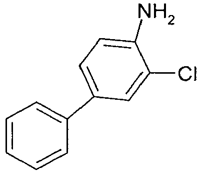 | 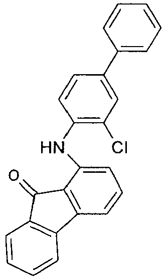 | 83% |
| | | 7285-66-7 | | |
| 1c | 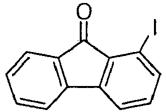 | 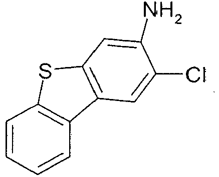 | 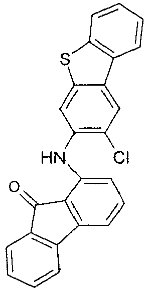 | 78% |
| | | 858426-71-8 | | |
| 1d | 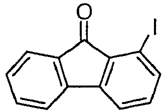 | 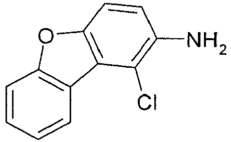 | 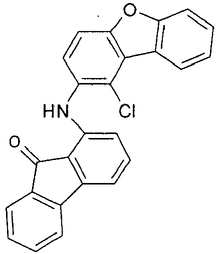 | 67% |
| | | 133617-97-7 | | |
| 1e | 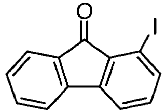 | 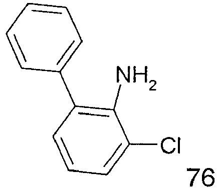 | 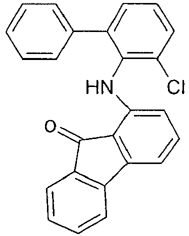 | 53% |
| | | 838-82-9 | | |
| 1f | 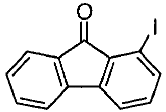 | 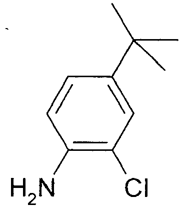 | 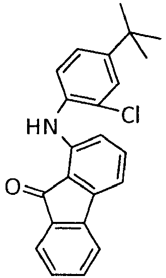 | 73% |
| | | 42265-67-8 | | |
| 1g | 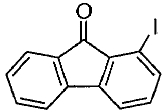 | 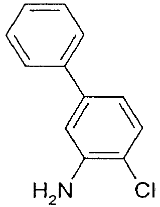 | 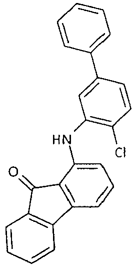 | 68% |
| | | 57013-94-2 | | |
| 1h | 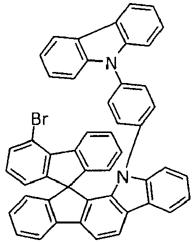 | 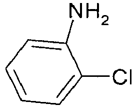 | 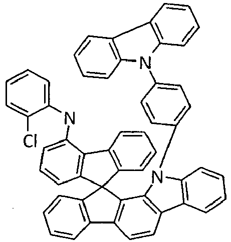 | 70% |
| | aus Bsp. 7q | 95-51-2 | | |
| 1i | 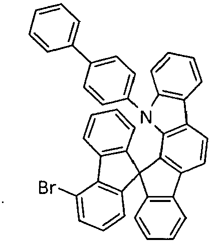 | 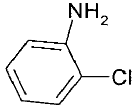 | 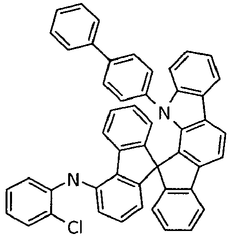 | 72% |
| | aus Bsp. 7p | 95-51-2 | | |

### Beispiel 2a: 11H-11-Aza-indeno[2,1-a]fluoren-12-on

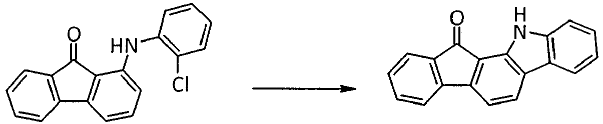

In einem 500 mL Vierhalskolben werden 24 g (78 mmol) **1a,** 28.2 g (204 mmol) Kaliumcarbonat, 35 mg (1.5 mmol) Palladiumdiacetat, 1.2 g (3.0 mmol) Tricyclohexylphosphintetrafluoroborat in 250 mL DMAc vorgelegt und drei Tage bei 145 °C gerührt. Nach vollständigem Umsatz wird auf Raumtemperatur abgekühlt und mit 200 mL Wasser hydrolysiert. Der ausgefallene Feststoff wird filtriert und mit Wasser(2x300 mL) gewaschen. Nach zweimaligem Ausrühren mit Ethanol bei 60 °C wird das Produkt als ockerfarbener Feststoff erhalten. Die Ausbeute beträgt 18.3 g (68 mmol, entspr. 87 % d.Th)

Analog können folgende Verbindungen hergestellt werden:

| | Edukt | Produkt | Ausbeute |
|---|---|---|---|
| 2b | 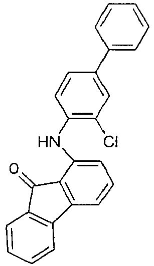 | 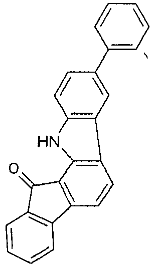 | 72% |
| 2c | 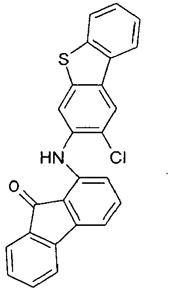 | 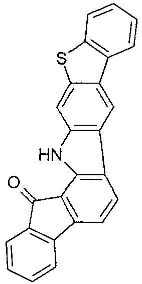 | 73% |
| 2d | 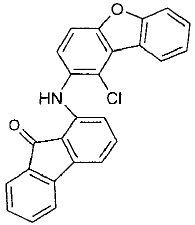 | 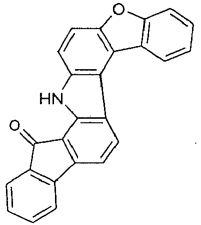 | 67% |
| 2e | 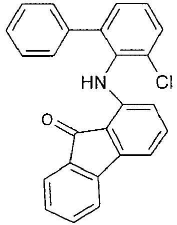 | 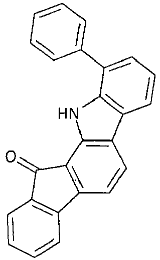 | 56% |
| 2f | 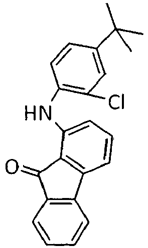 | 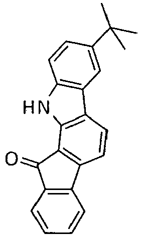 | 71% |
| 2g | 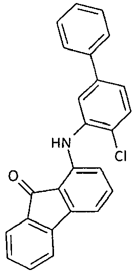 | 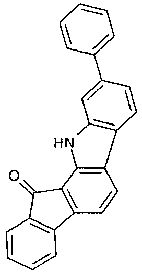 | 67% |
| 2h | 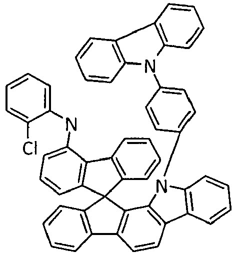 | 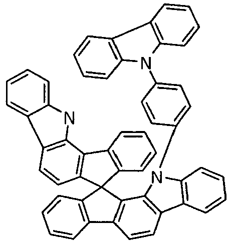 | 65% |
| 2i | 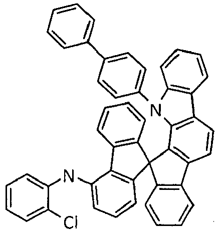 | 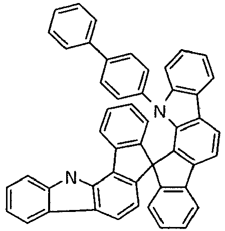 | 68% |

### Beispiel 3a: 11-Biphenyl-4-yl-11H-11-aza-indeno[2,1-a]fluoren-12-on

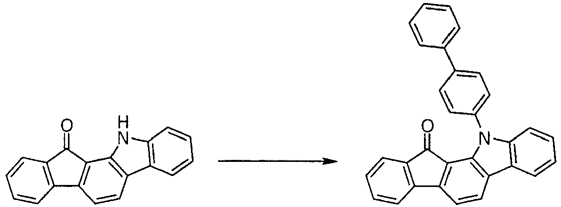

In einem 1-L-Vierhalskolben werden 16 g (59 mmol) **2a,** 29.6 mL (119 mmol) 4-Brombiphenyl und 30.5 g NaOtBu (32 mmol) in *p*-Xylol vorgelegt. Zu dieser Suspension werden 0.33 g (1.5 mmol) Pd(OAc)₂ und 2.9 ml einer 1 M Tri-tert-butylphosphin-Lösung in Toluol gegeben. Die Reaktionsmischung wird 16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert und aus Toluol umkristallisiert. Die Ausbeute beträgt 22.3 g (53 mmol, entsprechend 90 %).

Analog können folgende Verbindungen hergestellt werden:

| | Edukt1 | Edukt 2 | Produkt | Ausbeute |
|---|---|---|---|---|
| 3b | 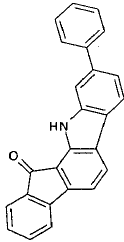 | 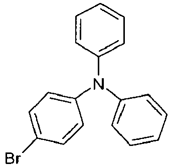 | 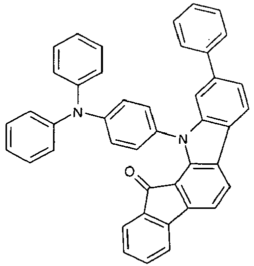 | 83% |
| | | [36809-26-4] | | |
| 3c | 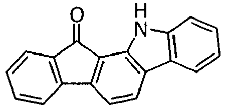 | 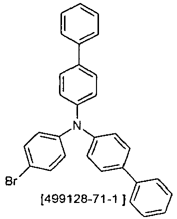 | 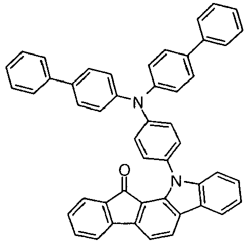 | 83% |
| 3d | 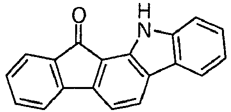 | 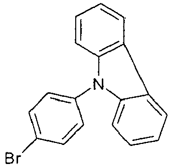 | 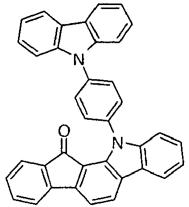 | 82% |
| | | [57102-42-8] | | |
| 3e | 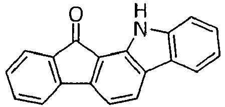 | 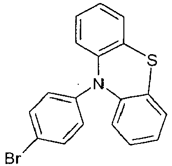 | 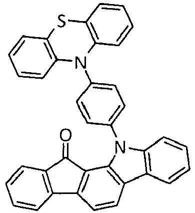 | 77% |
| | | [63524-03-8] | | |
| 3f | 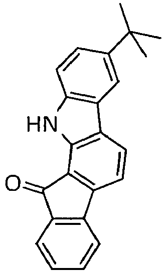 | 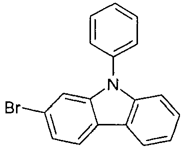 | 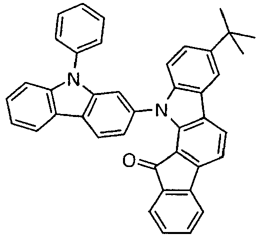 | 90% |
| | | [94994-62-4] | | |
| 3g | 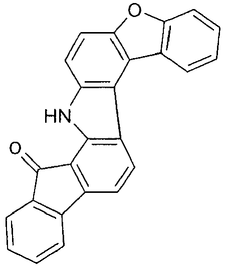 | 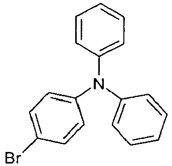 | 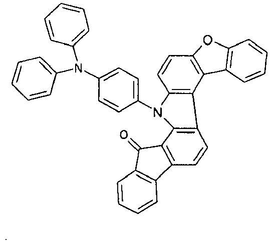 | 78% |
| | | [36809-26-4] | | |
| 3h | 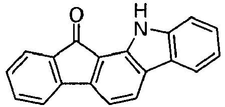 | 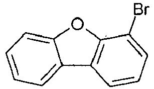 | 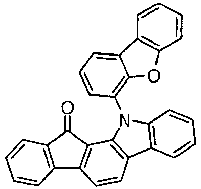 | 88% |
| | | [89827-45-2] | | |
| 3i | 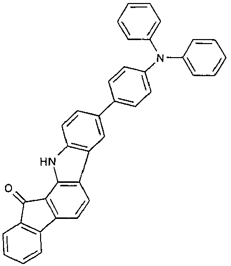 | 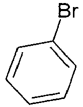 | 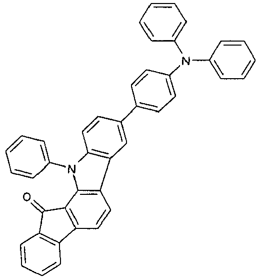 | 83% |
| 3j | 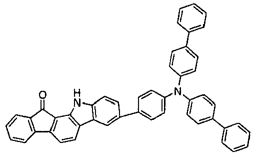 | 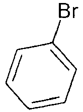 | 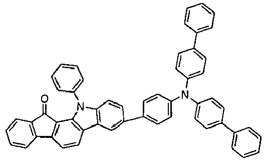 | 84% |
| 3k | 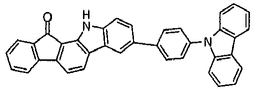 | 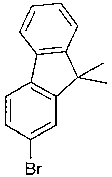 | 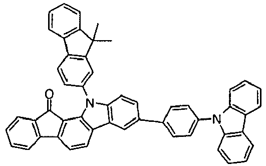 | 81% |
| | | 28320-31-2 | | |
| 3l | 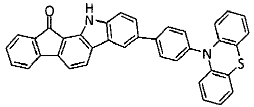 | 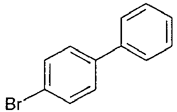 | 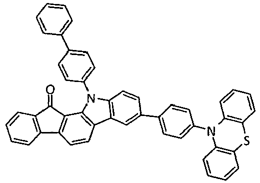 | 86% |
| | | 92-66-0 | | |
| 3m | 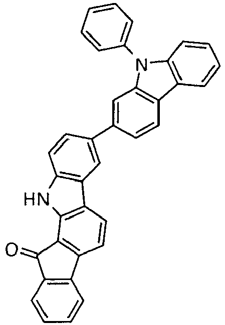 | 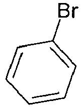 | 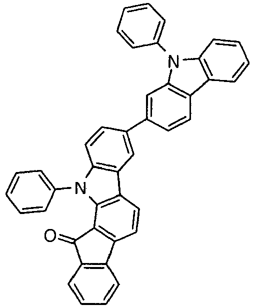 | 78% |
| 3n | 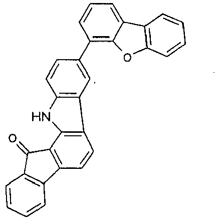 | 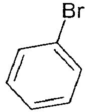 | 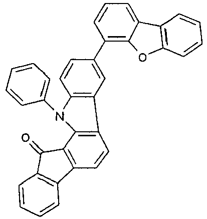 | 76% |
| 3o | 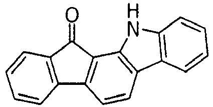 | 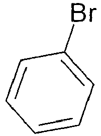 | 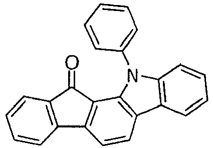 | 91% |
| 3p | 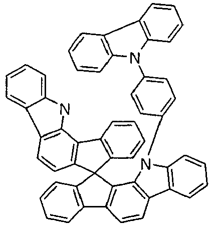 | 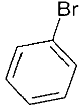 | 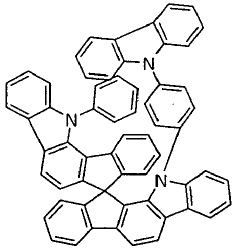 | 21%* |
| 3q | 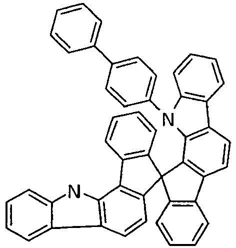 | 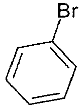 | 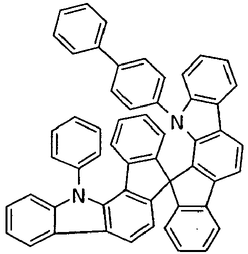 | 38%* |

| | | | | |
|---|---|---|---|---|
| * Weitere Aufreinigung mittels Umkristallisation aus Toluol / Heptan und Zonensublimation (340 °C, 10⁻⁵ bar) bis zu einer HPLC-Reinheit >99.9%. | | | | |

### Beispiel 4a: Bromierung

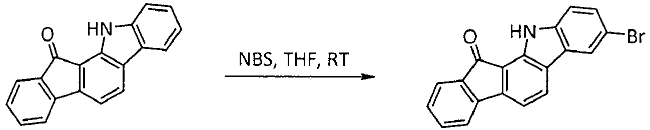

In einem 1000 mL Vierhalskolben werden 19.5 g (72.5 mmol) 11H-11-Aza-indeno[2,1-a]fluoren-12-on und 13.5 g (75 mmol) NBS in 700 mL THF gelöst und 48 h bei Raumtemperatur bis zur vollständigen Umsetzung gerührt. Anschließend werden mit 50 mL Wasser hydrolysiert und die organischen Lösemittel unter vermindertem Druck entfernt. Der erhaltene Feststoff wird einmal mit 300 mL Ethanol heiß ausgerührt. Nach Abkühlen auf Raumtemperatur wird der Feststoff filtriert. Nach Trocknung unter vermindertem Druck wird das Produkt als farbloser Feststoff erhalten. Die Ausbeute beträgt 22.6 g (65 mmol, entspr. 90 % d.Th).

### Beispiel 5a: Borylierung

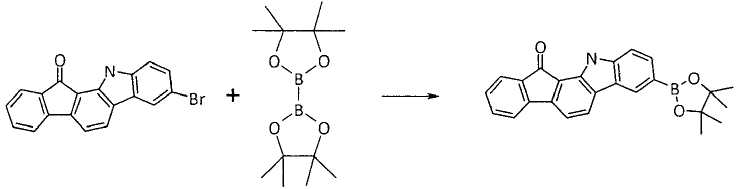

In einem 2-L-Vierhalskoben werden 24.7 g (71 mmol) 8-Brom-11H-11-aza-indeno[2,1-a]fluoren-12-on, 21.9 g (86 mmol) Bispinacolatodiboran (73183-34-3), 21.7 g (221 mmol) Kaliumacetat und 1.7 g (2.1 mmol) 1,1-Bis(diphenylphosphino)ferrocen-dichloropalladium(II)-Komplex mit DCM in 1000 mL wasserfreiem Dioxan 16 h bis zum vollständigen Umsatz unter Rückfluss erhitzt. Nach Abkühlen auf Raumtemperatur wird die organische Phase mit Ethylacetat erweitert, dreimal mit 300 mL Wasser gewaschen und über Natriumsulfat getrocknet. Die vereinigten organischen Phasen werden bis zur Trockene einrotiert. Nach Umkristallisation aus Heptan wird das Produkt als Feststoff erhalten. Die Ausbeute beträgt 21.3 g(54 mmol; 61 %).

### Beispiel 6a

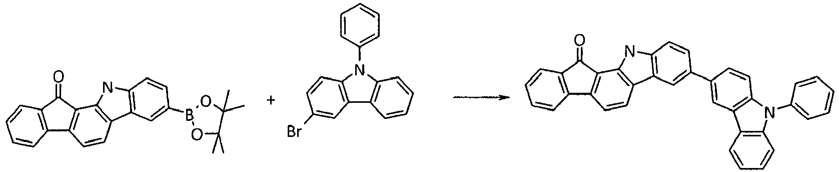

12.6 g (32 mmol) 8-(4,4,5,5-Tetramethyl-[1,3,2]dioxaborolan-2-yl)-11H-11-aza-indeno[2,1-a]fluoren-12-on, 10.3 g (32 mmol) 3-Brom-9-phenyl-9H-carbazol [1153-85-1] und 31 ml (63 mmol) Na₂CO₃ (2 M-Lösung) werden in 120 mL Toulol und 120 mL Ethanol suspendiert. Zu dieser Suspension werden 0.73 g (0.63 mmol) Pd(PPh₃)₄ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 14,8 g (29 mmol), entsprechend 91 % der Theorie.

Analog können folgende Verbindungen hergestellt werden:

| | Edukt 1 | Edukt 2 | Produkt | Ausbeute |
|---|---|---|---|---|
| 6b | 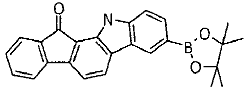 | 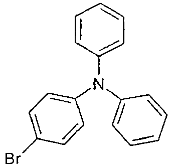 | 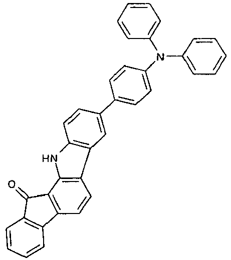 | 83% |
| | | [36809-26-4] | | |
| 6c | 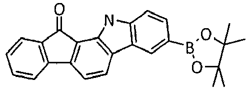 | 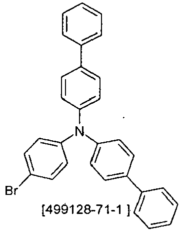 | 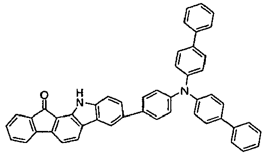 | 83% |
| 6d | 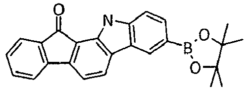 | 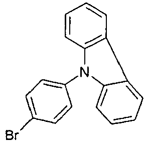 [57102-42-8] | 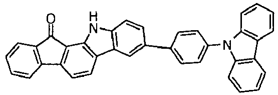 | 82% |
| 6e | 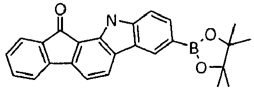 | 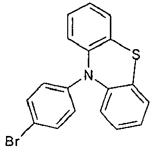 | 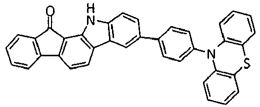 | 77% |
| | | [63524-03-8] | | |
| 6f | 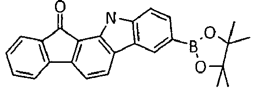 | 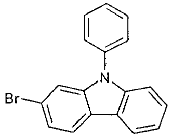 | 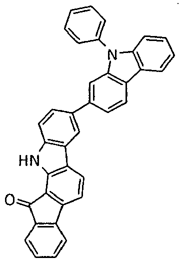 | 90% |
| | | [94994-62-4] | | |
| 6g | 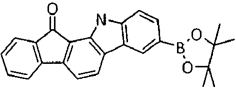 | 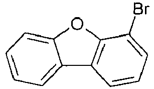 | 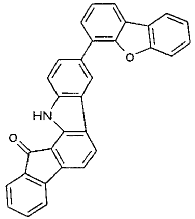 | 78% |
| | | [89827-45-2] | | |

### Beispiel 7a: 12'-Biphenyl-4yl-spiro[9H-fluoren-9,11(12H)-indeno[2,1a]-carbazol]'

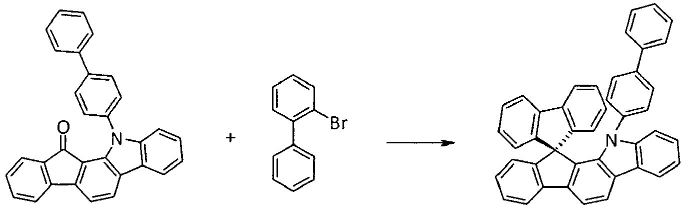

In einem 1-L-Vierhalskolben werden 23 g (99 mmol) 2-Brombiphenyl in 100 mL THF vorgelegt und auf -78 °C gekühlt. Über einen Tropftrichter werden 41.0 mL (103 mmol) *n*-Butyllithium (2.5 M in *n*-Hexan) bei dieser Temperatur hinzu getropft und 1 h gerührt. Anschließend werden 20.6 g (49 mmol) 11-Biphenyl-4-yl-11H-11-aza-indeno[2,1-a]fluoren-12-on, gelöst in 300 mL THF, über einen Tropftrichter hinzu gegeben und innerhalb von 3 h wird die Mischung auf Raumtemperatur erwärmt. Daraufhin wird mit 500 mL Wasser hydrolysiert und die organischen Lösungsmittel am Rotationsverdampfer entfernt. Der dabei ausfallende Feststoff wird filtriert, in 400 mL Eisessig suspendiert und nach Hinzugabe von 150 mL konzentrierter Salzsäure 2 h bei 100 °C gerührt. Nach Abkühlen auf Raumtemperatur wird mit 400 mL Wasser hydrolysiert, der dabei ausgefallene Feststoff filtriert und mit 200 mL Wasser, Ethanol (200 mL) und zuletzt mit 200 mL *n*-Heptan gewaschen. Der Feststoff wird über Aluminiumoxid mit *n-*Heptan/Toluol heißextrahiert und umkristallisiert. 20.1 g (42.0 mmol, entspr. 85 %) des Produkts werden als weißer Feststoff erhalten. Die weitere Aufreinigung erfolgt mittels Umkristallisation aus Toluol/Heptan und Zonensublimation (265 °C, 10⁻⁵ bar). Die Ausbeute beträgt 8.4 g (15 mmol, entspr. 32 %, HPLC-Reinheit >99.9 %)

Analog können folgende Verbindungen hergestellt werden:

| | Edukt1 | Edukt 2 | Produkt | Ausbeute |
|---|---|---|---|---|
| 7b | 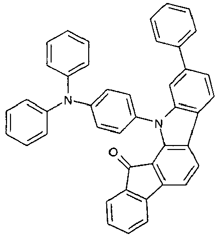 | 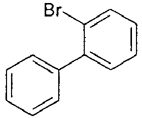 | 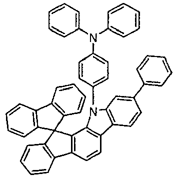 | 31% |
| | | 2052-07-5 | | |
| 7c | 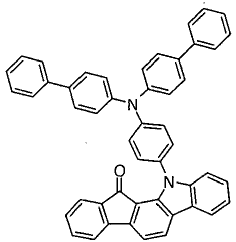 | 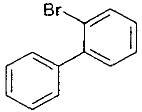 | 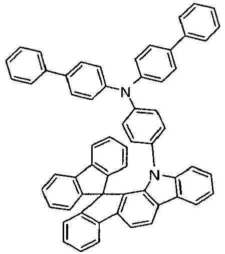 | 27% |
| | | 2052-07-5 | | |
| 7d | 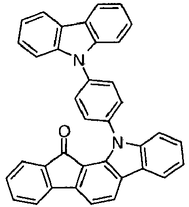 | 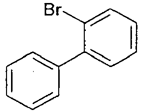 | 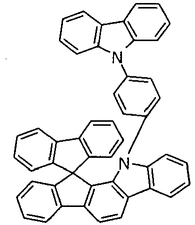 | 36% |
| | | 2052-07-5 | | |
| 7e | 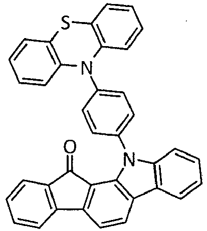 | 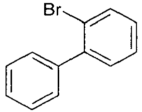 | 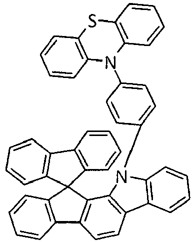 | 35% |
| | | 2052-07-5 | | |
| 7f | 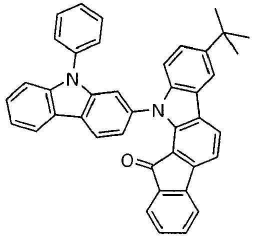 | 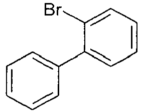 | 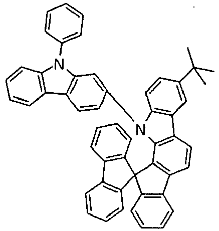 | 38% |
| | | 2052-07-5 | | |
| 7g | 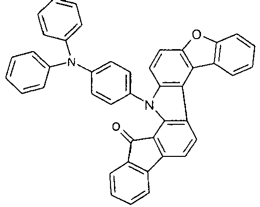 | 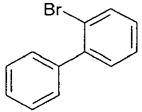 | 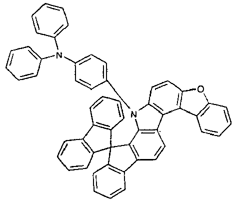 | 39% |
| | | 2052-07-5 | | |
| 7h | 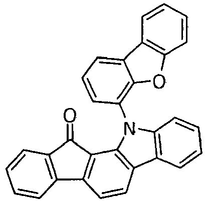 | 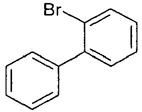 | 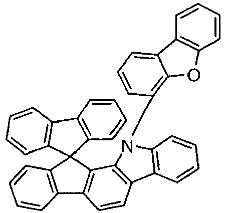 | 36% |
| | | 2052-07-5 | | |
| 7i | 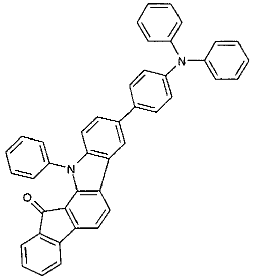 | 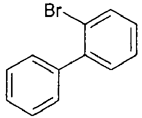 | 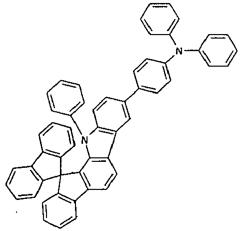 | 33% |
| | | 2052-07-5 | | |
| 7j | 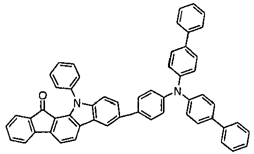 | 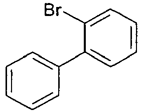 | 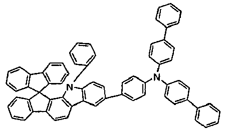 | 35% |
| | | 2052-07-5 | | |
| 7k | 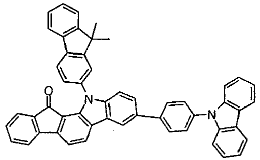 | 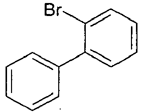 | 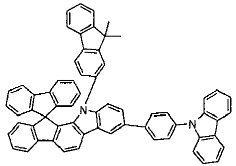 | 27% |
| | | 2052-07-5 | | |
| 7l | 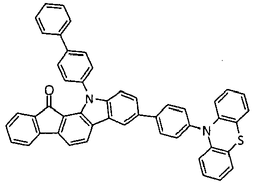 | 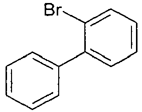 | 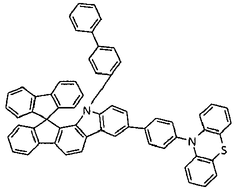 | 29% |
| | | 2052-07-5 | | |
| 7m | 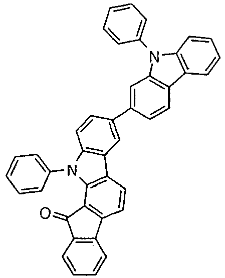 | 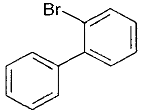 | 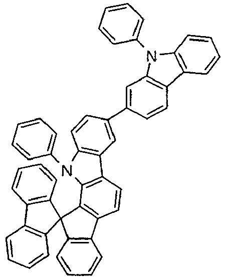 | 36% |
| | | 2052-07-5 | | |
| 7n | 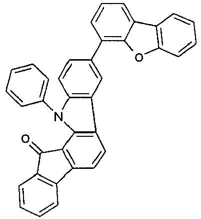 | 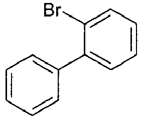 | 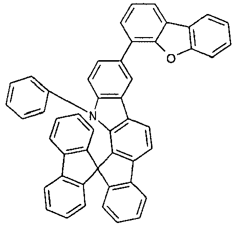 | 37% |
| | | 2052-07-5 | | |
| 7o = Vgl. | 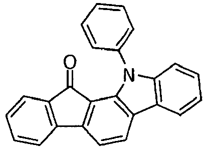 | 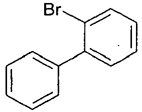 | 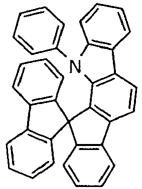 | 38% |
| | | 2052-07-5 | | |
| 7p | 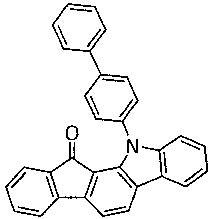 | 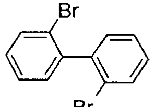 | 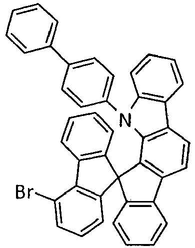 | 64% (ohne Sublimation) |
| | | 13029-09-9 | | |
| 7q | 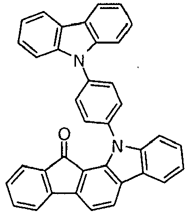 | 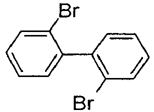 | 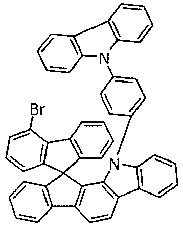 | 65% (ohne Sublimation) |
| | | 13029-09-9 | | |

### Herstellung der OLEDs

In den folgenden Beispielen V1 bis E16 (siehe Tabellen 1 und 2) werden die Daten verschiedener OLEDs vorgestellt.

### Vorbehandlung für die Beispiele V1-E16:

Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Dabei bedeutet eine Bezeichnung wie "7a" die Verbindung aus Beispiel 7a. Die weiteren zur Herstellung der OLEDs verwendeten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Das Beispiel V1 ist ein Vergleichsbeispiel gemäß dem Stand der Technik, die Beispiele E1-E16 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien zeigen bei Einsatz als Matrixmaterialien in phosphoreszierenden OLEDs Verbesserungen der Leistungseffizienz gegenüber dem Stand der Technik. Durch Einsatz der erfindungsgemäßen Verbindung 7a in Kombination mit dem grün emittierenden Dotanden TEG1 und der Matrix ST1 lässt sich eine Steigerung der der Leistungseffizienz um ca. 15% gegenüber dem Stand der Technik 7o beobachten (Beispiele V1, E1).

**Tabelle 1: Aufbau der OLEDs**

| Bsp | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | ST1:7o:TEG1 (30%:58%:12%) 30nm | IC1 10nm | ST2:LiQ (50%:50%) 30nm | --- |
| E1 | SpA1 70nm | HATCN 5nm | SpMA1 90nm | ST1:7a:TEG1 (30%:58%:12%) 30nm | IC1 10nm | ST2:LiQ (50%:50%) 30nm | --- |
| E2 | SpA1 70nm | HATCN 5nm | SpMA1 90nm | ST1:7b:TEG1 (44%:44%:12%) 30nm | IC1 10nm | ST2:LiQ (50%:50%) 30nm | --- |
| E3 | SpA1 70nm | HATCN 5nm | SpMA1 90nm | ST1:7c:TEG1 (58%:30%:12%)30nm | IC1 10nm | ST2:LiQ (50%:50%) 30nm | --- |
| E4 | SpA1 70nm | HATCN 5nm | SpMA1 90nm | ST1:7d:TEG1 (32%:56%:12%) 30nm | IC1 10nm | ST2:LiQ (50%:50%) 30nm | --- |
| E5 | SpA1 90nm | HATCN 5nm | SpMA1 130nm | IC2:7e:TER1 (30%:60%:10%) 40nm | --- | ST2:LiQ (50%:50%) 40nm | --- |
| E6 | SpA1 70nm | HATCN 5nm | SpMA1 90nm | ST1:7f:TEG1 (32%:56%:12%) 30nm | IC1 10nm | ST2:LiQ (50%:50%) 30nm | --- |
| E7 | SpA1 70nm | HATCN 5nm | SpMA1 90nm | ST1:7g:TEG1 (44%:44%:12%) 30nm | IC1 10nm | ST2:LiQ (50%:50%) 30nm | --- |
| E8 | SpA1 70nm | HATCN 5nm | SpMA1 90nm | ST1:7h:TEG1 (30%:58%:12%) 30nm | IC1 10nm | ST2:LiQ (50%:50%) 30nm | --- |
| E9 | SpA1 70nm | HATCN 5nm | SpMA1 90nm | ST1:7i:TEG1 (44%:44%:12%) 30nm | IC1 10nm | ST2:LiQ (50%:50%) 30nm | --- |
| E10 | SpA1 70nm | HATCN 5nm | SpMA1 90nm | ST1:7j:TEG1 (56%:32%:12%) 30nm | IC1 10nm | ST2:LiQ (50%:50%) 30nm | --- |
| E11 | SpA1 70nm | HATCN 5nm | SpMA1 90nm | ST1:7k:TEG1 (34%:54%:12%) 30nm | IC1 10nm | ST2:LiQ (50%:50%) 30nm | --- |
| E12 | SpA1 90nm | HATCN 5nm | SpMA1 130nm | IC2:7I:TER1 (30%:60%:10%) 40nm | --- | ST2:LiQ (50%:50%) 40nm | --- |
| E13 | SpA1 90nm | HATCN 5nm | SpMA1 130nm | IC2:7m:TER1 (45%:45%:10%) 40nm | --- | ST2:LiQ (50%:50%) 40nm | --- |
| E14 | SpA1 90nm | HATCN 5nm | SpMA1 130nm | IC2:7n:TER1 (30%:60%:10%) 40nm | --- | ST2:LiQ (50%:50%) 40nm | --- |
| E15 | SpA1 70nm | HATCN 5nm | SpMA1 90nm | ST1:3p:TEG1 (32%:56%:12%) 30nm | IC1 10nm | ST2:LiQ (50%:50%) 30nm | --- |
| E16 | SpA1 70nm | HATCN 5nm | SpMA1 90nm | ST1:3q:TEG1 (32%:56%:12%) 30nm | IC1 10nm | ST2:LiQ (50%:50%) 30nm | --- |

**Tabelle 2**

| Bsp. | U1000 (V) | SE1000 (cd/A) | LE1000 (lm/W) | EQE 1000 | CIE x/y bei 1000 cd/m^{2.} |
|---|---|---|---|---|---|
| V1 | 3.4 | 58 | 54 | 15.6% | 0.33/0.62 |
| E1 | 3.2 | 64 | 62 | 17.2% | 0.34/0.63 |
| E2 | 3.4 | 68 | 63 | 18.4% | 0.34/0.62 |
| E3 | 3.3 | 67 | 64 | 18.1% | 0.34/0.63 |
| E4 | 3.1 | 63 | 64 | 16.9% | 0.33/0.62 |
| E5 | 4.3 | 11 | 8 | 12.1% | 0.67/0.33 |
| E6 | 3.3 | 61 | 58 | 16.5% | 0.33/0.62 |
| E7 | 3.5 | 67 | 60 | 17.9% | 0.34/0.62 |
| E8 | 3.4 | 65 | 60 | 17.6% | 0.33/0.63 |
| E9 | 3.6 | 70 | 61 | 18.7% | 0.34/0.62 |
| E10 | 3.4 | 68 | 63 | 18.2% | 0.33/0.62 |
| E11 | 3.2 | 62 | 61 | 16.6% | 0.33/0.63 |
| E12 | 4.1 | 13 | 10 | 12.4% | 0.66/0.34 |
| E13 | 4.2 | 14 | 10 | 13.3% | 0.67/0.33 |
| E14 | 4.3 | 14 | 10 | 12.8% | 0.66/0.34 |
| E15 | 3.4 | 63 | 58 | 16.8% | 0.33/0.62 |
| E16 | 3.5 | 65 | 58 | 17.5% | 0.34/0.63 |

**Tabelle 3: Strukturformeln der Materialien für die OLEDs**

| | |
|---|---|
| 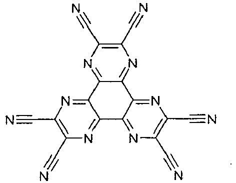 | 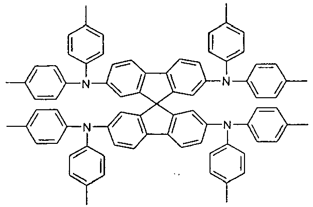 |
| HATCN | SpA1 |
| 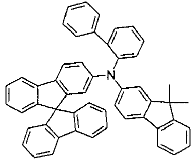 | 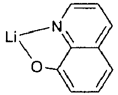 |
| SpMA1 | LiQ |
| 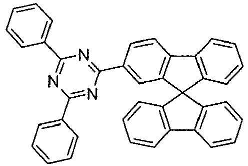 | 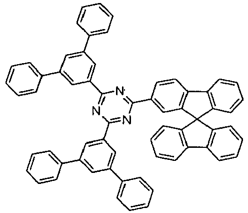 |
| ST1 | ST2 |
| 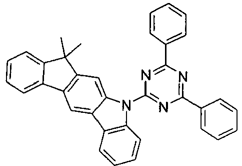 | 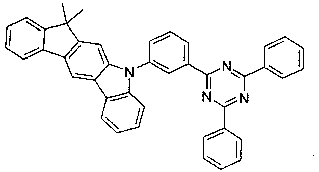 |
| IC1 | IC2 |
| 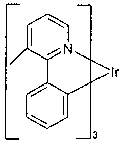 | 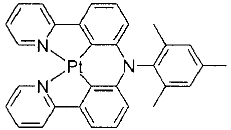 |
| TEG1 | TER1 |

## Patentansprüche

1. Verbindung gemäß Formel (1), Formel (2) oder Formel (3), 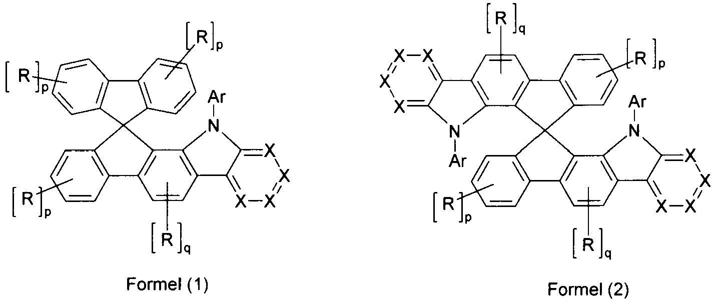 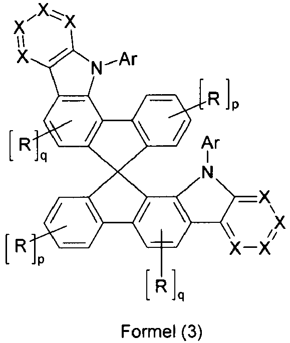 wobei für die verwendeten Symbole und Indizes gilt:
X ist bei jedem Auftreten gleich oder verschieden CR¹ oder N; oder zwei benachbarte X stehen für eine Gruppe der folgenden Formel (4), (5) oder (6), 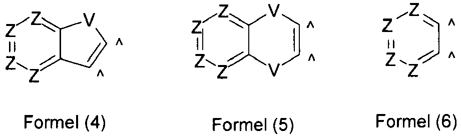 wobei ^ die entsprechenden benachbarten Gruppen X in der Formel (1) bzw. (2) bzw. (3) andeutet;
V ist bei jedem Auftreten gleich oder verschieden C(R¹)₂, NR¹, O, S, BR¹, Si(R¹)₂ oder C=O;
Z ist bei jedem Auftreten gleich oder verschieden CR¹ oder N;
Ar ist bei jedem Auftreten gleich oder verschieden ein aromatisches Ringsystem mit 6 bis 40 aromatischen Ringatomen, das mit einem oder mehreren Resten R² substituiert sein kann, oder ein heteroaromatisches Ringsystem mit 5 bis 40 aromatischen Ringatomen, das keine elektronenarmen Heteroarylgruppen enthält und das mit einem oder mehreren Resten R² substituiert sein kann;
R, R¹ ist bei jedem Auftreten gleich oder verschieden ausgewählt aus der Gruppe bestehend aus H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³, Si(R³)₂, C=O, C=S, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R³ substituiert sein kann, einer Aryloxy- oder Heteroaryloxygruppe mit 5 bis 40 aromatischen Ringatomen, die mit einem oder mehreren Resten R³ substituiert sein kann, oder einer Aralkyl- oder Heteroaralkylgruppe mit 5 bis 40 aromatischen Ringatomen, die mit einem oder mehreren Resten R³ substituiert sein kann; dabei können optional zwei Substituenten R¹, die an dasselbe Kohlenstoffatom gebunden sind, ein monocyclisches oder polycyclisches, aliphatisches, aromatisches oder heteroaromatisches Ringsystem bilden, das mit einem oder mehreren Resten R³ substituiert sein kann;
R² ist gleich oder verschieden bei jedem Auftreten ausgewählt aus der Gruppe bestehend aus H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, einer geradkettigen Alkyl-, Alkoxy- oder Thioalkylgruppe mit 1 bis 20 C-Atomen oder einer verzweigten oder cyclischen Alkyl-, Alkoxy-oder Thioalkylgruppe mit 3 bis 20 C-Atomen oder einer Alkenyl-gruppe mit 2 bis 20 C-Atomen, die jeweils mit einem oder mehreren Resten R³ substituiert sein kann, wobei eine oder mehrere nichtbenachbarte CH₂-Gruppen durch R³C=CR³, Si(R³)₂, C=O, C=S, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder hetero-aromatischen Ringsystem mit 5 bis 40 aromatischen Ringatomen, das keine elektronenarmen Heteroarylgruppen enthält und das jeweils mit einem oder mehreren Resten R³ substituiert sein kann, einer Aryloxy- oder Heteroaryloxygruppe mit 5 bis 40 aromatischen Ringatomen, die keine elektronenarmen Heteroarylgruppen enthält und die mit einem oder mehreren Resten R³ substituiert sein kann, oder einer Aralkyl- oder Heteroaralkylgruppe mit 5 bis 40 aromatischen Ringatomen, die keine elektronenarmen Heteroarylgruppen enthält und die mit einem oder mehreren Resten R³ substituiert sein kann; dabei können zwei oder mehr benachbarte Substituenten R³ miteinander ein mono- oder polycyclisches, aliphatisches Ringsystem bilden;
Ar¹ ist bei jedem Auftreten gleich oder verschieden ein aromatisches oder heteroaromatisches Ringsystem mit 5-30 aromatischen Ring-atomen, das mit einem oder mehreren nicht-aromatischen Resten R³ substituiert sein kann; dabei können zwei Reste Ar¹, welche an dasselbe N-Atom, P-Atom oder B-Atom binden, auch durch eine Einfachbindung oder eine Brücke, ausgewählt aus N(R³), C(R³)₂, O oder S, miteinander verbrückt sein;
R³ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches Ringsystem bilden können;
p ist bei jedem Auftreten gleich oder verschieden 0, 1, 2, 3 oder 4;
q ist 0, 1 oder 2;
dabei sind die beiden folgenden Verbindungen von der Erfindung ausgenommen:

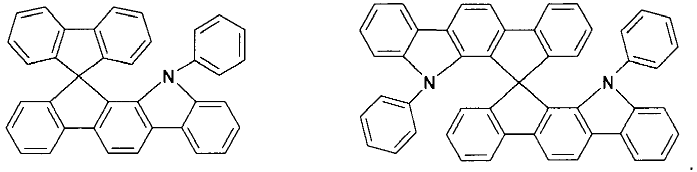

2. Verbindung nach Anspruch 1, **dadurch gekennzeichnet, dass** X gleich oder verschieden bei jedem Auftreten für CR¹ oder N steht, wobei maximal eine Gruppe X pro Cyclus für N steht, oder dass zwei benachbarte Gruppen X für eine Gruppe der Formel (4) stehen, wobei Z gleich oder verschieden bei jedem Auftreten für CR¹ steht und V gleich oder verschieden bei jedem Auftreten für NR¹, C(R¹)₂, O oder S steht, und die restlichen Gruppen X in dem Cyclus für CR¹ stehen.

3. Verbindung nach Anspruch 1 oder 2, ausgewählt aus den Verbindungen der Formeln (7) bis (15), 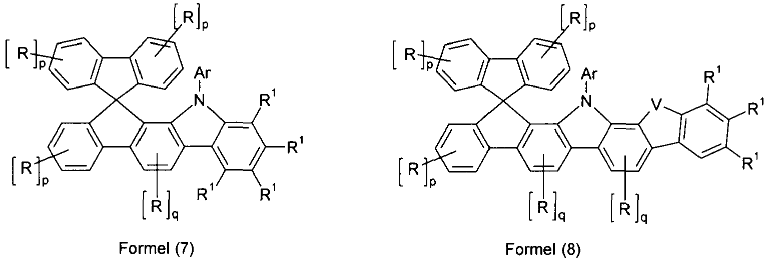 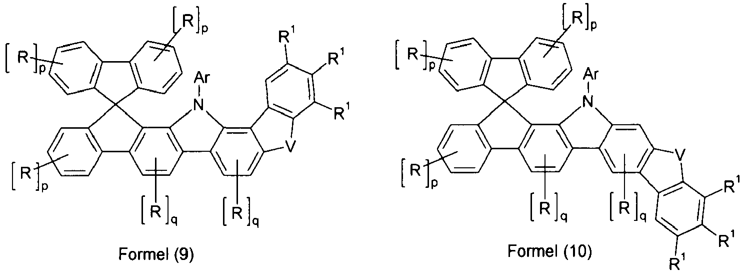 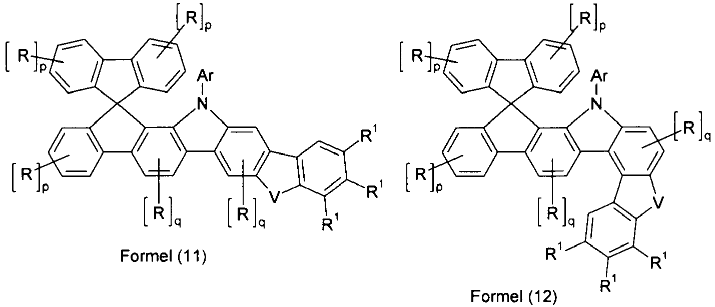 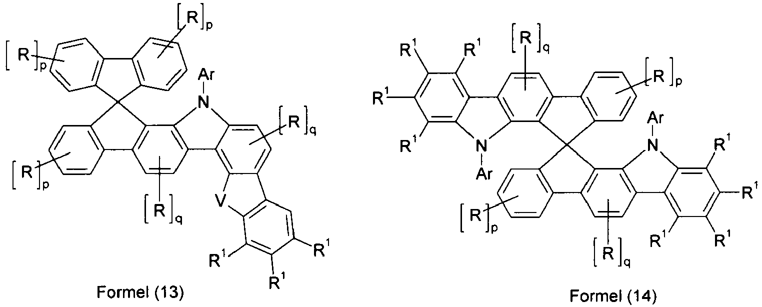 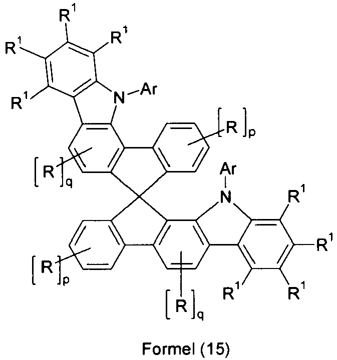 wobei die verwendeten Symbole und Indizes die in Anspruch 1 genannten Bedeutungen aufweisen.

4. Verbindung nach einem oder mehreren der Ansprüche 1 bis 3, ausgewählt aus Verbindungen der Formeln (7a) bis (15a), 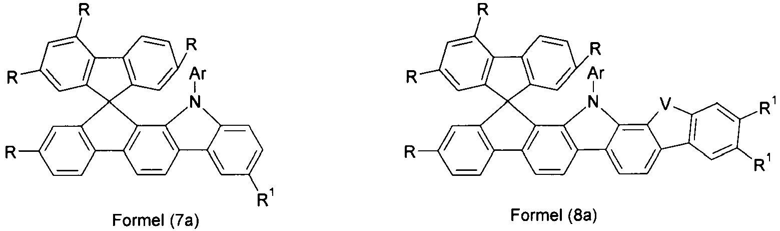 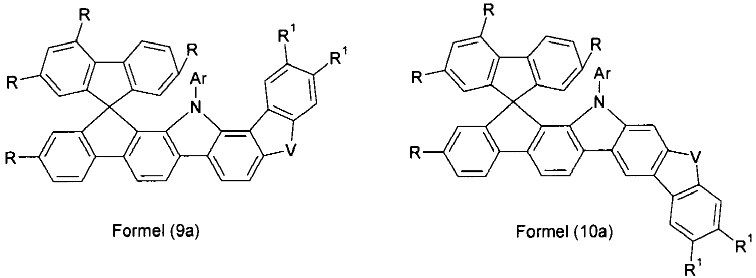 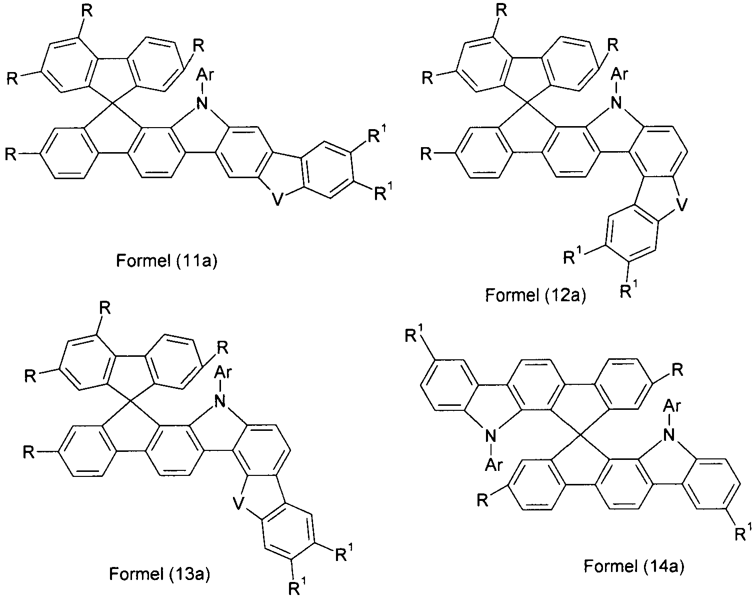 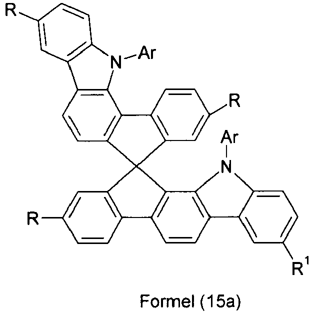 wobei die verwendeten Symbole und Indizes die in Anspruch 1 genannten Bedeutungen aufweisen.

5. Verbindung nach einem oder mehreren der Ansprüche 1 bis 4, **dadurch gekennzeichnet, dass** R gleich oder verschieden bei jedem Auftreten ausgewählt ist aus der Gruppe bestehend aus H, F, CN, N(Ar¹)₂, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 aromatischen Ringatomen, das mit einem oder mehreren nicht-aromatischen Resten R³ substituiert sein kann.

6. Verbindung nach einem oder mehreren der Ansprüche 1 bis 5, ausgewählt aus Verbindungen der Formeln (7b) bis (15b), 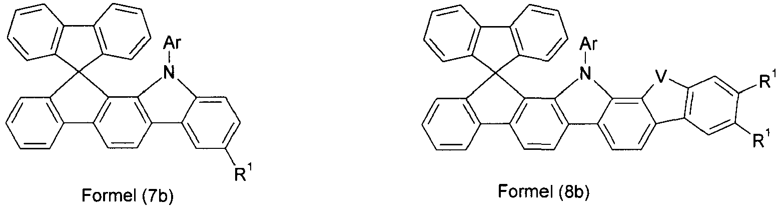 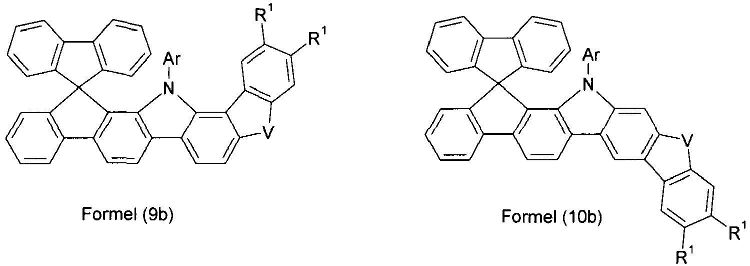 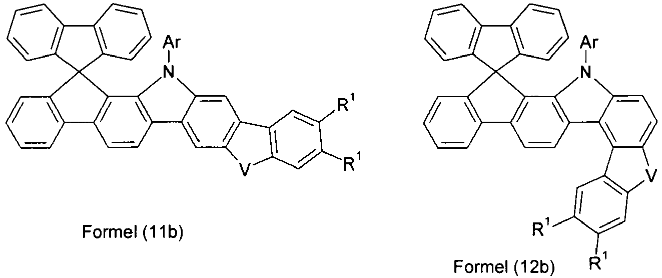 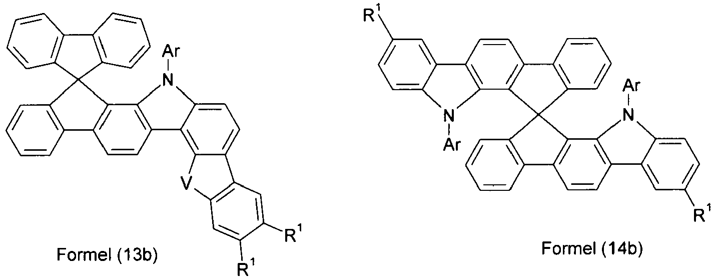 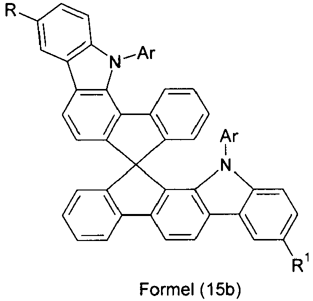 wobei die verwendeten Symbole die in Anspruch 1 genannten Bedeutungen aufweisen.

7. Verbindung nach einem oder mehreren der Ansprüche 1 bis 6, **dadurch gekennzeichnet, dass** die Verbindungen der Formel (1) und (3) in den Substituenten Ar, R¹ und R² insgesamt mindestens 12 aromatische Ringatomen enthalten und dass die Verbindungen der Formel (2) in den Substituenten Ar, R¹ und R² insgesamt mindestens 24 aromatische Ringatomen enthalten.

8. Verbindung nach einem oder mehreren der Ansprüche 1 bis 7, **dadurch gekennzeichnet, dass** Ar ausgewählt ist aus den Gruppen der Formeln Ar-1 bis Ar-58, 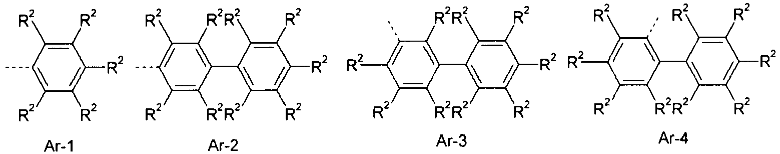 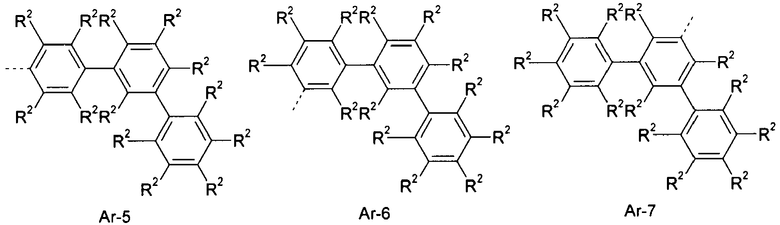 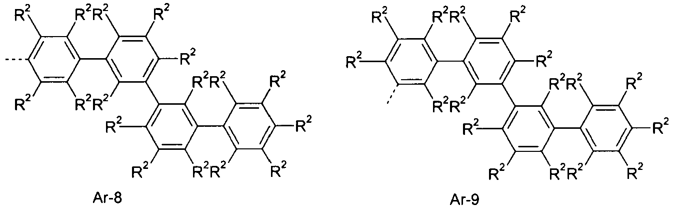 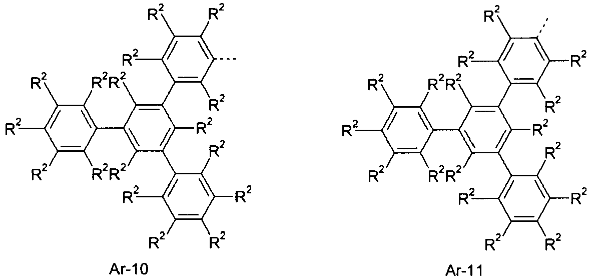 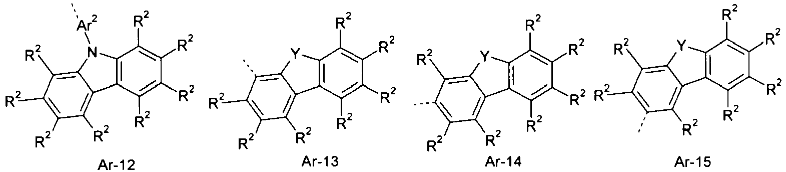 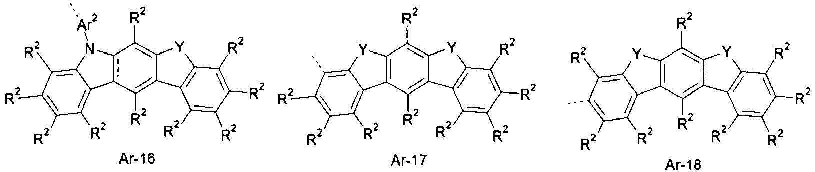 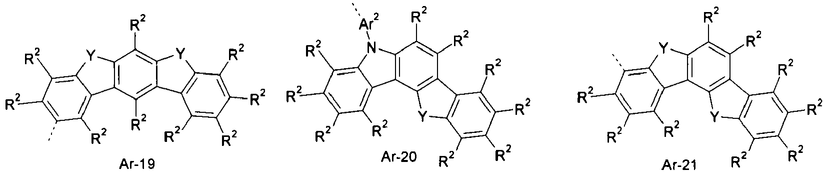 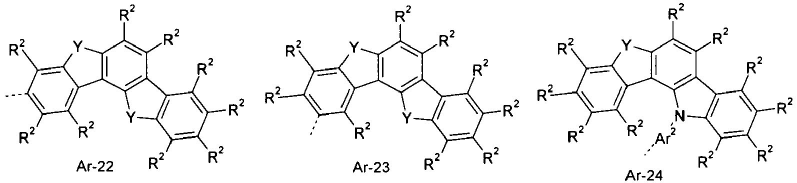 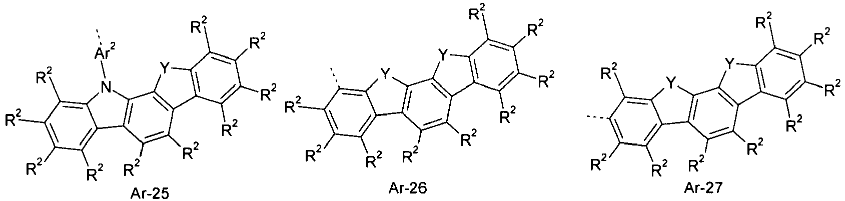 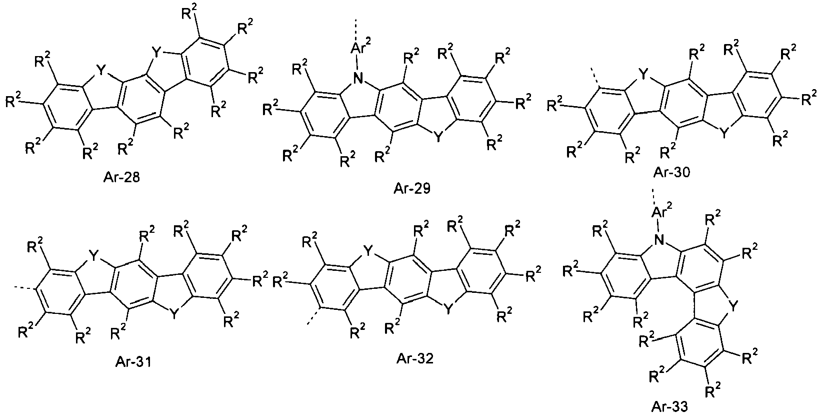 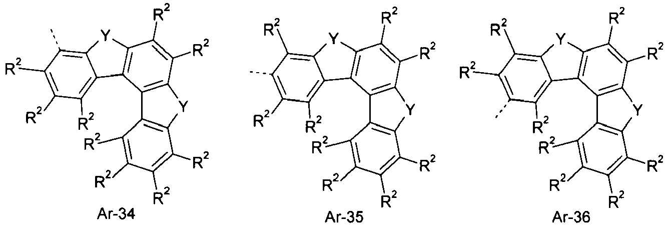 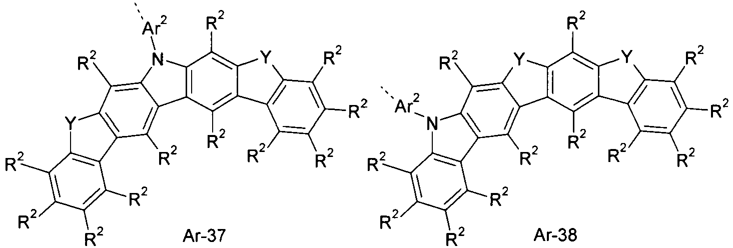 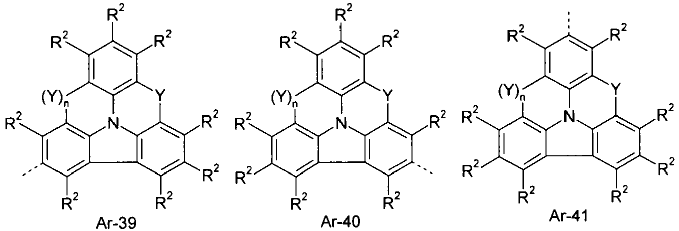 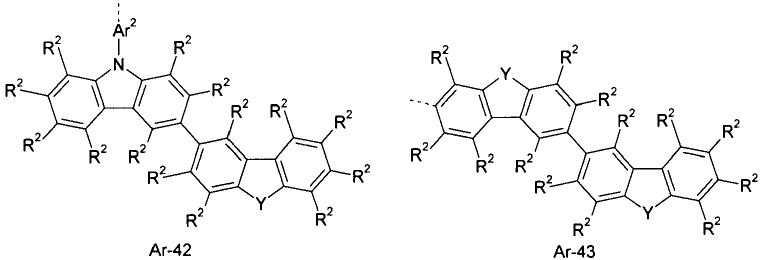 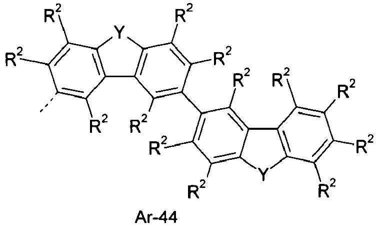 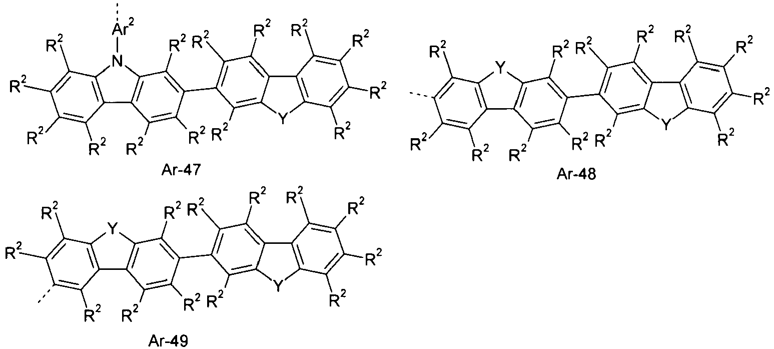 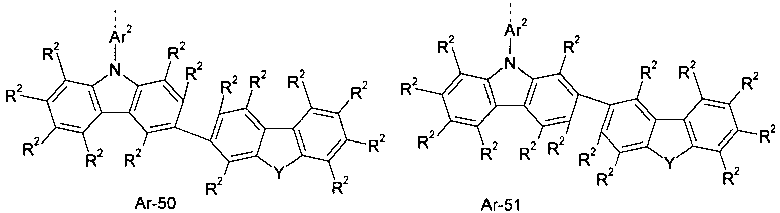 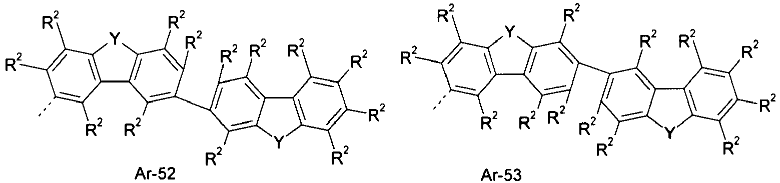 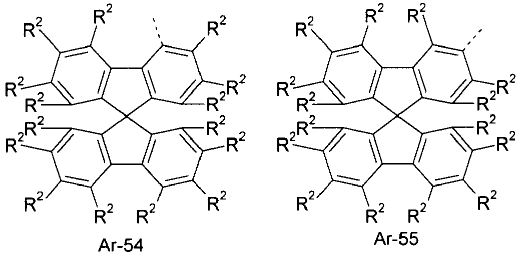 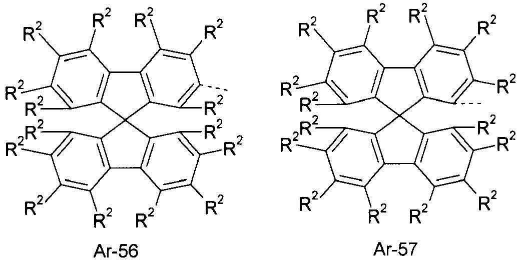 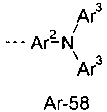 wobei R² die in Anspruch 1 genannten Bedeutungen aufweist, die gestrichelte Bindung die Bindung an die Gruppe der Formel (1) bzw. (2) bzw. (3) darstellt und weiterhin gilt:
Ar² ist bei jedem Auftreten gleich oder verschieden ein bivalentes aromatisches oder heteroaromatisches Ringsystem mit 6 bis 18 aromatischen Ringatomen, welches keine elektronenarmen Heteroarylgruppen enthält und welches jeweils mit einem oder mehreren Resten R² substituiert sein kann;
Ar³ ist gleich oder verschieden bei jedem Auftreten ein aromatisches oder heteroaromatisches Ringsystem mit 6 bis 18 aromatischen Ringatomen, welches keine elektronenarmen Heteroarylgruppen enthält und welches jeweils mit einem oder mehreren Resten R² substituiert sein kann;
Y ist bei jedem Auftreten gleich oder verschieden C(R²)₂, NR², O oder S;
n ist 0 oder 1, wobei n gleich 0 bedeutet, dass an dieser Position keine Gruppe Y gebunden ist und an den entsprechenden Kohlenstoffatomen statt dessen Reste R² gebunden sind;
wobei die Gruppen Ar-1 bis Ar-57 auch über eine verbrückende Gruppe an das Stickstoffatom in Formel (1) bzw. Formel (2) bzw. Formel (3) binden können.

9. Verbindung nach einem oder mehreren der Ansprüche 1 bis 8, **dadurch gekennzeichnet, dass** R¹ gleich oder verschieden bei jedem Auftreten ausgewählt ist aus der Gruppe bestehend aus H, D, N(Ar¹)₂, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R³ substituiert sein kann.

10. Verbindung nach einem oder mehreren der Ansprüche 1 bis 9, **dadurch gekennzeichnet, dass** R¹, wenn es für ein aromatisches oder heteroaromatisches Ringsystem steht, ausgewählt ist aus den Gruppen R¹-1 bis R¹-43, 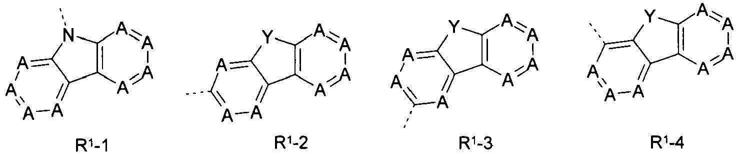 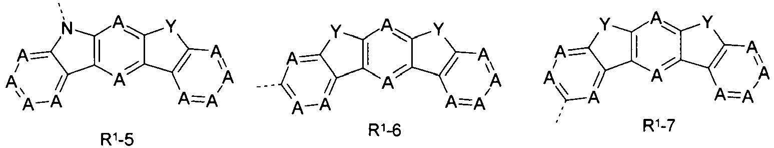 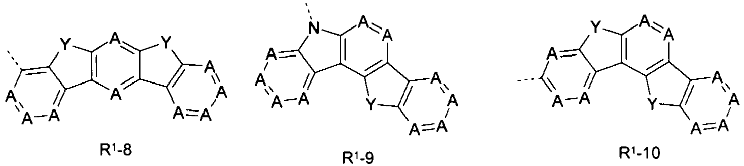 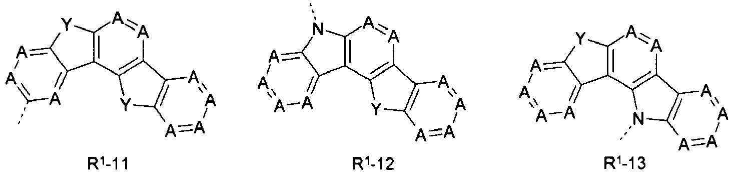 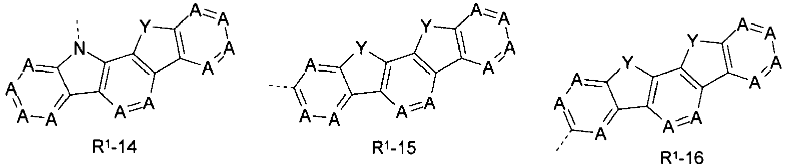 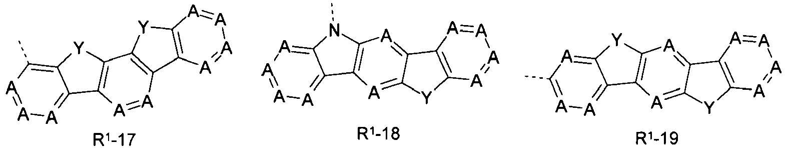 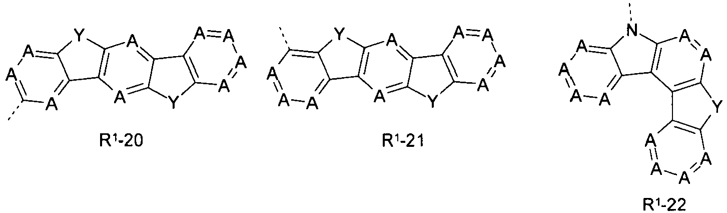 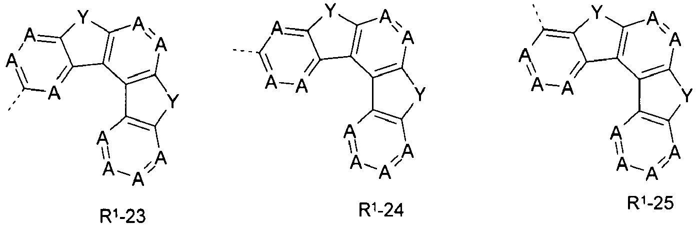 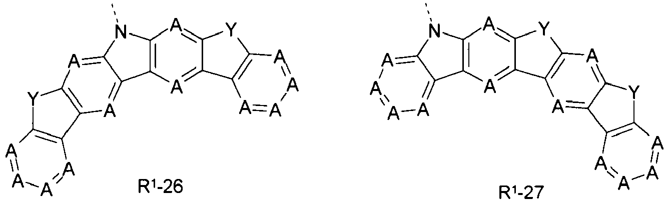 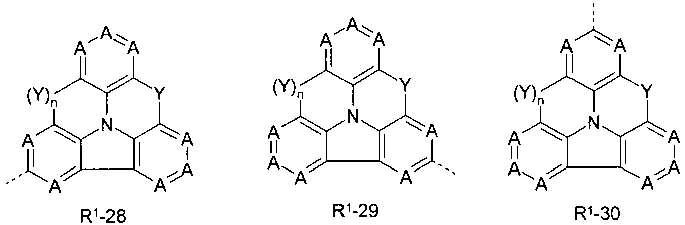 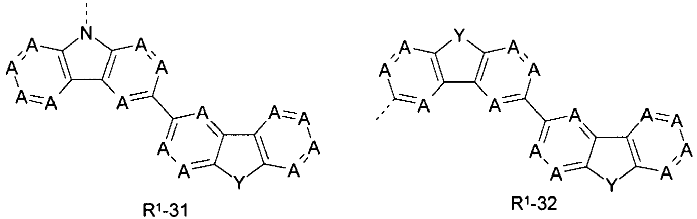 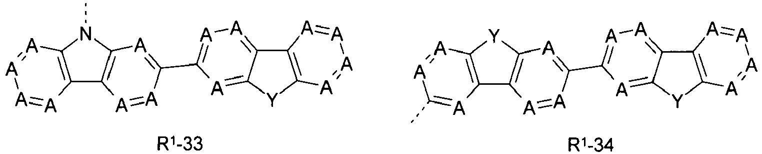 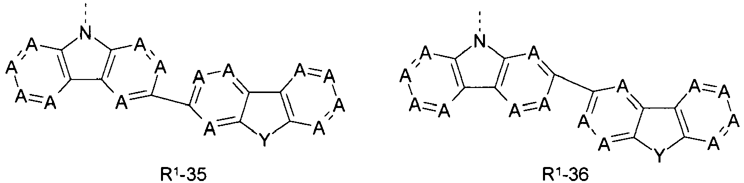 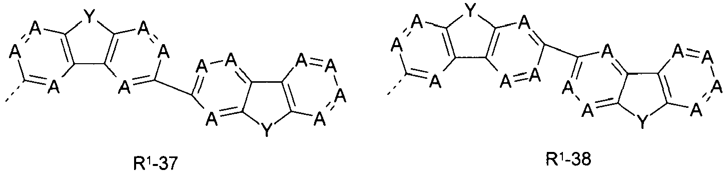 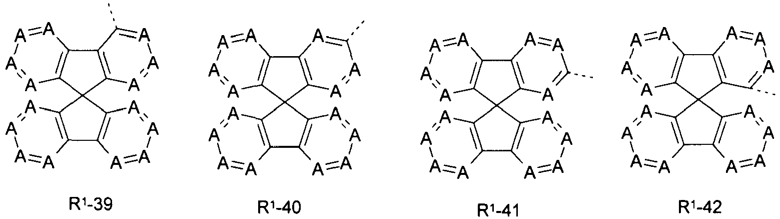 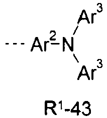 wobei R³ die in Anspruch 1 genannten Bedeutungen aufweist, die gestrichelte Bindung die Bindung an die Gruppe der Formel (1) bzw. (2) bzw. (3) darstellt und weiterhin gilt:
A ist bei jedem Auftreten gleich oder verschieden CR³ oder N, wobei maximal 3 Symbole X pro Cyclus für N stehen;
Y ist bei jedem Auftreten gleich oder verschieden C(R³)₂, NR³, O oder S;
Ar², Ar³ ist gleich oder verschieden bei jedem Auftreten ein aromatisches oder heteroaromatisches Ringsystem mit 5 bis 18 aromatischen Ringatomen steht, welches jeweils mit einem oder mehreren Resten R³ substituiert sein kann
n ist 0 oder 1, wobei n gleich 0 bedeutet, dass an dieser Position keine Gruppe Y gebunden ist und an den entsprechenden Kohlenstoffatomen statt dessen Reste R³ gebunden sind;
wobei die Gruppen R¹-1 bis R¹-42 auch über eine verbrückende Gruppe an das Stickstoffatom in Formel (1) bzw. Formel (2) bzw. Formel (3) binden können.

11. Oligomer, Polymer oder Dendrimer enthaltend eine oder mehrere Verbindungen nach einem oder mehreren der Ansprüche 1 bis 10, wobei an einer oder mehreren Positionen statt Substituenten eine oder mehrere Bindungen der Verbindung zum Polymer, Oligomer oder Dendrimer vorhanden sind.

12. Formulierung, enthaltend mindestens eine Verbindung nach einem oder mehreren der Ansprüche 1 bis 10 und/oder mindestens ein Oligomer, Polymer oder Dendrimer nach Anspruch 11 und mindestens eine weitere Verbindung, insbesondere ein Lösemittel.

13. Verwendung einer Verbindung nach einem oder mehreren der Ansprüche 1 bis 10 und/oder eines Oligomers, Polymers oder Dendrimers nach Anspruch 11 in einer elektronischen Vorrichtung.

14. Elektronische Vorrichtung enthaltend mindestens eine Verbindung nach einem oder mehreren der Ansprüche 1 bis 10 und/oder mindestens ein Oligomer, Polymer oder Dendrimer nach Anspruch 11, bevorzugt ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und Organic Plasmon Emitting Devices.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 12 als Matrixmaterial für phosphoreszierende oder fluoreszierende Emitter und/oder in einer elektronenblockierenden bzw. exzitonenblockierenden Schicht und/oder in einer Lochtransportschicht und/oder in einer Lochblockierschicht und/oder in einer Lochblockier- oder Elektronentransportschicht eingesetzt wird.

## Claims

1. Compound of the formula (1), formula (2) or formula (3), 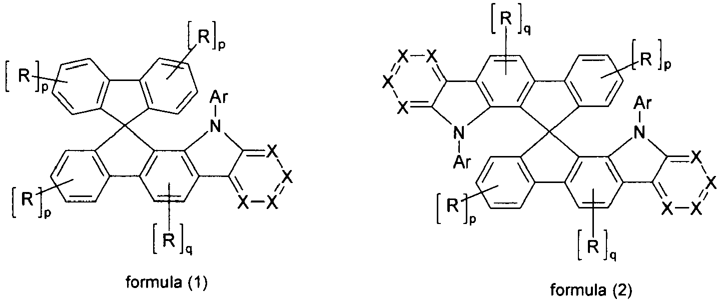 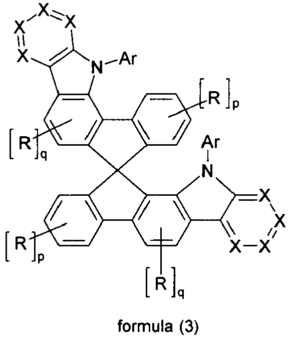 where the following applies to the symbols and indices used:
X is on each occurrence, identically or differently, CR¹ or N; or two adjacent X stand for a group of the following formula (4), (5) or (6), 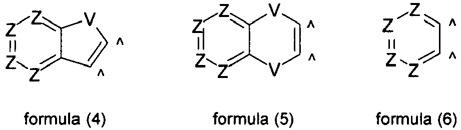 where ^ indicates the corresponding adjacent groups X in the formula (1) or (2) or (3);
V is on each occurrence, identically or differently, C(R¹)₂, NR¹, O, S, BR¹, Si(R¹)₂ or C=O;
Z is on each occurrence, identically or differently, CR¹ or N;
Ar is on each occurrence, identically or differently, an aromatic ring system having 6 to 40 aromatic ring atoms, which may be substituted by one or more radicals R², or a heteroaromatic ring system having 5 to 40 aromatic ring atoms, which contains no electrondeficient heteroaryl groups and which may be substituted by one or more radicals R²;
R, R¹ are selected on each occurrence, identically or differently, from the group consisting of H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals R³, where one or more non-adjacent CH₂ groups may be replaced by R³C=CR³, Si(R³)₂, C=O, C=S, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 40 aromatic ring atoms, which may in each case be substituted by one or more radicals R³, an aryloxy or heteroaryloxy group having 5 to 40 aromatic ring atoms, which may be substituted by one or more radicals R³, or an aralkyl or heteroaralkyl group having 5 to 40 aromatic ring atoms, which may be substituted by one or more radicals R³; two substituents R¹ here which are bonded to the same carbon atom may form a monocyclic or polycyclic, aliphatic, aromatic or heteroaromatic ring system, which may be substituted by one or more radicals R³;
R² is selected on each occurrence, identically or differently, from the group consisting of H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals R³, where one or more non-adjacent CH₂ groups may be replaced by R³C=CR³, Si(R³)₂, C=O, C=S, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 40 aromatic ring atoms which contained no electron-deficient heteroaryl groups and which may in each case be substituted by one or more radicals R³, an aryloxy or heteroaryloxy group having 5 to 40 aromatic ring atoms which contains no electron-deficient heteroaryl groups and which may be substituted by one or more radicals R³, or an aralkyl or heteroaralkyl group having 5 to 40 aromatic ring atoms which contains no electron-deficient heteroaryl groups and which may be substituted by one or more radicals R³; two or more adjacent substituents R³ here may form a mono- or polycyclic aliphatic ring system with one another;
Ar¹ is on each occurrence, identically or differently, an aromatic or heteroaromatic ring system having 5-30 aromatic ring atoms, which may be substituted by one or more non-aromatic radicals R³; two radicals Ar¹ here which are bonded to the same N atom, P atom or B atom may also be bridged to one another by a single bond or a bridge selected from N(R³), C(R³)₂, O or S;
R³ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN, where two or more adjacent substituents R³ may form a mono- or polycyclic aliphatic ring system with one another;
p is on each occurrence, identically or differently, 0, 1, 2, 3 or 4;
q is 0, 1 or 2;
the two following compounds are excluded from the invention:

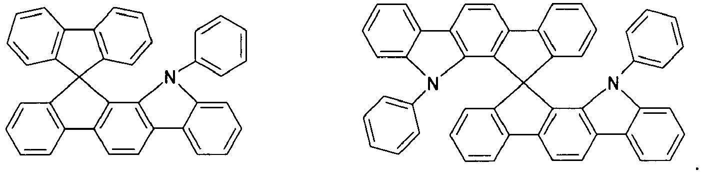

2. Compound according to Claim 1, **characterised in that** X stands, identically or differently on each occurrence, for CR¹ or N, where a maximum of one group X per ring stands for N, or is at two adjacent groups X stand for a group of the formula (4), where Z stands, identically or differently on each occurrence, for CR¹ and V stands, identically or differently on each occurrence, for NR¹, C(R¹)₂, O or S, and the remaining groups X in the ring stand for CR¹.

3. Compound according to Claim 1 or 2, selected from the compounds of the formulae (7) to (15), 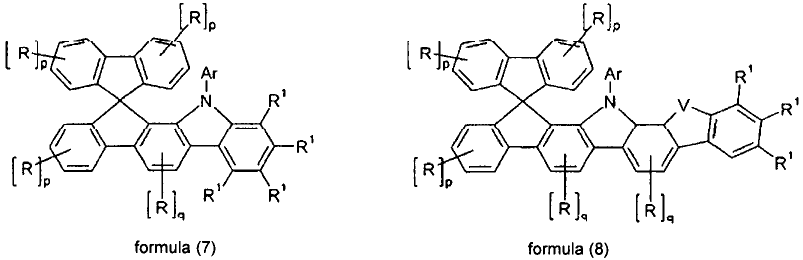 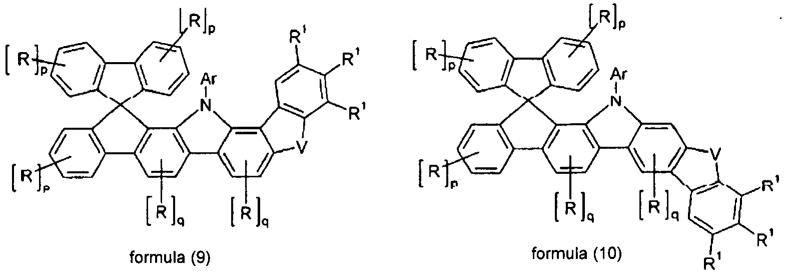 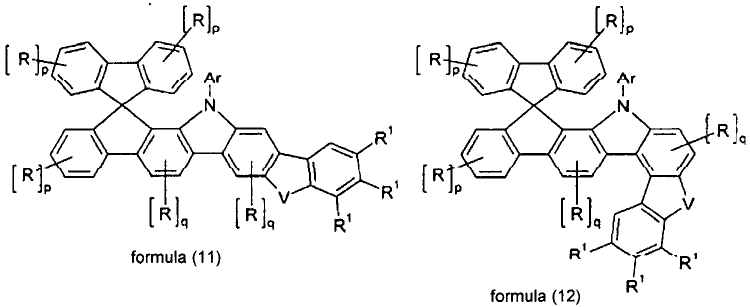 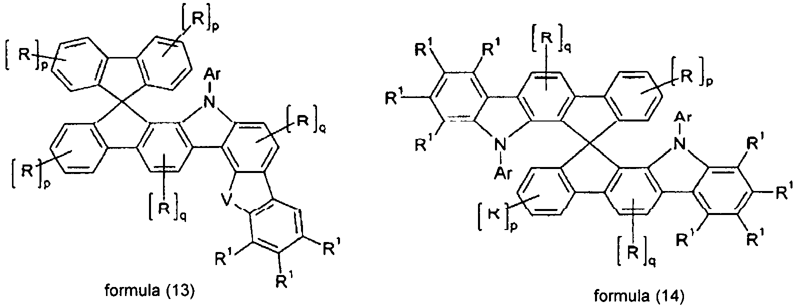 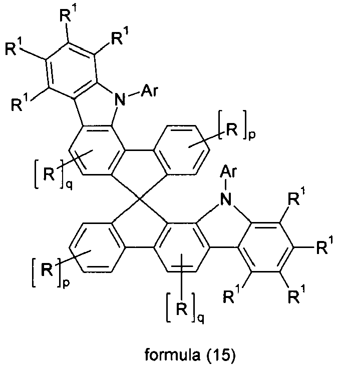 where the symbols and indices used have the meanings given in Claim 1.

4. Compound according to one or more of Claims 1 to 3, selected from compounds of the formulae (7a) to (15a), 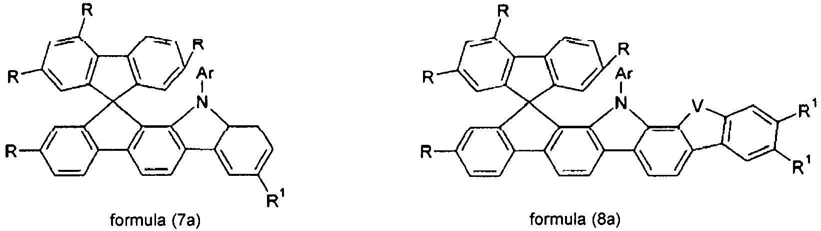 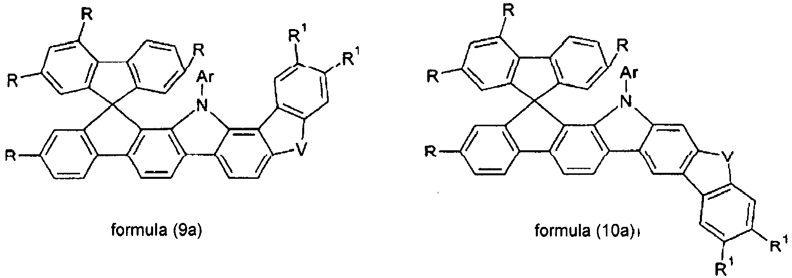 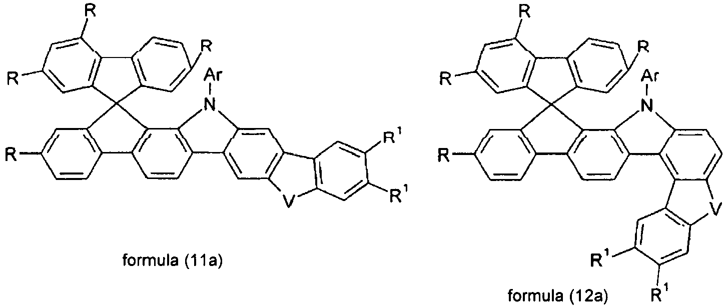 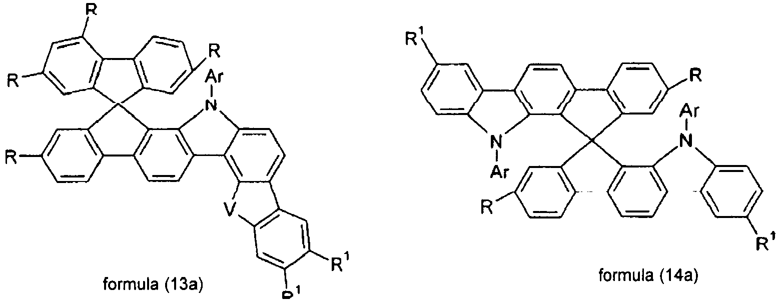 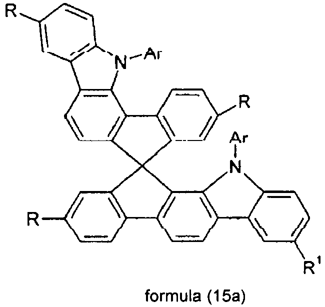 where the symbols and indices used have the meanings given in Claim 1.

5. Compound according to one or more of Claims 1 to 4, **characterised in that** R is selected, identically or differently on each occurrence, from the group consisting of H, F, CN, N(Ar¹)₂, a straight-chain alkyl group having 1 to 10 C atoms or a branched or cyclic alkyl group having 3 to 10 C atoms or an aromatic ring system having 6 to 30 aromatic ring atoms, which may be substituted by one or more non-aromatic radicals R³.

6. Compound according to one or more of Claims 1 to 5, selected from compounds of the formulae (7b) to (15b), 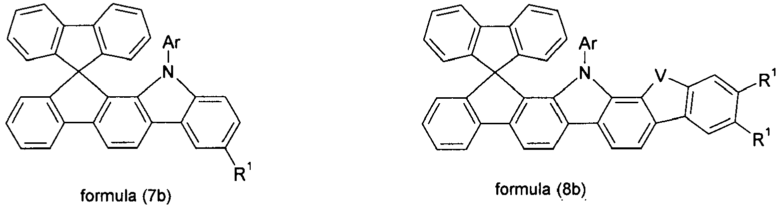 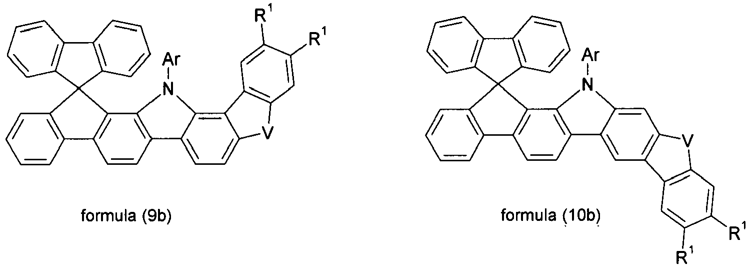 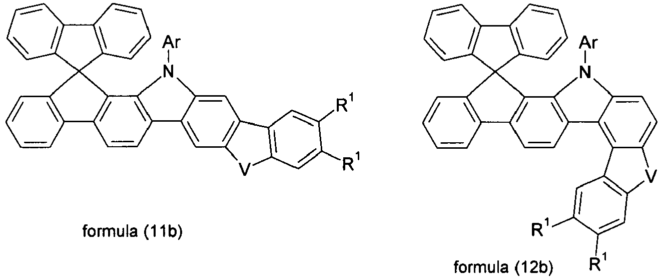 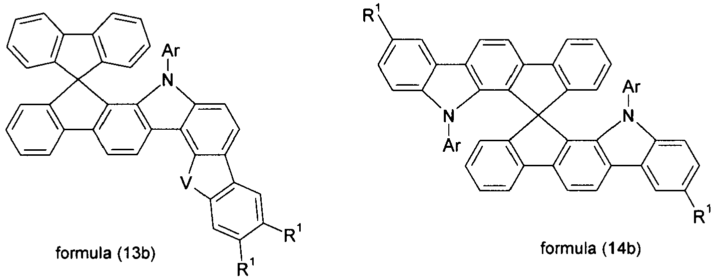 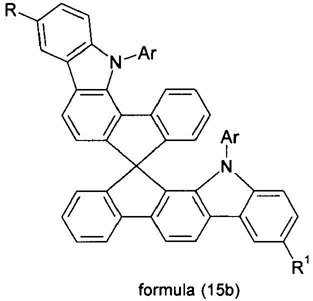 where the symbols used have the meanings given in Claim 1.

7. Compound according to one or more of Claims 1 to 6, **characterised in that** the compounds of the formulae (1) and (3) contain in total at least 12 aromatic ring atoms in the substituents Ar, R¹ and R² and that the compounds of the formula (2) contain in total at least 24 aromatic ring atoms in the substituents Ar, R¹ and R².

8. Compound according to one or more of Claims 1 to 7, **characterised in that** Ar is selected from the groups of the formulae Ar-1 to Ar-58, 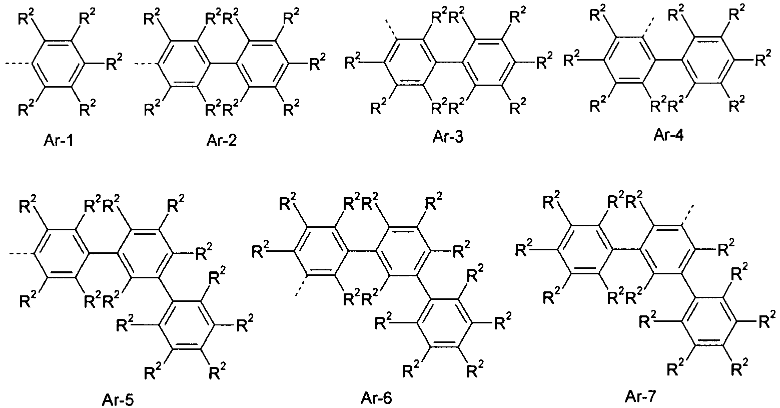 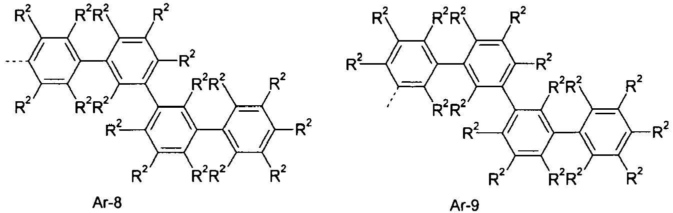 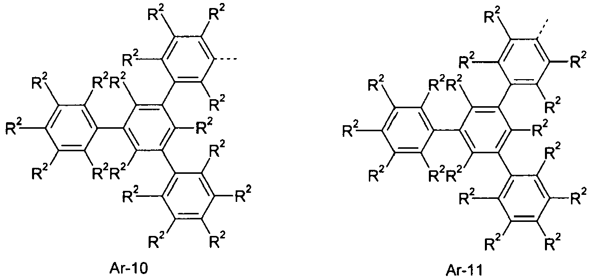 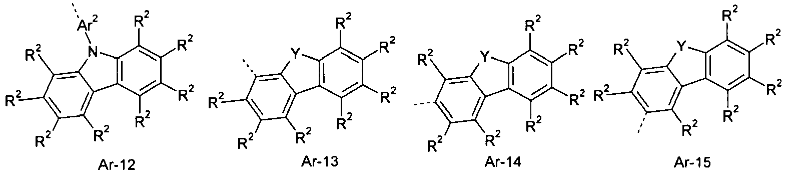 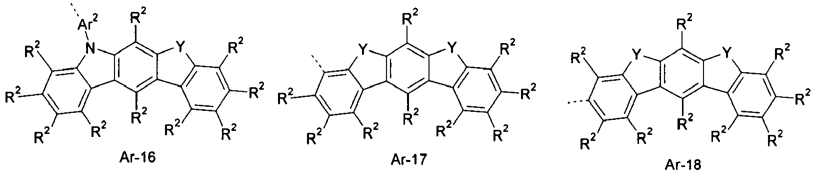 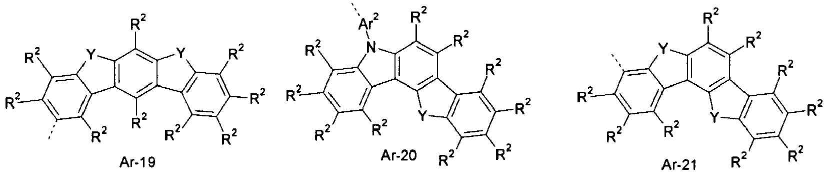 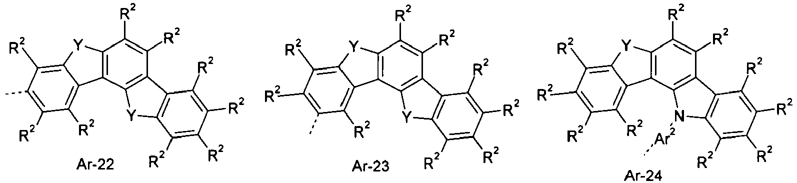 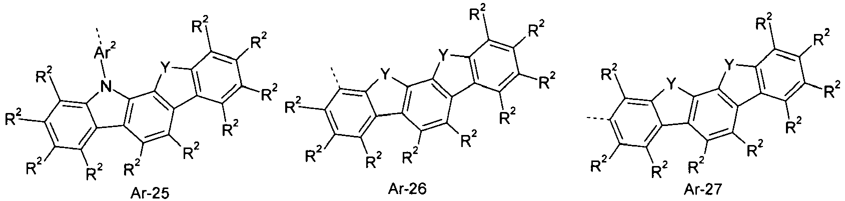 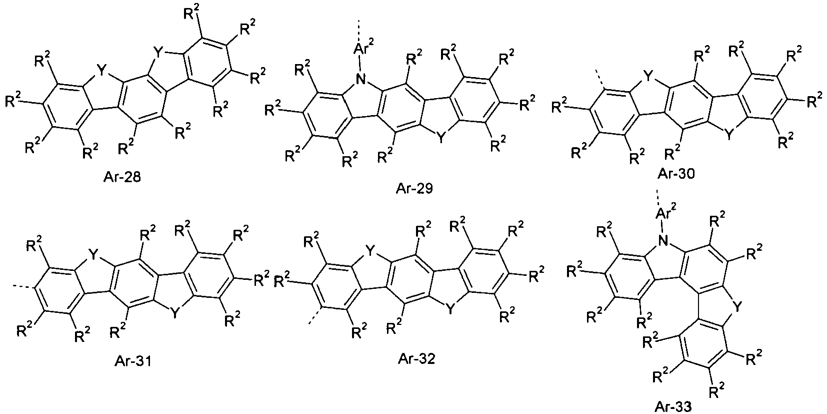 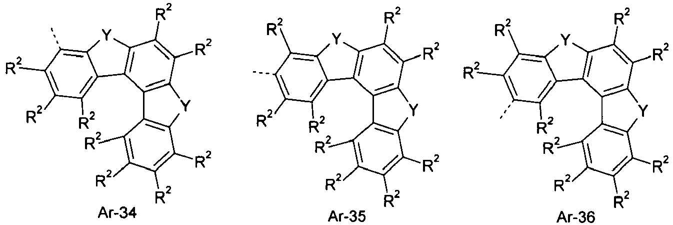 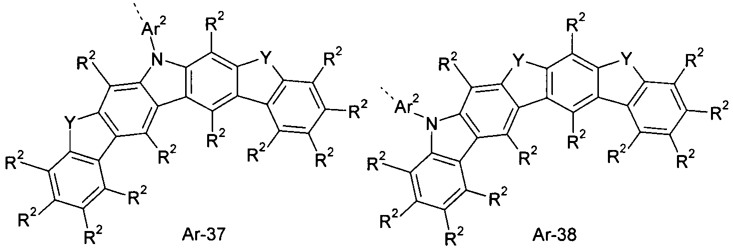 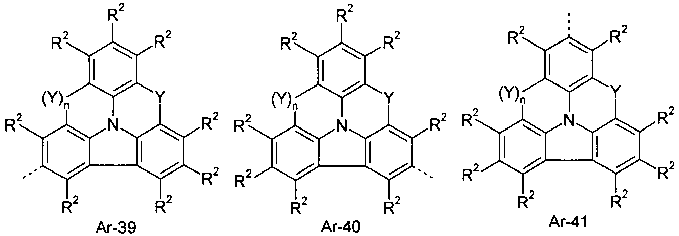 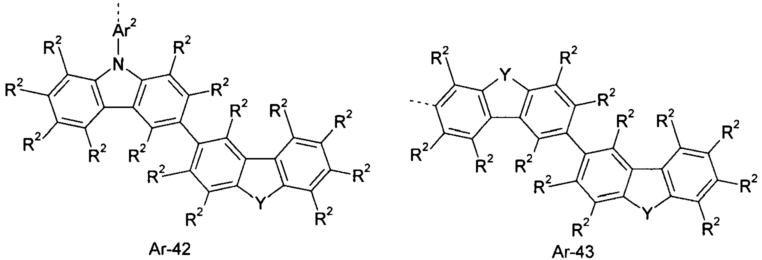 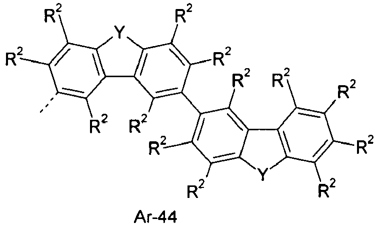 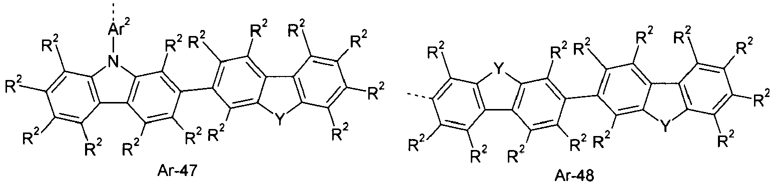 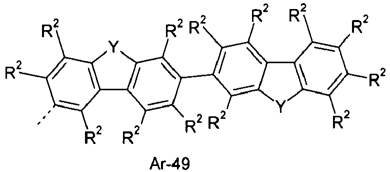 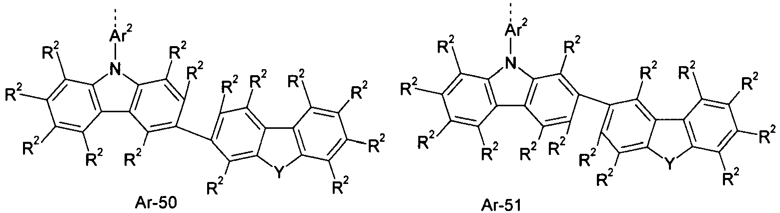 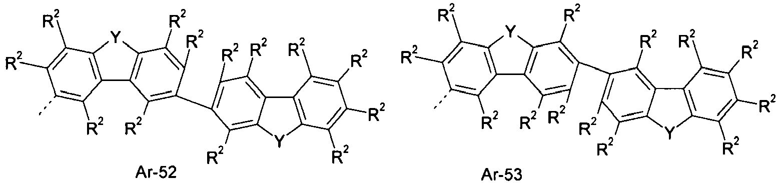 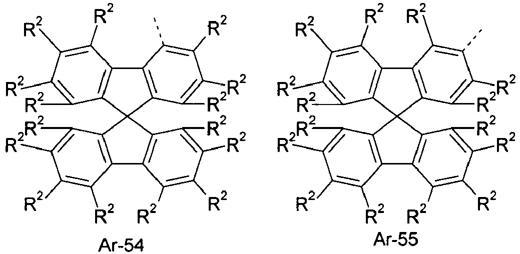 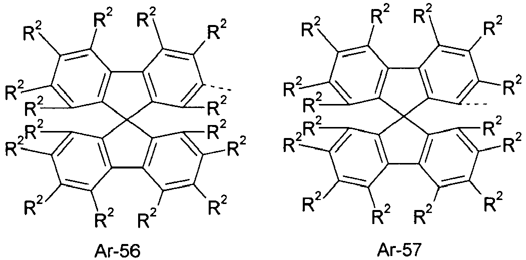 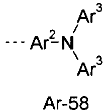 where R² has the meanings given in Claim 1, the dashed bond represents the bond to the group of the formula (1) or (2) or (3) and furthermore:
Ar² is on each occurrence, identically or differently, a divalent aromatic or heteroaromatic ring system having 6 to 18 aromatic ring atoms, which contains no electron-deficient heteroaryl groups and which may in each case be substituted by one or more radicals R²;
Ar³ is, identically or differently on each occurrence, an aromatic or heteroaromatic ring system having 6 to 18 aromatic ring atoms, which contains no electron-deficient heteroaryl groups and which may in each case be substituted by one or more radicals R²;
Y is on each occurrence, identically or differently, C(R²)₂, NR², O or S;
n is 0 or 1, where n equals 0 means that no group Y is bonded at this position and instead radicals R² are bonded to the corresponding carbon atoms;
where the groups Ar-1 to Ar-57 may also be bonded to the nitrogen atom in formula (1) or formula (2) or formula (3) via a bridging group.

9. Compound according to one or more of Claims 1 to 8, **characterised in that** R¹ is selected, identically or differently on each occurrence, from the group consisting of H, D, N(Ar¹)₂, a straight-chain alkyl group having 1 to 10 C atoms or a branched or cyclic alkyl group having 3 to 10 C atoms, which may in each case be substituted by one or more radicals R³, where one or more H atoms may be replaced by D or F, or an aromatic or heteroaromatic ring system having 5 to 30 aromatic ring atoms, which may in each case be substituted by one or more radicals R³.

10. Compound according to one or more of Claims 1 to 9, **characterised in that** R¹, if it stands for an aromatic or heteroaromatic ring system, is selected from the groups R¹-1 to R¹-43, 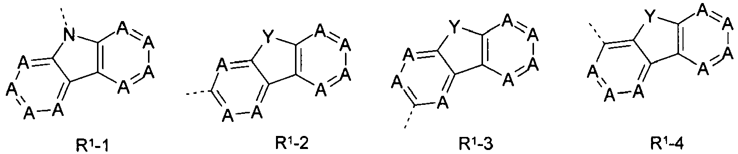 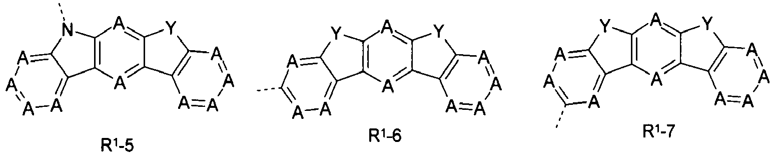 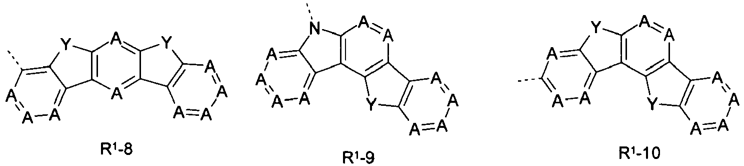 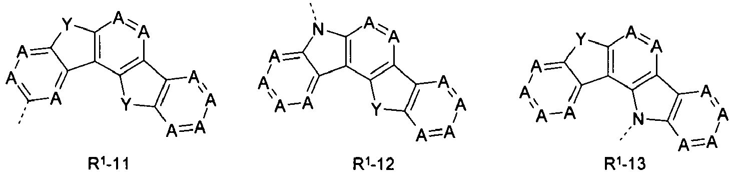 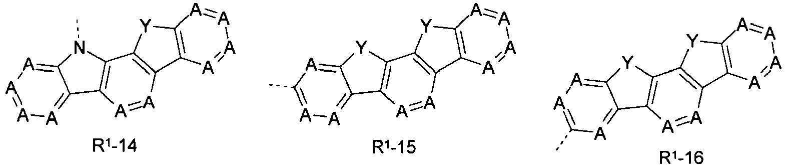 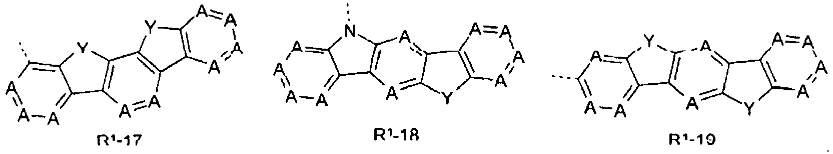 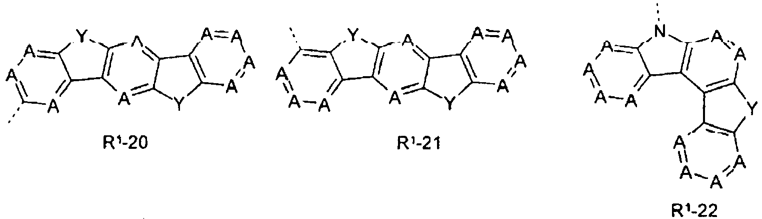 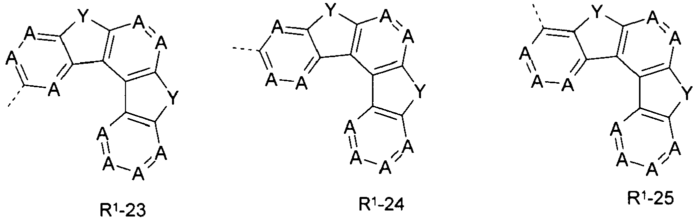 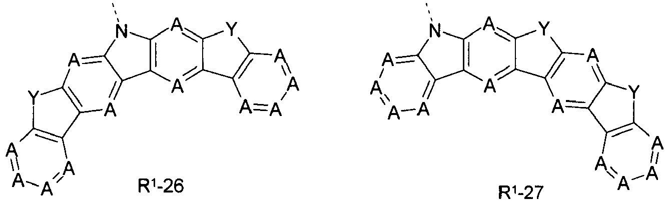 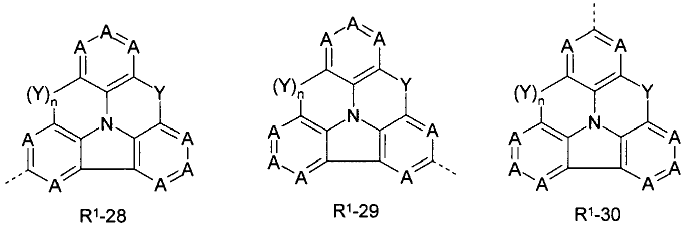 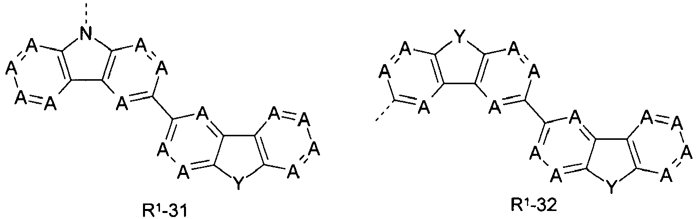 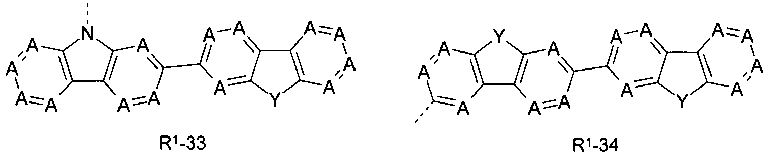 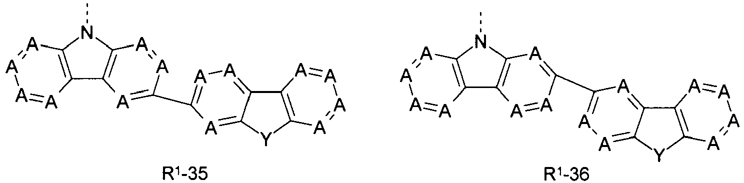 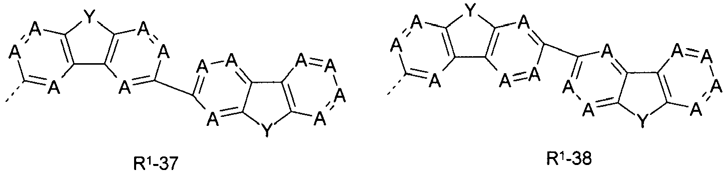 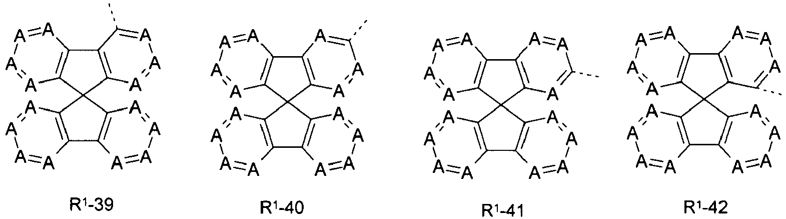 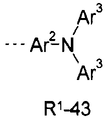 where R³ has the meanings given in Claim 1, the dashed bond represents the bond to the group of the formula (1) or (2) or (3) and furthermore:
A is on each occurrence, identically or differently, CR³ or N, where a maximum of 3 symbols X per ring stand for N;
Y is on each occurrence, identically or differently, C(R³)₂, NR³, O or S;
Ar², Ar³ are, identically or differently on each occurrence, an aromatic or heteroaromatic ring system having 5 to 18 aromatic ring atoms, which may in each case be substituted by one or more radicals R³
n is 0 or 1, where n equals 0 means that no group Y is bonded at this position and instead radicals R³ are bonded to the corresponding carbon atoms;
where the groups R¹-1 to R¹-42 may also be bonded to the nitrogen atom in formula (1) or formula (2) or formula (3) via a bridging group.

11. Oligomer, polymer or dendrimer containing one or more compounds according to one or more of Claims 1 to 10, where one or more bonds from the compound to the polymer, oligomer or dendrimers are present at one or more positions instead of substituents.

12. Formulation comprising at least one compound according to one or more of Claims 1 to 10 and/or at least one oligomer, polymer or dendrimers according to Claim 11 and at least one further compound, in particular a solvent.

13. Use of a compound according to one or more of Claims 1 to 10 and/or an oligomer, polymer or dendrimers according to Claim 11 in an electronic device.

14. Electronic device containing at least one compound according to one or more of Claims 1 to 10 and/or at least one oligomer, polymer or dendrimers according to Claim 11, preferably selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

15. Electronic device according to Claim 14, which is an organic electroluminescent device, **characterised in that** the compound according to one or more of Claims 1 to 12 is employed as matrix material for phosphorescent of fluorescent emitter is and/or in an electron-blocking or exciton-blocking layer and/or in a hole-transport layer and/or in a hole-blocking layer and/or in a hole-blocking or electron-transport layer.

## Revendications

1. Composé de la formule (1), de la formule (2) ou de la formule (3) : 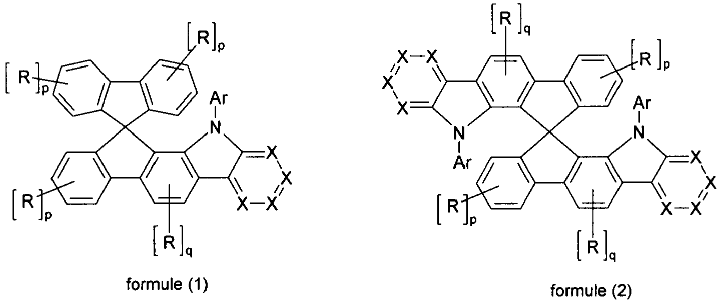 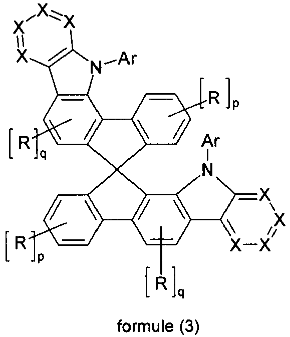 formules dans lesquelles ce qui suit s'applique aux symboles et indices qui sont utilisés :
X est pour chaque occurrence, de manière identique ou différente, CR¹ ou N ; ou deux X adjacents représentent un groupe de la formule (4), de la formule (5) ou de la formule (6) qui suivent : 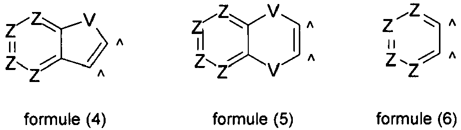 formules dans lesquelles ^ indique les groupes X adjacents correspondants dans la formule (1) ou dans la formule (2) ou dans la formule (3) ;
V est pour chaque occurrence, de manière identique ou différente, C(R¹)₂, NR¹, O, S, BR¹, Si(R¹)₂ ou C=O ;
Z est pour chaque occurrence, de manière identique ou différente, CR¹ ou N;
Ar est pour chaque occurrence, de manière identique ou différente, un système de cycle aromatique qui comporte 6 à 40 atomes de cycle aromatique, lequel peut être substitué par un radical ou par plusieurs radicaux R², ou un système de cycle hétéroaromatique qui comporte 5 à 40 atomes de cycle aromatique, lequel ne contient pas de groupes hétéroaryle déficients en électrons et lequel peut être substitué par un radical ou par plusieurs radicaux R²;
R, R¹ sont sélectionnés pour chaque occurrence, de manière identique ou différente, parmi le groupe qui est constitué par H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle qui comporte 2 à 20 atomes de C, lequel peut dans chaque cas être substitué par un radical ou par plusieurs radicaux R³, où un ou plusieurs groupe(s) CH₂ non adjacents peut/peuvent être remplacé(s) par R³C=CR³, Si(R³)₂, C=O, C=S, C=NR³, P(=O)(R³), SO, SO₂, NR³, O, S ou CONR³ et où un ou plusieurs atome(s) de H peut/ peuvent être remplacé(s) par D, F, Cl, Br, I, CN ou NO₂, un système de cycle aromatique ou hétéroaromatique qui comporte 5 à 40 atomes de cycle aromatique, lequel peut dans chaque cas être substitué par un radical ou par plusieurs radicaux R³, un groupe aryloxy ou hétéroaryloxy qui comporte 5 à 40 atomes de cycle aromatique, lequel peut être substitué par un radical ou par plusieurs radicaux R³, ou un groupe aralkyle ou hétéroaralkyle qui comporte 5 à 40 atomes de cycle aromatique, lequel peut être substitué par un radical ou par plusieurs radicaux R³; deux substituants R¹ qui sont liés ici au même atome de carbone peuvent former un système de cycle, aliphatique, aromatique ou hétéroaromatique monocyclique ou polycyclique, lequel peut être substitué par un radical ou par plusieurs radicaux R³;
R² est sélectionné pour chaque occurrence, de manière identique ou différente, parmi le groupe qui est constitué par H, D, F, Cl, Br, I, CN, NO₂, N(Ar¹)₂, N(R³)₂, C(=O)Ar¹, C(=O)R³, P(=O)(Ar¹)₂, P(Ar¹)₂, B(Ar¹)₂, Si(Ar¹)₃, Si(R³)₃, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle qui comporte 2 à 20 atomes de C, lequel peut dans chaque cas être substitué par un radical ou par plusieurs radicaux R³, où un ou plusieurs groupe(s) CH₂ non adjacents peut/peuvent être remplacé(s) par R³C=CR³, Si(R³)₂, C=O, C=S, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, un système de cycle aromatique ou hétéroaromatique qui comporte 5 à 40 atomes de cycle aromatique, lequel ne contient pas de groupes hétéroaryle déficients en électrons et lequel peut dans chaque cas être substitué par un radical ou par plusieurs radicaux R³, un groupe aryloxy ou hétéroaryloxy qui comporte 5 à 40 atomes de cycle aromatique, lequel ne contient pas de groupes hétéroaryle déficients en électrons et lequel peut être substitué par un radical ou par plusieurs radicaux R³, ou un groupe aralkyle ou hétéroaralkyle qui comporte 5 à 40 atomes de cycle aromatique, lequel ne contient pas de groupes hétéroaryle déficients en électrons et lequel peut être substitué par un radical ou par plusieurs radicaux R³; deux substituants R³ adjacents ou plus peuvent ici former un système de cycle aliphatique monocyclique ou polycyclique l'un avec l'autre ou les uns avec les autres ;
Ar¹ est pour chaque occurrence, de manière identique ou différente, un système de cycle aromatique ou hétéroaromatique qui comporte 5 à 30 atomes de cycle aromatique, lequel peut être substitué par un radical ou par plusieurs radicaux R³ non aromatique(s) ; deux radicaux Ar¹ qui sont ici liés au même atome de N, au même atome de P ou au même atome de B peuvent également être pontés l'un à l'autre au moyen d'une liaison simple ou d'un pont sélectionné parmi N(R³), C(R³)₂, O ou S ;
R³ est sélectionné pour chaque occurrence, de manière identique ou différente, parmi le groupe qui est constitué par H, D, F, CN, un radical hydrocarbone aliphatique qui comporte 1 à 20 atome(s) de C ou un système de cycle aromatique ou hétéroaromatique qui comporte 5 à 30 atomes de cycle aromatique, où un ou plusieurs atome(s) de H peut/peuvent être remplacé(s) par D, F, Cl, Br, I ou CN, où deux substituants R³ adjacents ou plus peuvent former un système de cycle aliphatique monocyclique ou polycyclique l'un avec l'autre ou les uns avec les autres ;
p est pour chaque occurrence, de manière identique ou différente, 0, 1, 2, 3 ou 4;
q est 0, 1 ou 2 ;
les composés qui suivent sont exclus de l'invention :

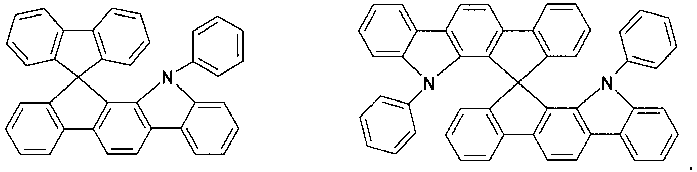

2. Composé selon la revendication 1, **caractérisé en ce que** X représente, de manière identique ou différente pour chaque occurrence, CR¹ ou N, où un maximum d'un groupe X par cycle représente N, ou représente, au niveau de deux groupes X adjacents, un groupe de la formule (4), où Z représente, de manière identique ou différente pour chaque occurrence, CR¹ et V représente, de manière identique ou différente pour chaque occurrence, NR¹, C(R¹)₂, O ou S, et les groupes X restants dans le cycle représentent CR¹.

3. Composé selon la revendication 1 ou 2, sélectionné parmi les composés des formules (7) à (15) : 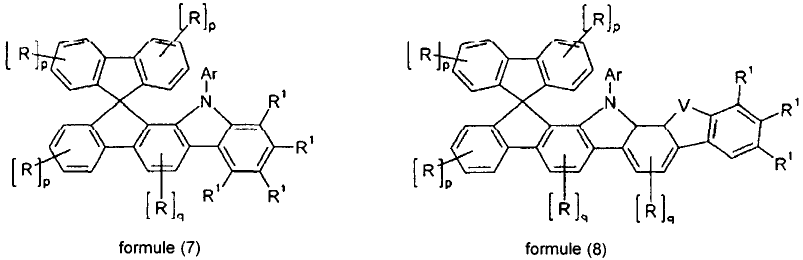 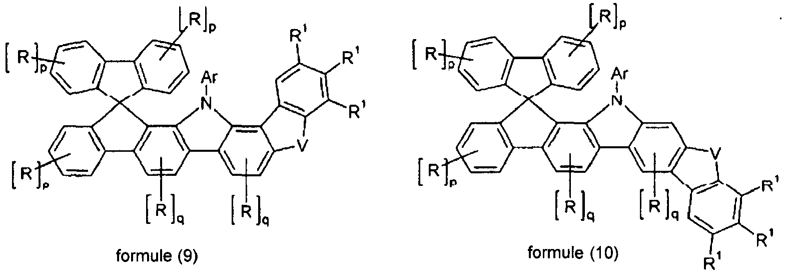 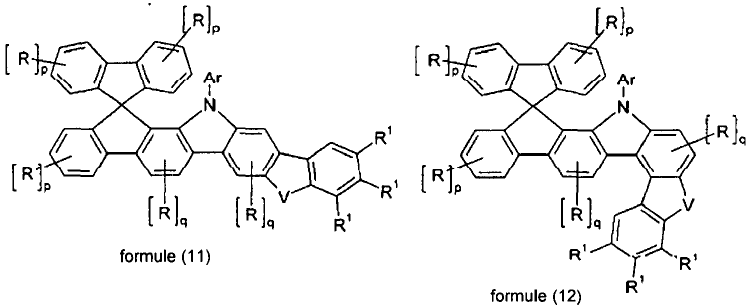 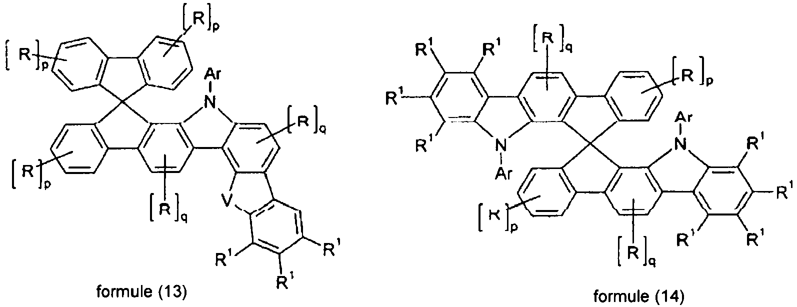 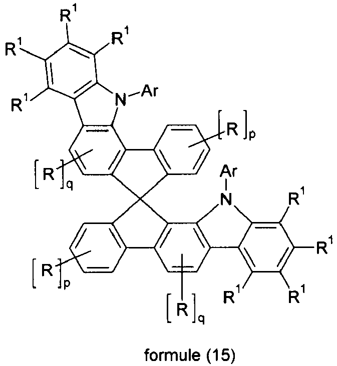 formules dans lesquelles les symboles et indices qui sont utilisés présentent les significations qui ont été données dans la revendication 1.

4. Composé selon une ou plusieurs des revendications 1 à 3, sélectionné parmi les composés des formules (7a) à (15a) : 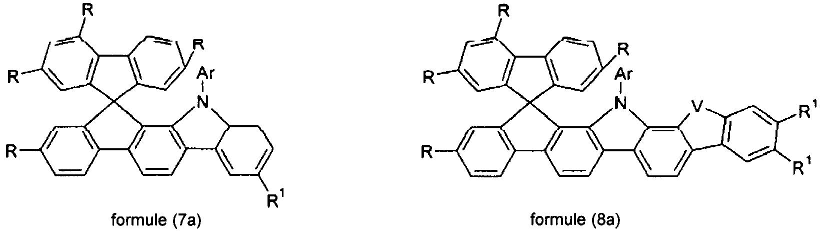 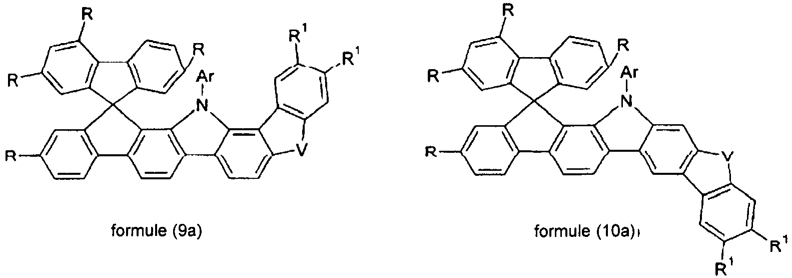 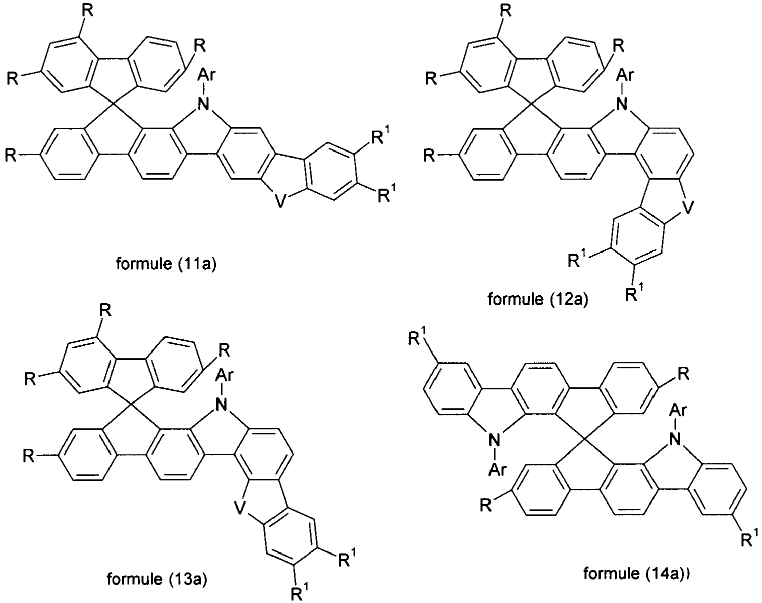 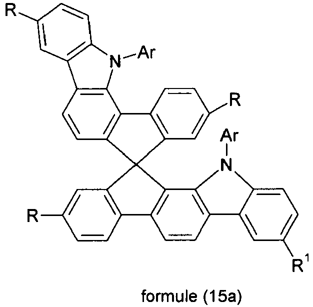 formules dans lesquelles les symboles et indices qui sont utilisés présentent les significations qui ont été données dans la revendication 1.

5. Composé selon une ou plusieurs des revendications 1 à 4, **caractérisé en ce que** R est sélectionné, de manière identique ou différente pour chaque occurrence, parmi le groupe qui est constitué par H, F, CN, N(Ar¹)₂, un groupe alkyle en chaîne droite qui comporte 1 à 10 atome(s) de C ou un groupe alkyle ramifié ou cyclique qui comporte 3 à 10 atomes de C ou un système de cycle aromatique qui comporte 6 à 30 atomes de cycle aromatique, lequel peut être substitué par un radical ou par plusieurs radicaux R³ non aromatiques.

6. Composé selon une ou plusieurs des revendications 1 à 5, sélectionné parmi les composés des formules (7b) à (15b) : 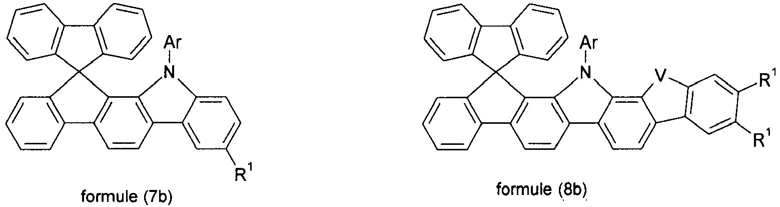 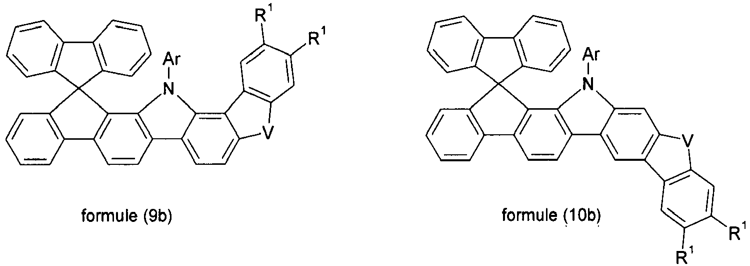 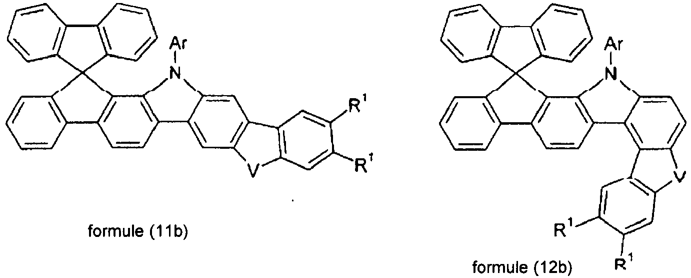 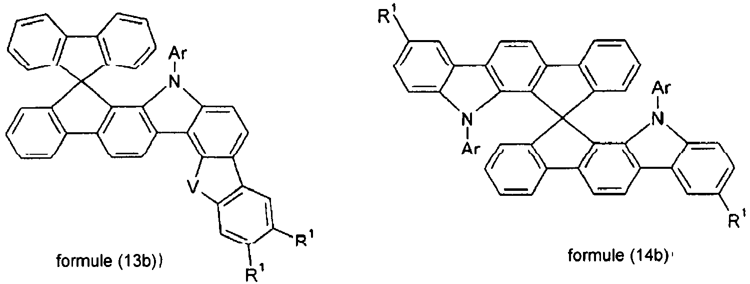 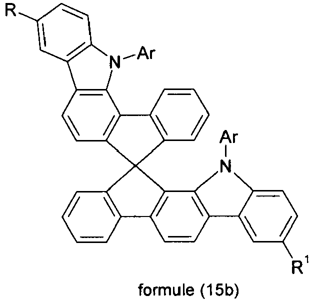 formules dans lesquelles les symboles qui sont utilisés présentent les significations qui ont été données dans la revendication 1.

7. Composé selon une ou plusieurs des revendications 1 à 6, **caractérisé en ce que** les composés des formules (1) et (3) contiennent au total au moins 12 atomes de cycle aromatique dans les substituants Ar, R¹ et R² et **en ce que** les composés de la formule (2) contiennent au total au moins 24 atomes de cycle aromatique dans les substituants Ar, R¹ et R².

8. Composé selon une ou plusieurs des revendications 1 à 7, **caractérisé en ce que** Ar est sélectionné parmi les groupes des formules Ar-1 à Ar-58 : 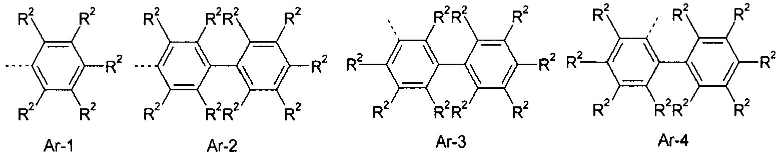 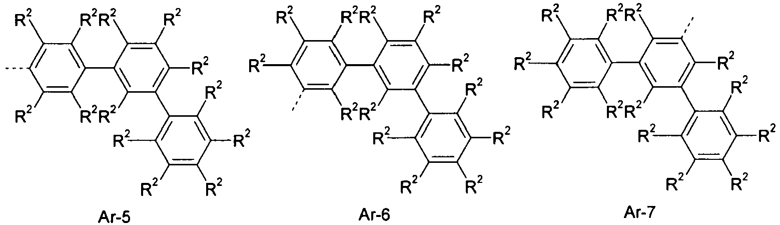 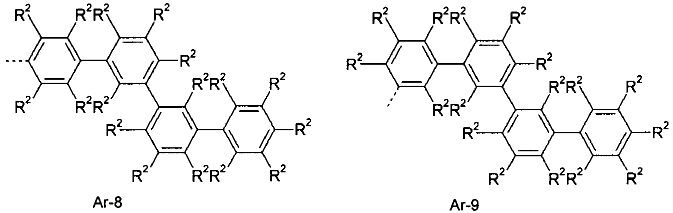 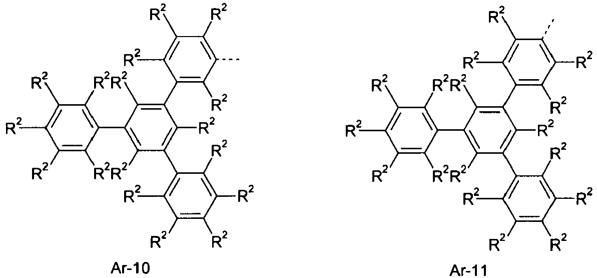 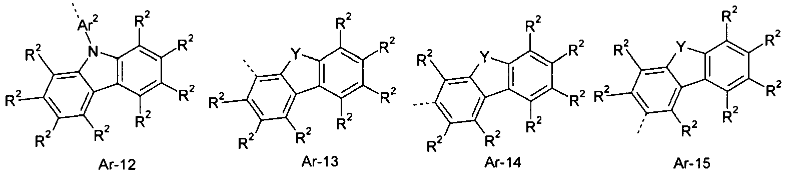 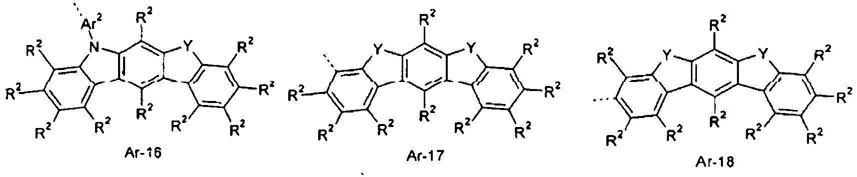 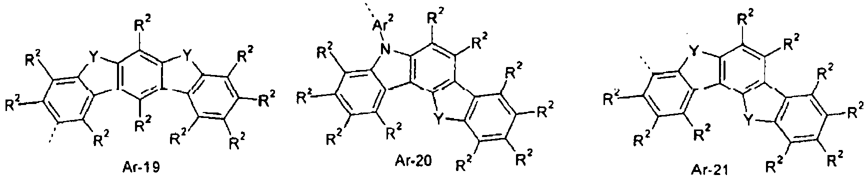 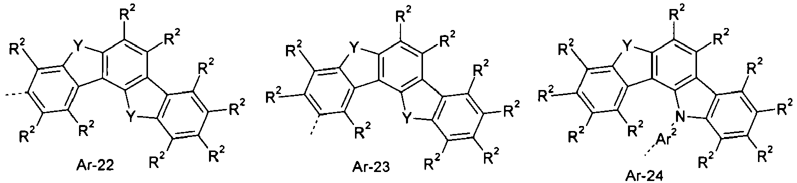 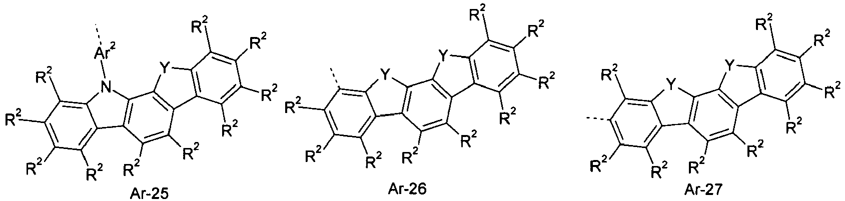 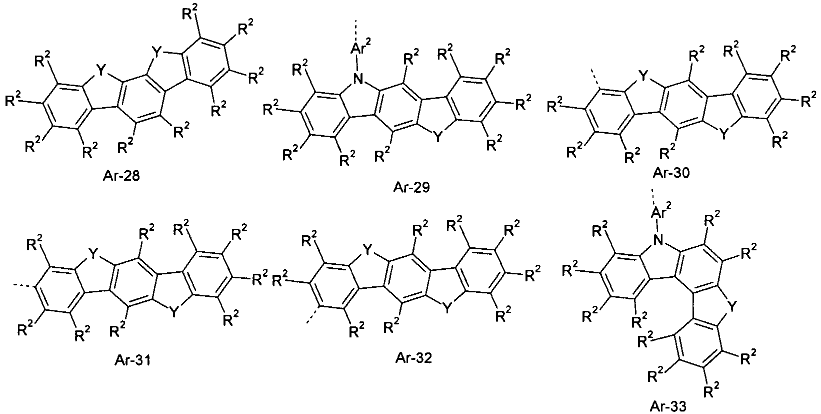 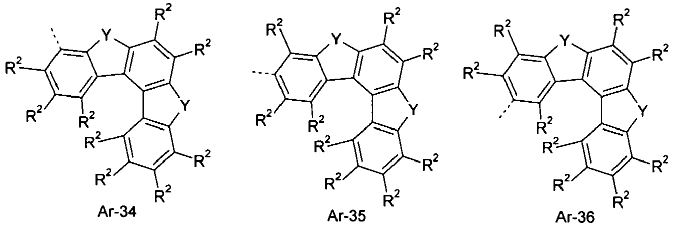 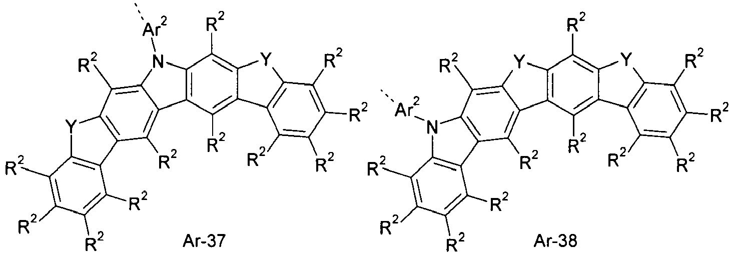 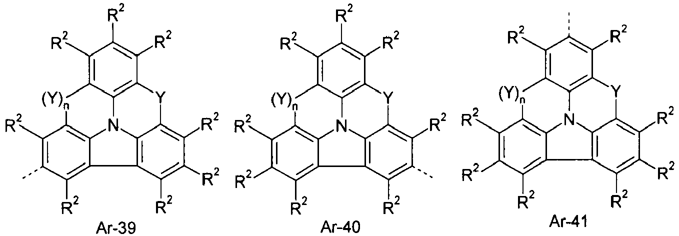 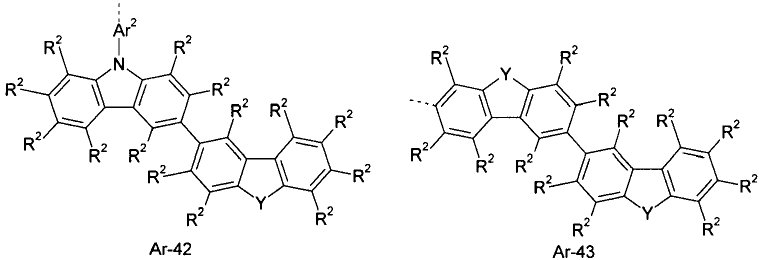 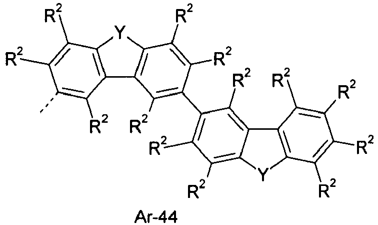 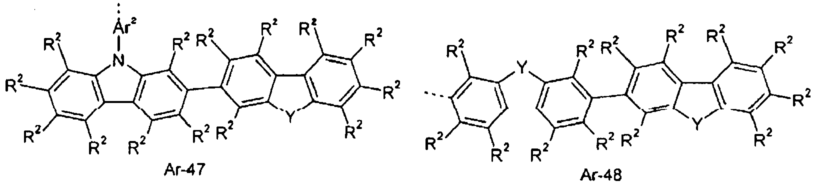 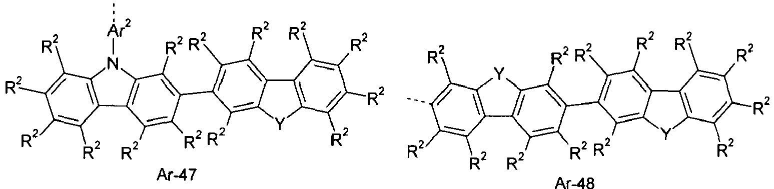 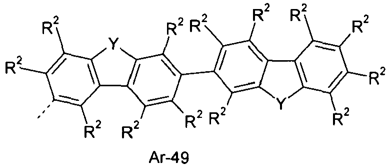 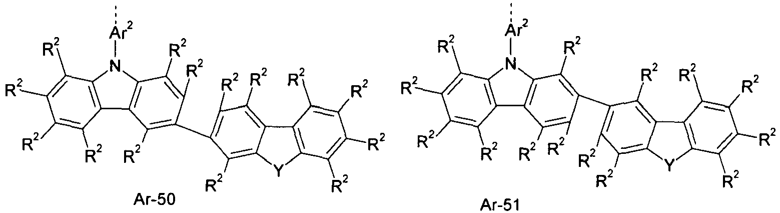 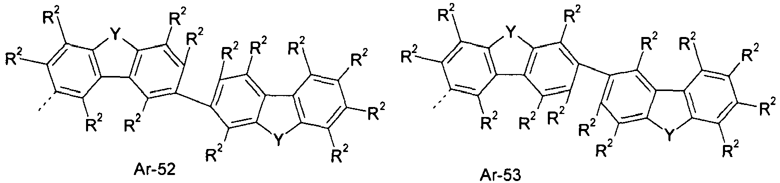 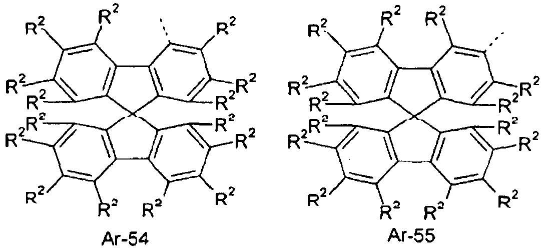 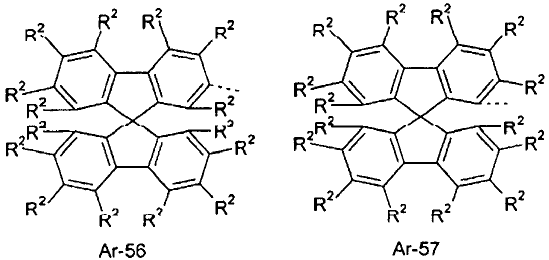 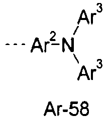 formules dans lesquelles R² présente les significations qui ont été données dans la revendication 1, la liaison en pointillés représente la liaison sur le groupe de la formule (1) ou de la formule (2) ou de la formule (3) et en outre :
Ar² est pour chaque occurrence, de manière identique ou différente, un système de cycle aromatique ou hétéroaromatique divalent qui comporte 6 à 18 atomes de cycle aromatique, lequel ne contient pas de groupes hétéroaryle déficients en électrons et lequel peut dans chaque cas être substitué par un radical ou par plusieurs radicaux R² ;
Ar³ est, de manière identique ou différente pour chaque occurrence, un système de cycle aromatique ou hétéroaromatique qui comporte 6 à 18 atomes de cycle aromatique, lequel ne contient pas de groupes hétéroaryle déficients en électrons et lequel peut dans chaque cas être substitué par un radical ou par plusieurs radicaux R²;
Y est pour chaque occurrence, de manière identique ou différente, C(R²)₂, NR², O ou S ;
n est 0 ou 1, où n égal à 0 signifie qu'aucun groupe Y n'est lié au niveau de cette position et qu'en lieu et place, des radicaux R² sont liés aux atomes de carbone correspondants ;
où les groupes Ar-1 à Ar-57 peuvent également être liés à l'atome d'azote dans la formule (1) ou dans la formule (2) ou dans la formule (3) via un groupe de pontage.

9. Composé selon une ou plusieurs des revendications 1 à 8, **caractérisé en ce que** R¹ est sélectionné, de manière identique ou différente pour chaque occurrence, parmi le groupe qui est constitué par H, D, N(Ar¹)₂, un groupe alkyle en chaîne droite qui comporte 1 à 10 atome(s) de C ou un groupe alkyle ramifié ou cyclique qui comporte 3 à 10 atomes de C, lequel peut dans chaque cas être substitué par un radical ou par plusieurs radicaux R³, où un ou plusieurs atome(s) de H peut/peuvent être remplacé(s) par D ou F, ou un système de cycle aromatique ou hétéroaromatique qui comporte 5 à 30 atomes de cycle aromatique, lequel peut dans chaque cas être substitué par un radical ou par plusieurs radicaux R³.

10. Composé selon une ou plusieurs des revendications 1 à 9, **caractérisé en ce que** R¹, s'il représente un système de cycle aromatique ou hétéroaromatique, est sélectionné parmi les groupes R¹-1 à R¹-43 : 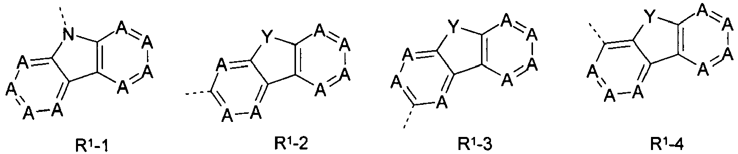 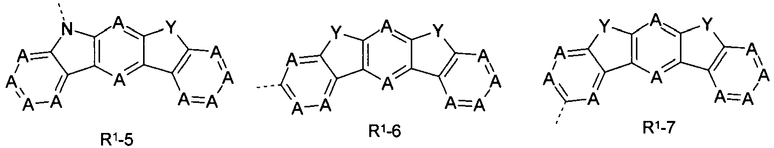 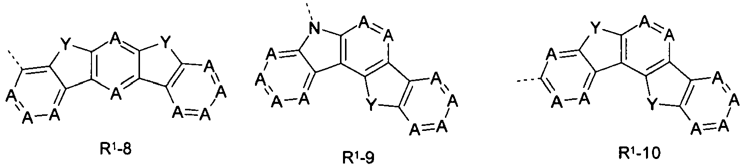 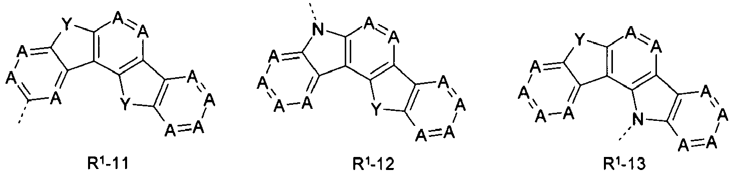 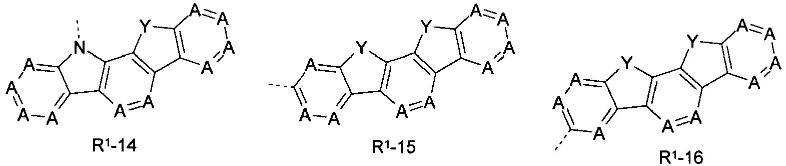 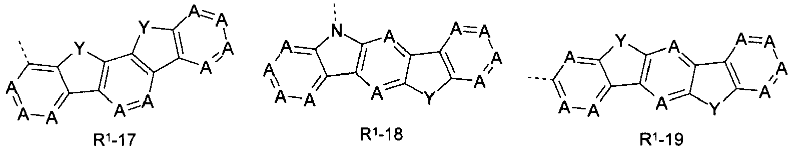 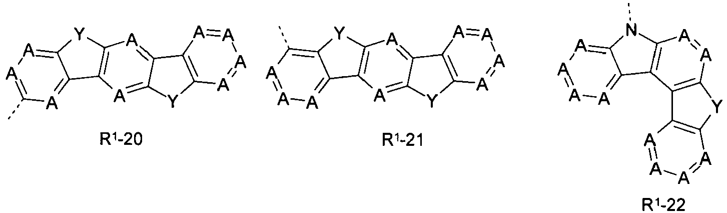 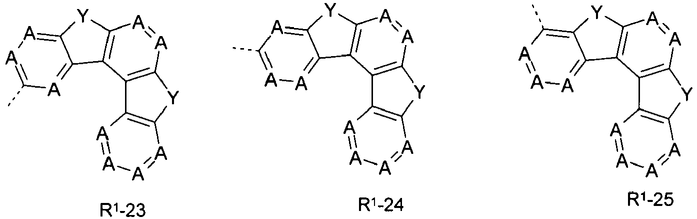 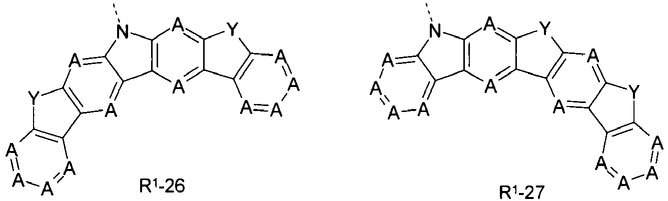 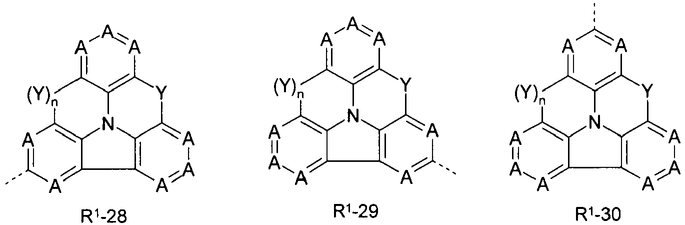 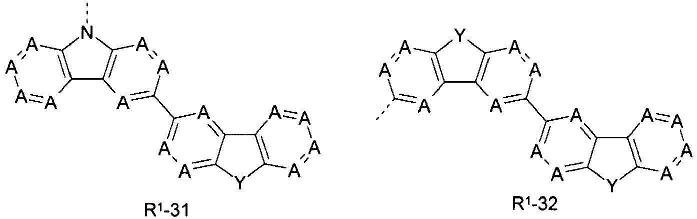 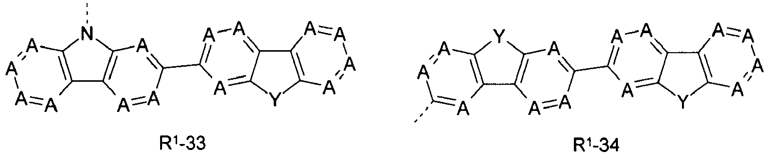 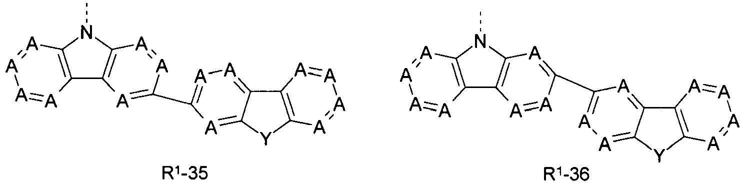 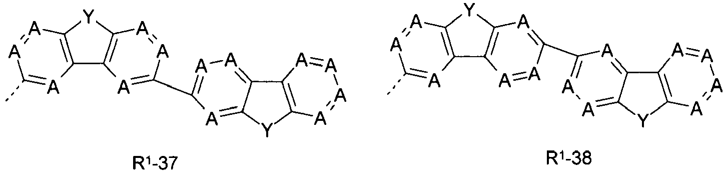 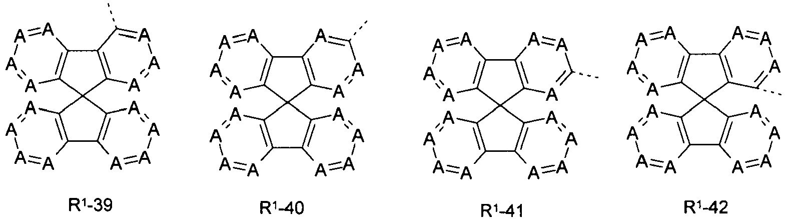 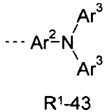 groupes dans lesquels R³ présente les significations qui ont été données dans la revendication 1, la liaison en pointillés représente la liaison sur le groupe de la formule (1) ou de la formule (2) ou de la formule (3) et en outre :
A est pour chaque occurrence, de manière identique ou différente, CR³ ou N, où un maximum de 3 symboles X par cycle représentent N ;
Y est pour chaque occurrence, de manière identique ou différente, C(R³)₂, NR³, O ou S ;
Ar², Ar³ sont, de manière identique ou différente pour chaque occurrence, un système de cycle aromatique ou hétéroaromatique qui comporte 5 à 18 atomes de cycle aromatique, lequel peut dans chaque cas être substitué par un radical ou par plusieurs radicaux R³;
n est 0 ou 1, où n égal à 0 signifie qu'aucun groupe Y n'est lié au niveau de cette position et qu'en lieu et place, des radicaux R³ sont liés aux atomes de carbone correspondants ;
où les groupes R¹-1 à R¹-42 peuvent également être liés à l'atome d'azote dans la formule (1) ou dans la formule (2) ou dans la formule (3) via un groupe de pontage.

11. Oligomère, polymère ou dendrimère contenant un ou plusieurs composé(s) selon une ou plusieurs des revendications 1 à 10, où une ou plusieurs liaison(s) depuis le composé sur le polymère, l'oligomère ou le dendrimère est/sont présente(s) au niveau d'une ou de plusieurs position(s) en lieu et place de substituants.

12. Formulation comprenant au moins un composé selon une ou plusieurs des revendications 1 à 10 et/ou au moins un oligomère, un polymère ou un dendrimère selon la revendication 11 et au moins un autre composé, en particulier un solvant.

13. Utilisation d'un composé selon une ou plusieurs des revendications 1 à 10 et/ou d'un oligomère, d'un polymère ou d'un dendrimère selon la revendication 11 dans un dispositif électronique.

14. Dispositif électronique contenant au moins un composé selon une ou plusieurs des revendications 1 à 10 et/ou au moins un oligomère, un polymère ou un dendrimère selon la revendication 11, de préférence sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires organiques sensibilisées par colorant(s), les détecteurs optiques organiques, les photorécepteurs organiques, les dispositifs à extinction de champ organiques, les cellules électrochimiques à émission de lumière, les diodes laser organiques et les dispositifs à émission de plasmons organiques.

15. Dispositif électronique selon la revendication 14, lequel est un dispositif électroluminescent organique, **caractérisé en ce que** le composé selon une ou plusieurs des revendications 1 à 12 est employé en tant que matériau de matrice pour un émetteur phosphorescent ou fluorescent et/ou dans une couche de blocage d'électrons ou de blocage d'excitons et/ou dans une couche de transport de trous et/ou dans une couche de blocage de trous et/ou dans une couche de blocage de trous ou de transport d'électrons.
